# EUROPEAN PATENT SPECIFICATION

(11) **EP 2 748 202 B1**
(45) Date of publication and mention of the grant of the patent: **04.07.2018**
(21) Application number: 12755967.2
(22) Date of filing: 21.08.2012
(51) Int. Cl.: C07K 16/30, A61K 39/395, A61P 35/00

(54) **BISPECIFIC ANTIGEN BINDING MOLECULES**
BISPEZIFISCHE ANTIGENBINDENDE MOLEKÜLE
MOLÉCULES BISPÉCIFIQUES DE LIAISON À UN ANTIGÈNE

(30) Priority: 23.08.2011 EP 11178371; 16.05.2012 EP 12168189
(43) Date of publication of application: 02.07.2014
(73) Proprietor: Roche Glycart AG, 8952 Schlieren (CH)
(72) Inventor: AUER, Johannes, 82445 Schwaigen (DE); BRUENKER, Peter, CH-8335 Hittnau (CH); FAUTI, Tanja, CH-8049 Zuerich (CH); NEUMANN, Christiane, CH-8166 Niederweiningen (CH); KLEIN, Christian, CH-8906 Bonstetten (CH); SCHAEFER, Wolfgang, 68199 Mannheim (DE); SUSTMANN, Claudio, 81373 München (DE); UMANA, Pablo, CH-8832 Wollerau (CH)
(74) Representative: Cueni, Leah Noëmi
(86) International application number: PCT/EP2012/066213
(87) International publication number: WO 2013/026831

(56) References cited:
- WO-A1-2009/018386
- WO-A1-2009/080251
- WO-A1-2009/080253
- WO-A1-2009/080254
- WO-A1-2010/115551
- WO-A1-2010/115552
- WO-A1-2010/115589
- WO-A1-2010/136172
- WO-A1-2010/145792
- WO-A1-2010/145793
- WO-A2-2007/073499
- WO-A2-2007/075270
- WO-A2-2008/119567
- W. SCHAEFER ET AL: "Immunoglobulin domain crossover as a generic approach for the production of bispecific IgG antibodies", PROCEEDINGS OF THE NATIONAL ACADEMY OF SCIENCES, vol. 108, no. 27, 5 July 2011 (2011-07-05) , pages 11187-11192, XP055003817, ISSN: 0027-8424, DOI: 10.1073/pnas.1019002108
- CHAN L A ET AL: "Variable region domain exchange in human IgGs promotes antibody complex formation with accompanying structural changes and altered effector functions", MOLECULAR IMMUNOLOGY, PERGAMON, GB, vol. 41, no. 5, 1 July 2004 (2004-07-01), pages 527-538, XP002519620, ISSN: 0161-5890, DOI: 10.1016/J.MOLIMM.2004.03.034
- WOLF ET AL: "BiTEs: bispecific antibody constructs with unique anti-tumor activity", DRUG DISCOVERY TODAY, ELSEVIER, RAHWAY, NJ, US, vol. 10, no. 18, 15 September 2005 (2005-09-15), pages 1237-1244, XP005103829, ISSN: 1359-6446, DOI: 10.1016/S1359-6446(05)03554-3
- DIRK NAGORSEN ET AL: "Immunomodulatory therapy of cancer with T cell-engaging BiTE antibody blinatumomab", EXPERIMENTAL CELL RESEARCH, ACADEMIC PRESS, US, vol. 317, no. 9, 10 March 2011 (2011-03-10), pages 1255-1260, XP028205663, ISSN: 0014-4827, DOI: 10.1016/J.YEXCR.2011.03.010 [retrieved on 2011-03-16]
- BOSCH J J ET AL: "MCSP/CD3-bispecific single-chain antibody construct engages CD4+ and CD8+ T cells for lysis of MCSP-expressing human uveal melanoma cells", INTERNET CITATION, 21 April 2010 (2010-04-21), XP002685799, Retrieved from the Internet: URL:http://www.abstractsonline.com/Plan/Vi ewAbstract.aspx?mID=2521&sKey=ef81a5fa-b15 c-4c4e-aa57-870b6479e274&cKey=a695d122-de2 0-451f-aeba-daf2b2a898f1&mKey={0591FA3B-AF EF-49D2-8E65-55F41EE8117E} [retrieved on 2012-10-23]
- OSHIMI ET AL: "Increased lysis of patient CD10-positive leukemic cells by T cells coated with anti-CD3 Fab' antibody cross-linked to anti-CD10 Fab' antibody.", BLOOD, vol. 77, no. 5, 1 March 1991 (1991-03-01), pages 1044-1049, XP055041891, ISSN: 0006-4971
- TUTT A ET AL: "TRISPECIFIC F(AB')3 DERIVATIVES THAT USE COOPERATIVE SIGNALING VIA THE TCR/CD3 COMPLEX AND CD2 TO ACTIVATE AND REDIRECT RESTING CYTOTOXIC T CELLS", THE JOURNAL OF IMMUNOLOGY, THE AMERICAN ASSOCIATION OF IMMUNOLOGISTS, US, vol. 147, no. 1, 1 July 1991 (1991-07-01), pages 60-69, XP000863699, ISSN: 0022-1767
- MILLER KATHY ET AL: "Design, construction, and in vitro analyses of multivalent antibodies", THE JOURNAL OF IMMUNOLOGY, THE AMERICAN ASSOCIATION OF IMMUNOLOGISTS, US, vol. 170, no. 9, 1 May 2003 (2003-05-01), pages 4854-4861, XP002508787, ISSN: 0022-1767
- EVAN P BOOY ET AL: "Monoclonal and bispecific antibodies as novel therapeutics", ARCHIVUM IMMUNOLOGIAE ET THERAPIAE EXPERIMENTALIS, BIRKHAEUSER-VERLAG, BASEL, CH, vol. 54, no. 2, 1 April 2006 (2006-04-01), pages 85-101, XP019200147, ISSN: 1661-4917, DOI: 10.1007/S00005-006-0011-5

### SEQUENCE LISTING

<110> Roche Glycart AG
<120> Bispecific antigen binding molecules
<130> 30601
<150> EP 11178371.8
   <151> 2011-08-23
<160> 111
<170> PatentIn version 3.5
<210> 1
   <211> 664
   <212> PRT
   <213> Artificial Sequence
<220>
   <223> V9 (VL-CH1)-LC007 (VH-CH1)-Fc(hole) P329G LALA
<400> 1
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Arg Asn Tyr
   20 25 30
   Leu Asn Trp Tyr Gln Gln Lys Pro Gly Lys Ala Pro Lys Leu Leu Ile
   35 40 45
   Tyr Tyr Thr Ser Arg Leu Glu Ser Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Gln Gln Gly Asn Thr Leu Pro Trp
   85 90 95
   Thr Phe Gly Gln Gly Thr Lys Val Glu Ile Lys Ser Ser Ala Ser Thr
   100 105 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Lys Asp Tyr Phe Pro Glu
   130 135 140
   Pro Val Thr Val Ser Trp Asn Ser Gly Ala Leu Thr Ser Gly Val His
   145 150 155 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Gly Gly Gly Gly Ser Gly Gly Gly Gly Ser Glu Val
       210                 215                 220
   Gln Leu Gln Glu Ser Gly Pro Gly Leu Val Lys Pro Ser Gln Ser Leu
   225                 230                 235                 240
   Ser Leu Thr Cys Ser Val Thr Gly Tyr Ser Ile Thr Ser Gly Tyr Tyr
                   245                 250                 255
   Trp Asn Trp Ile Arg Gln Phe Pro Gly Asn Lys Leu Glu Trp Met Gly
               260                 265                 270
   Tyr Ile Thr Tyr Asp Gly Ser Asn Asn Tyr Asn Pro Ser Leu Lys Asn
           275                 280                 285
   Arg Ile Ser Ile Thr Arg Asp Thr Ser Lys Asn Gln Phe Phe Leu Lys
       290                 295                 300
   Leu Asn Ser Val Thr Thr Glu Asp Thr Ala Thr Tyr Tyr Cys Ala Asp
   305                 310                 315                 320
   Phe Asp Tyr Trp Gly Gln Gly Thr Thr Leu Thr Val Ser Ser Ala Ser
                   325                 330                 335
   Thr Lys Gly Pro Ser Val Phe Pro Leu Ala Pro Ser Ser Lys Ser Thr
               340                 345                 350
   Ser Gly Gly Thr Ala Ala Leu Gly Cys Leu Val Lys Asp Tyr Phe Pro
           355                 360                 365
   Glu Pro Val Thr Val Ser Trp Asn Ser Gly Ala Leu Thr Ser Gly Val
       370                 375                 380
   His Thr Phe Pro Ala Val Leu Gln Ser Ser Gly Leu Tyr Ser Leu Ser
   385                 390                 395                 400
   Ser Val Val Thr Val Pro Ser Ser Ser Leu Gly Thr Gln Thr Tyr Ile
                   405                 410                 415
   Cys Asn Val Asn His Lys Pro Ser Asn Thr Lys Val Asp Lys Lys Val
               420                 425                 430
   Glu Pro Lys Ser Cys Asp Lys Thr His Thr Cys Pro Pro Cys Pro Ala
           435                 440                 445
   Pro Glu Ala Ala Gly Gly Pro Ser Val Phe Leu Phe Pro Pro Lys Pro
       450                 455                 460
   Lys Asp Thr Leu Met Ile Ser Arg Thr Pro Glu Val Thr Cys Val Val
   465                 470                 475                 480
   Val Asp Val Ser His Glu Asp Pro Glu Val Lys Phe Asn Trp Tyr Val
                   485                 490                 495
   Asp Gly Val Glu Val His Asn Ala Lys Thr Lys Pro Arg Glu Glu Gln
               500                 505                 510
   Tyr Asn Ser Thr Tyr Arg Val Val Ser Val Leu Thr Val Leu His Gln
           515                 520                 525
   Asp Trp Leu Asn Gly Lys Glu Tyr Lys Cys Lys Val Ser Asn Lys Ala
       530                 535                 540
   Leu Gly Ala Pro Ile Glu Lys Thr Ile Ser Lys Ala Lys Gly Gln Pro
   545                 550                 555                 560
   Arg Glu Pro Gln Val Cys Thr Leu Pro Pro Ser Arg Asp Glu Leu Thr
                   565                 570                 575
   Lys Asn Gln Val Ser Leu Ser Cys Ala Val Lys Gly Phe Tyr Pro Ser
               580                 585                 590
   Asp Ile Ala Val Glu Trp Glu Ser Asn Gly Gln Pro Glu Asn Asn Tyr
           595                 600                 605
   Lys Thr Thr Pro Pro Val Leu Asp Ser Asp Gly Ser Phe Phe Leu Val
       610                 615                 620
   Ser Lys Leu Thr Val Asp Lys Ser Arg Trp Gln Gln Gly Asn Val Phe
   625                 630                 635                 640
   Ser Cys Ser Val Met His Glu Ala Leu His Asn His Tyr Thr Gln Lys
                   645                 650                 655
   Ser Leu Ser Leu Ser Pro Gly Lys
               660
<210> 2
   <211> 229
   <212> PRT
   <213> Artificial Sequence
<220>
   <223> Fc(knob) wt
<400> 2 225
<210> 3
   <211> 229
   <212> PRT
   <213> Artificial Sequence
<220>
   <223> V9 (VH-CL)
<400> 3
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Gln Gly Thr Leu Val Thr Val Ser Ser Ala Ser Val Ala Ala Pro
           115                 120                 125
   Ser Val Phe Ile Phe Pro Pro Ser Asp Glu Gln Leu Lys Ser Gly Thr
       130                 135                 140
   Ala Ser Val Val Cys Leu Leu Asn Asn Phe Tyr Pro Arg Glu Ala Lys
   145                 150                 155                 160
   Val Gln Trp Lys Val Asp Asn Ala Leu Gln Ser Gly Asn Ser Gln Glu
                   165                 170                 175
   Ser Val Thr Glu Gln Asp Ser Lys Asp Ser Thr Tyr Ser Leu Ser Ser
               180                 185                 190
   Thr Leu Thr Leu Ser Lys Ala Asp Tyr Glu Lys His Lys Val Tyr Ala
           195                 200                 205
   Cys Glu Val Thr His Gln Gly Leu Ser Ser Pro Val Thr Lys Ser Phe
       210                 215                 220
   Asn Arg Gly Glu Cys
   225
<210> 4
   <211> 214
   <212> PRT
   <213> Artificial Sequence
<220>
   <223> LC007 (VL-CL)
<400> 4
   Asp Ile Val Leu Thr Gln Ser Pro Ser Ser Leu Ser Ala Ser Leu Gly
   1               5                   10                  15
   Asp Arg Val Thr Ile Ser Cys Ser Ala Ser Gln Gly Ile Arg Asn Tyr
               20                  25                  30
   Leu Asn Trp Tyr Gln Gln Arg Pro Asp Gly Thr Val Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Lys Leu Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 5
   <211> 442
   <212> PRT
   <213> Artificial Sequence
<220>
   <223> LC007 (VH-CH1)-Fc(knob) P329G LALA
<400> 5
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Gly Ala Pro Ile Glu Lys Thr Ile Ser Lys Ala Lys Gly
                   325                 330                 335
   Gln Pro Arg Glu Pro Gln Val Tyr Thr Leu Pro Pro Cys Arg Asp Glu
               340                 345                 350
   Leu Thr Lys Asn Gln Val Ser Leu Trp Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440
<210> 6
   <211> 670
   <212> PRT
   <213> Artificial Sequence
<220>
   <223> FN18 (VL-CH1)-LC007 (VH-CH1)-Fc(hole) P329G LALA
<400> 6
   Asp Ile Val Met Ser Gln Ser Pro Ser Ser Leu Ala Val Ser Val Gly
   1               5                   10                  15
   Glu Lys Val Thr Met Ser Cys Lys Ser Ser Gln Ser Leu Leu Tyr Ser
               20                  25                  30
   Ser Asn Gln Lys Asn Tyr Leu Ala Trp Tyr Gln Gln Lys Pro Gly Gln
           35                  40                  45
   Ser Pro Lys Leu Leu Ile Asn Trp Ala Ser Thr Arg Glu Ser Gly Val
       50                  55                  60
   Pro Asp Arg Phe Thr Gly Ser Gly Ser Arg Thr Asp Phe Thr Leu Thr
   65                  70                  75                  80
   Ile Ser Ser Val Lys Ala Glu Asp Leu Ala Val Tyr Phe Cys Gln Gln
                   85                  90                  95
   Phe Tyr Ser Tyr Pro Pro Thr Phe Gly Gly Gly Thr Lys Leu Glu Ile
               100                 105                 110
   Lys Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Lys Val Glu Pro Lys Ser Cys Gly Gly Gly Gly Ser Gly
       210                 215                 220
   Gly Gly Gly Ser Glu Val Gln Leu Gln Glu Ser Gly Pro Gly Leu Val
   225                 230                 235                 240
   Lys Pro Ser Gln Ser Leu Ser Leu Thr Cys Ser Val Thr Gly Tyr Ser
                   245                 250                 255
   Ile Thr Ser Gly Tyr Tyr Trp Asn Trp Ile Arg Gln Phe Pro Gly Asn
               260                 265                 270
   Lys Leu Glu Trp Met Gly Tyr Ile Thr Tyr Asp Gly Ser Asn Asn Tyr
           275                 280                 285
   Asn Pro Ser Leu Lys Asn Arg Ile Ser Ile Thr Arg Asp Thr Ser Lys
       290                 295                 300
   Asn Gln Phe Phe Leu Lys Leu Asn Ser Val Thr Thr Glu Asp Thr Ala
   305                 310                 315                 320
   Thr Tyr Tyr Cys Ala Asp Phe Asp Tyr Trp Gly Gln Gly Thr Thr Leu
                   325                 330                 335
   Thr Val Ser Ser Ala Ser Thr Lys Gly Pro Ser Val Phe Pro Leu Ala
               340                 345                 350
   Pro Ser Ser Lys Ser Thr Ser Gly Gly Thr Ala Ala Leu Gly Cys Leu
           355                 360                 365
   Val Lys Asp Tyr Phe Pro Glu Pro Val Thr Val Ser Trp Asn Ser Gly
       370                 375                 380
   Ala Leu Thr Ser Gly Val His Thr Phe Pro Ala Val Leu Gln Ser Ser
   385                 390                 395                 400
   Gly Leu Tyr Ser Leu Ser Ser Val Val Thr Val Pro Ser Ser Ser Leu
                   405                 410                 415
   Gly Thr Gln Thr Tyr Ile Cys Asn Val Asn His Lys Pro Ser Asn Thr
               420                 425                 430
   Lys Val Asp Lys Lys Val Glu Pro Lys Ser Cys Asp Lys Thr His Thr
           435                 440                 445
   Cys Pro Pro Cys Pro Ala Pro Glu Ala Ala Gly Gly Pro Ser Val Phe
       450                 455                 460
   Leu Phe Pro Pro Lys Pro Lys Asp Thr Leu Met Ile Ser Arg Thr Pro
   465                 470                 475                 480
   Glu Val Thr Cys Val Val Val Asp Val Ser His Glu Asp Pro Glu Val
                   485                 490                 495
   Lys Phe Asn Trp Tyr Val Asp Gly Val Glu Val His Asn Ala Lys Thr
               500                 505                 510
   Lys Pro Arg Glu Glu Gln Tyr Asn Ser Thr Tyr Arg Val Val Ser Val
           515                 520                 525
   Leu Thr Val Leu His Gln Asp Trp Leu Asn Gly Lys Glu Tyr Lys Cys
       530                 535                 540
   Lys Val Ser Asn Lys Ala Leu Gly Ala Pro Ile Glu Lys Thr Ile Ser
   545                 550                 555                 560
   Lys Ala Lys Gly Gln Pro Arg Glu Pro Gln Val Cys Thr Leu Pro Pro
                   565                 570                 575
   Ser Arg Asp Glu Leu Thr Lys Asn Gln Val Ser Leu Ser Cys Ala Val
               580                 585                 590
   Lys Gly Phe Tyr Pro Ser Asp Ile Ala Val Glu Trp Glu Ser Asn Gly
           595                 600                 605
   Gln Pro Glu Asn Asn Tyr Lys Thr Thr Pro Pro Val Leu Asp Ser Asp
       610                 615                 620
   Gly Ser Phe Phe Leu Val Ser Lys Leu Thr Val Asp Lys Ser Arg Trp
   625                 630                 635                 640
   Gln Gln Gly Asn Val Phe Ser Cys Ser Val Met His Glu Ala Leu His
                   645                 650                 655
   Asn His Tyr Thr Gln Lys Ser Leu Ser Leu Ser Pro Gly Lys
               660                 665                 670
<210> 7
   <211> 231
   <212> PRT
   <213> Artificial Sequence
<220>
   <223> FN18 (VH-CL)
<400> 7
   Gln Val Gln Leu Gln Gln Ser Glu Ala Glu Leu Ala Arg Pro Gly Ala
   1 5 10 15
   Ser Val Lys Met Ser Cys Lys Ala Ser Gly Tyr Thr Phe Thr Asp Tyr
   20 25 30
   Thr Ile His Trp Leu Lys Gln Arg Pro Gly Gln Gly Leu Asp Trp Ile
   35 40 45
   Gly Tyr Phe Asn Pro Ser Ser Glu Ser Thr Glu Tyr Asn Arg Lys Phe
   50 55 60
   Lys Asp Arg Thr Ile Leu Thr Ala Asp Arg Ser Ser Thr Thr Ala Tyr
   65 70 75 80
   Met Gln Leu Ser Ser Leu Thr Ser Glu Asp Ser Ala Val Tyr Tyr Cys
   85 90 95
   Ser Arg Lys Gly Glu Lys Leu Leu Gly Asn Arg Tyr Trp Tyr Phe Asp
   100 105 110
   Val Trp Gly Ala Gly Thr Ser Val Thr Val Ser Ser Ala Ser Val Ala
   115 120 125
   Ala Pro Ser Val Phe Ile Phe Pro Pro Ser Asp Glu Gln Leu Lys Ser
   130 135 140
   Gly Thr Ala Ser Val Val Cys Leu Leu Asn Asn Phe Tyr Pro Arg Glu
   145 150 155 160
   Ala Lys Val Gln Trp Lys Val Asp Asn Ala Leu Gln Ser Gly Asn Ser
   165 170 175
   Gln Glu Ser Val Thr Glu Gln Asp Ser Lys Asp Ser Thr Tyr Ser Leu
   180 185 190
   Ser Ser Thr Leu Thr Leu Ser Lys Ala Asp Tyr Glu Lys His Lys Val
   195 200 205
   Tyr Ala Cys Glu Val Thr His Gln Gly Leu Ser Ser Pro Val Thr Lys
   210 215 220
   Ser Phe Asn Arg Gly Glu Cys
   225 230
<210> 8
   <211> 673
   <212> PRT
   <213> Artificial Sequence
<220>
   <223> CH1A1A (VH-CH1)- V9 (VL-CH1)-Fc(knob) P329G LALA
<400> 8
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Gly Gly Gly Gly Ser Gly Gly Gly Gly Ser Asp Ile Gln Met Thr Gln
   225                 230                 235                 240
   Ser Pro Ser Ser Leu Ser Ala Ser Val Gly Asp Arg Val Thr Ile Thr
                   245                 250                 255
   Cys Arg Ala Ser Gln Asp Ile Arg Asn Tyr Leu Asn Trp Tyr Gln Gln
               260                 265                 270
   Lys Pro Gly Lys Ala Pro Lys Leu Leu Ile Tyr Tyr Thr Ser Arg Leu
           275                 280                 285
   Glu Ser Gly Val Pro Ser Arg Phe Ser Gly Ser Gly Ser Gly Thr Asp
       290                 295                 300
   Tyr Thr Leu Thr Ile Ser Ser Leu Gln Pro Glu Asp Phe Ala Thr Tyr
   305                 310                 315                 320
   Tyr Cys Gln Gln Gly Asn Thr Leu Pro Trp Thr Phe Gly Gln Gly Thr
                   325                 330                 335
   Lys Val Glu Ile Lys Ser Ser Ala Ser Thr Lys Gly Pro Ser Val Phe
               340                 345                 350
   Pro Leu Ala Pro Ser Ser Lys Ser Thr Ser Gly Gly Thr Ala Ala Leu
           355                 360                 365
   Gly Cys Leu Val Lys Asp Tyr Phe Pro Glu Pro Val Thr Val Ser Trp
       370                 375                 380
   Asn Ser Gly Ala Leu Thr Ser Gly Val His Thr Phe Pro Ala Val Leu
   385                 390                 395                 400
   Gln Ser Ser Gly Leu Tyr Ser Leu Ser Ser Val Val Thr Val Pro Ser
                   405                 410                 415
   Ser Ser Leu Gly Thr Gln Thr Tyr Ile Cys Asn Val Asn His Lys Pro
               420                 425                 430
   Ser Asn Thr Lys Val Asp Lys Lys Val Glu Pro Lys Ser Cys Asp Lys
           435                 440                 445
   Thr His Thr Cys Pro Pro Cys Pro Ala Pro Glu Ala Ala Gly Gly Pro
       450                 455                 460
   Ser Val Phe Leu Phe Pro Pro Lys Pro Lys Asp Thr Leu Met Ile Ser
   465                 470                 475                 480
   Arg Thr Pro Glu Val Thr Cys Val Val Val Asp Val Ser His Glu Asp
                   485                 490                 495
   Pro Glu Val Lys Phe Asn Trp Tyr Val Asp Gly Val Glu Val His Asn
               500                 505                 510
   Ala Lys Thr Lys Pro Arg Glu Glu Gln Tyr Asn Ser Thr Tyr Arg Val
           515                 520                 525
   Val Ser Val Leu Thr Val Leu His Gln Asp Trp Leu Asn Gly Lys Glu
       530                 535                 540
   Tyr Lys Cys Lys Val Ser Asn Lys Ala Leu Gly Ala Pro Ile Glu Lys
   545                 550                 555                 560
   Thr Ile Ser Lys Ala Lys Gly Gln Pro Arg Glu Pro Gln Val Tyr Thr
                   565                 570                 575
   Leu Pro Pro Cys Arg Asp Glu Leu Thr Lys Asn Gln Val Ser Leu Trp
               580                 585                 590
   Cys Leu Val Lys Gly Phe Tyr Pro Ser Asp Ile Ala Val Glu Trp Glu
           595                 600                 605
   Ser Asn Gly Gln Pro Glu Asn Asn Tyr Lys Thr Thr Pro Pro Val Leu
       610                 615                 620
   Asp Ser Asp Gly Ser Phe Phe Leu Tyr Ser Lys Leu Thr Val Asp Lys
   625                 630                 635                 640
   Ser Arg Trp Gln Gln Gly Asn Val Phe Ser Cys Ser Val Met His Glu
                   645                 650                 655
   Ala Leu His Asn His Tyr Thr Gln Lys Ser Leu Ser Leu Ser Pro Gly
               660                 665                 670
   Lys
<210> 9
   <211> 451
   <212> PRT
   <213> Artificial Sequence
<220>
   <223> CH1A1A (VH-CH1)-Fc(hole) P329G LALA
<400> 9
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Ala Ala Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Gly Ala Pro Ile
                   325                 330                 335
   Glu Lys Thr Ile Ser Lys Ala Lys Gly Gln Pro Arg Glu Pro Gln Val
               340                 345                 350
   Cys Thr Leu Pro Pro Ser Arg Asp Glu Leu Thr Lys Asn Gln Val Ser
           355                 360                 365
   Leu Ser Cys Ala Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Val Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 10
   <211> 215
   <212> PRT
   <213> Artificial Sequence
<220>
   <223> CH1A1A (VL-CL)
<400> 10
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 11
   <211> 450
   <212> PRT
   <213> Artificial Sequence
<220>
   <223> anti-Her3 (VH-CH1)-Fc(knob)
<400> 11
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Arg Ser Ser
   20 25 30
   Tyr Ile Ser Trp Val Arg Gln Ala Pro Gly Gln Gly Leu Glu Trp Met
   35 40 45
   Gly Trp Ile Tyr Ala Gly Thr Gly Ser Pro Ser Tyr Asn Gln Lys Leu
   50 55 60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg His Arg Asp Tyr Tyr Ser Asn Ser Leu Thr Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Cys
   340 345 350
   Thr Leu Pro Pro Ser Arg Asp Glu Leu Thr Lys Asn Gln Val Ser Leu
   355 360 365
   Ser Cys Ala Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Val Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 12
   <211> 220
   <212> PRT
   <213> Artificial Sequence
<220>
   <223> anti-Her3 (VL-CL)
<400> 12
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Asn Ser
               20                  25                  30
   Gly Asn Gln Lys Asn Tyr Leu Thr Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Ser
                   85                  90                  95
   Asp Tyr Ser Tyr Pro Tyr Thr Phe Gly Gln Gly Thr Lys Leu Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 13
   <211> 696
   <212> PRT
   <213> Artificial Sequence
<220>
   <223> anti-Her3 (VH-CH1)-Fc(hole)-5D5 (VH-CL)
<400> 13
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Arg Ser Ser
               20                  25                  30
   Tyr Ile Ser Trp Val Arg Gln Ala Pro Gly Gln Gly Leu Glu Trp Met
           35                  40                  45
   Gly Trp Ile Tyr Ala Gly Thr Gly Ser Pro Ser Tyr Asn Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg His Arg Asp Tyr Tyr Ser Asn Ser Leu Thr Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Cys Arg Asp Glu Leu Thr Lys Asn Gln Val Ser Leu
           355                 360                 365
   Trp Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys Gly Gly Gly Gly Ser Gly Gly Gly Gly Ser Gly Gly Gly Gly
       450                 455                 460
   Ser Gly Gly Gly Gly Ser Glu Val Gln Leu Val Glu Ser Gly Gly Gly
   465                 470                 475                 480
   Leu Val Gln Pro Gly Gly Ser Leu Arg Leu Ser Cys Ala Ala Ser Gly
                   485                 490                 495
   Tyr Thr Phe Thr Ser Tyr Trp Leu His Trp Val Arg Gln Ala Pro Gly
               500                 505                 510
   Lys Gly Leu Glu Trp Val Gly Met Ile Asp Pro Ser Asn Ser Asp Thr
           515                 520                 525
   Arg Phe Asn Pro Asn Phe Lys Asp Arg Phe Thr Ile Ser Ala Asp Thr
       530                 535                 540
   Ser Lys Asn Thr Ala Tyr Leu Gln Met Asn Ser Leu Arg Ala Glu Asp
   545                 550                 555                 560
   Thr Ala Val Tyr Tyr Cys Ala Thr Tyr Arg Ser Tyr Val Thr Pro Leu
                   565                 570                 575
   Asp Tyr Trp Gly Gln Gly Thr Leu Val Thr Val Ser Ser Ala Ser Val
               580                 585                 590
   Ala Ala Pro Ser Val Phe Ile Phe Pro Pro Ser Asp Glu Gln Leu Lys
           595                 600                 605
   Ser Gly Thr Ala Ser Val Val Cys Leu Leu Asn Asn Phe Tyr Pro Arg
       610                 615                 620
   Glu Ala Lys Val Gln Trp Lys Val Asp Asn Ala Leu Gln Ser Gly Asn
   625                 630                 635                 640
   Ser Gln Glu Ser Val Thr Glu Gln Asp Ser Lys Asp Ser Thr Tyr Ser
                   645                 650                 655
   Leu Ser Ser Thr Leu Thr Leu Ser Lys Ala Asp Tyr Glu Lys His Lys
               660                 665                 670
   Val Tyr Ala Cys Glu Val Thr His Gln Gly Leu Ser Ser Pro Val Thr
           675                 680                 685
   Lys Ser Phe Asn Arg Gly Glu Cys
       690                 695
<210> 14
   <211> 218
   <212> PRT
   <213> Artificial Sequence
<220>
   <223> 5D5 (VL-CH1)
<400> 14
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Tyr Thr
   20 25 30
   Ser Ser Gln Lys Asn Tyr Leu Ala Trp Tyr Gln Gln Lys Pro Gly Lys
   35 40 45
   Ala Pro Lys Leu Leu Ile Tyr Trp Ala Ser Thr Arg Glu Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Tyr Tyr Ala Tyr Pro Trp Thr Phe Gly Gln Gly Thr Lys Val Glu Ile
   100 105 110
   Lys Ser Ser Ala Ser Thr Lys Gly Pro Ser Val Phe Pro Leu Ala Pro
   115 120 125
   Ser Ser Lys Ser Thr Ser Gly Gly Thr Ala Ala Leu Gly Cys Leu Val
   130 135 140
   Lys Asp Tyr Phe Pro Glu Pro Val Thr Val Ser Trp Asn Ser Gly Ala
   145 150 155 160
   Leu Thr Ser Gly Val His Thr Phe Pro Ala Val Leu Gln Ser Ser Gly
   165 170 175
   Leu Tyr Ser Leu Ser Ser Val Val Thr Val Pro Ser Ser Ser Leu Gly
   180 185 190
   Thr Gln Thr Tyr Ile Cys Asn Val Asn His Lys Pro Ser Asn Thr Lys
   195 200 205
   Val Asp Lys Lys Val Glu Pro Lys Ser Cys
   210 215
<210> 15
   <211> 11
   <212> PRT
   <213> Artificial Sequence
<220>
   <223> LC007 HCDR1
<400> 15
   Gly Tyr Ser Ile Thr Ser Gly Tyr Tyr Trp Asn
   1 5 10
<210> 16
   <211> 16
   <212> PRT
   <213> Artificial Sequence
<220>
   <223> LC007 HCDR2
<400> 16
   Tyr Ile Thr Tyr Asp Gly Ser Asn Asn Tyr Asn Pro Ser Leu Lys Asn
   1 5 10 15
<210> 17
   <211> 3
   <212> PRT
   <213> Artificial Sequence
<220>
   <223> LC007 HCDR3
<400> 17
   Phe Asp Tyr
   1
<210> 18
   <211> 112
   <212> PRT
   <213> Artificial Sequence
<220>
   <223> LC007 VH
<400> 18
   Glu Val Gln Leu Gln Glu Ser Gly Pro Gly Leu Val Lys Pro Ser Gln
   1 5 10 15
   Ser Leu Ser Leu Thr Cys Ser Val Thr Gly Tyr Ser Ile Thr Ser Gly
   20 25 30
   Tyr Tyr Trp Asn Trp Ile Arg Gln Phe Pro Gly Asn Lys Leu Glu Trp
   35 40 45
   Met Gly Tyr Ile Thr Tyr Asp Gly Ser Asn Asn Tyr Asn Pro Ser Leu
   50 55 60
   Lys Asn Arg Ile Ser Ile Thr Arg Asp Thr Ser Lys Asn Gln Phe Phe
   65 70 75 80
   Leu Lys Leu Asn Ser Val Thr Thr Glu Asp Thr Ala Thr Tyr Tyr Cys
   85 90 95
   Ala Asp Phe Asp Tyr Trp Gly Gln Gly Thr Thr Leu Thr Val Ser Ser
   100 105 110
<210> 19
   <211> 11
   <212> PRT
   <213> Artificial Sequence
<220>
   <223> LC007 LCDR1
<400> 19
   Ser Ala Ser Gln Gly Ile Arg Asn Tyr Leu Asn
   1 5 10
<210> 20
   <211> 7
   <212> PRT
   <213> Artificial Sequence
<220>
   <223> LC007 LCDR2
<400> 20
   Tyr Thr Ser Ser Leu His Ser
   1 5
<210> 21
   <211> 9
   <212> PRT
   <213> Artificial Sequence
<220>
   <223> LC007 LCDR3
<400> 21
   Gln Gln Tyr Ser Lys Leu Pro Trp Thr
   1 5
<210> 22
   <211> 107
   <212> PRT
   <213> Artificial Sequence
<220>
   <223> LC007 VL
<400> 22
   Asp Ile Val Leu Thr Gln Ser Pro Ser Ser Leu Ser Ala Ser Leu Gly
   1 5 10 15
   Asp Arg Val Thr Ile Ser Cys Ser Ala Ser Gln Gly Ile Arg Asn Tyr
   20 25 30
   Leu Asn Trp Tyr Gln Gln Arg Pro Asp Gly Thr Val Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Tyr Ser Leu Thr Ile Ser Asn Leu Glu Pro
   65 70 75 80
   Glu Asp Ile Ala Thr Tyr Tyr Cys Gln Gln Tyr Ser Lys Leu Pro Trp
   85 90 95
   Thr Phe Gly Gly Gly Thr Lys Leu Glu Ile Lys
   100 105
<210> 23
   <211> 5
   <212> PRT
   <213> Artificial Sequence
<220>
   <223> GA201 HCDR1
<400> 23
   Asp Tyr Lys Ile His
   1 5
<210> 24
   <211> 17
   <212> PRT
   <213> Artificial Sequence
<220>
   <223> GA201 HCDR2
<400> 24
   Tyr Phe Asn Pro Asn Ser Gly Tyr Ser Thr Tyr Ala Gln Lys Phe Gln
   1               5                   10                  15
   Gly
<210> 25
   <211> 11
   <212> PRT
   <213> Artificial Sequence
<220>
   <223> GA201 HCDR3
<400> 25
   Leu Ser Pro Gly Gly Tyr Tyr Val Met Asp Ala
   1               5                   10
<210> 26
   <211> 120
   <212> PRT
   <213> Artificial Sequence
<220>
   <223> GA201 VH
<400> 26
<210> 27
   <211> 11
   <212> PRT
   <213> Artificial Sequence
<220>
   <223> GA201 LCDR1
<400> 27
   Arg Ala Ser Gln Gly Ile Asn Asn Tyr Leu Asn
   1 5 10
<210> 28
   <211> 7
   <212> PRT
   <213> Artificial Sequence
<220>
   <223> GA201 LCDR2
<400> 28
   Asn Thr Asn Asn Leu Gln Thr
   1 5
<210> 29
   <211> 8
   <212> PRT
   <213> Artificial Sequence
<220>
   <223> GA201 LCDR3
<400> 29
   Leu Gln His Asn Ser Phe Pro Thr
   1 5
<210> 30
   <211> 106
   <212> PRT
   <213> Artificial Sequence
<220>
   <223> GA201 VL
<400> 30
<210> 31
   <211> 5
   <212> PRT
   <213> Artificial Sequence
<220>
   <223> 3F2 HCDR1
<400> 31
   Ser Tyr Ala Met Ser
   1 5
<210> 32
   <211> 16
   <212> PRT
   <213> Artificial Sequence
<220>
   <223> 3F2 HCDR2
<400> 32
   Ala Ile Ser Gly Ser Gly Gly Ser Thr Tyr Tyr Ala Asp Ser Val Lys
   1 5 10 15
<210> 33
   <211> 8
   <212> PRT
   <213> Artificial Sequence
<220>
   <223> 3F2 HCDR3
<400> 33
   Tyr Cys Ala Lys Gly Trp Phe Gly
   1               5
<210> 34
   <211> 117
   <212> PRT
   <213> Artificial Sequence
<220>
   <223> 3F2 VH
<400> 34
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 35
   <211> 11
   <212> PRT
   <213> Artificial Sequence
<220>
   <223> 3F2 LCDR1
<400> 35
   Arg Ala Ser Gln Ser Val Thr Ser Ser Tyr Leu
   1               5                   10
<210> 36
   <211> 7
   <212> PRT
   <213> Artificial Sequence
<220>
   <223> 3F2 LCDR2
<400> 36
   Asn Val Gly Ser Arg Arg Ala
   1               5
<210> 37
   <211> 9
   <212> PRT
   <213> Artificial Sequence
<220>
   <223> 3F2 LCDR3
<400> 37
   Cys Gln Gln Gly Ile Met Leu Pro Pro
   1               5
<210> 38
   <211> 108
   <212> PRT
   <213> Artificial Sequence
<220>
   <223> 3F2 VL
<400> 38
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Thr Ser Ser
   20 25 30
   Tyr Leu Ala Trp Tyr Gln Gln Lys Pro Gly Gln Ala Pro Arg Leu Leu
   35 40 45
   Ile Asn Val Gly Ser Arg Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Ile Met Leu Pro
   85 90 95
   Pro Thr Phe Gly Gln Gly Thr Lys Val Glu Ile Lys
   100 105
<210> 39
   <211> 5
   <212> PRT
   <213> Artificial Sequence
<220>
   <223> CH1A1A HCDR1
<400> 39
   Glu Phe Gly Met Asn
   1 5
<210> 40
   <211> 17
   <212> PRT
   <213> Artificial Sequence
<220>
   <223> CH1A1A HCDR2
<400> 40
   Trp Ile Asn Thr Lys Thr Gly Glu Ala Thr Tyr Val Glu Glu Phe Lys
   1 5 10 15
   Gly
<210> 41
   <211> 12
   <212> PRT
   <213> Artificial Sequence
<220>
   <223> CH1A1A HCDR3
<400> 41
   Trp Asp Phe Ala Tyr Tyr Val Glu Ala Met Asp Tyr
   1               5                   10
<210> 42
   <211> 121
   <212> PRT
   <213> Artificial Sequence
<220>
   <223> CH1A1A VH
<400> 42
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser
           115                 120
<210> 43
   <211> 11
   <212> PRT
   <213> Artificial Sequence
<220>
   <223> CH1A1A LCDR1
<400> 43
   Lys Ala Ser Ala Ala Val Gly Thr Tyr Val Ala
   1               5                   10
<210> 44
   <211> 7
   <212> PRT
   <213> Artificial Sequence
<220>
   <223> CH1A1A LCDR2
<400> 44
   Ser Ala Ser Tyr Arg Lys Arg
   1               5
<210> 45
   <211> 10
   <212> PRT
   <213> Artificial Sequence
<220>
   <223> CH1A1A LCDR3
<400> 45
   His Gln Tyr Tyr Thr Tyr Pro Leu Phe Thr
   1               5                   10
<210> 46
   <211> 108
   <212> PRT
   <213> Artificial Sequence
<220>
   <223> CH1A1A VL
<400> 46
   Asp Ile Gln Met Thr Gln Ser Pro Ser Ser Leu Ser Ala Ser Val Gly
   1 5 10 15
   Asp Arg Val Thr Ile Thr Cys Lys Ala Ser Ala Ala Val Gly Thr Tyr
   20 25 30
   Val Ala Trp Tyr Gln Gln Lys Pro Gly Lys Ala Pro Lys Leu Leu Ile
   35 40 45
   Tyr Ser Ala Ser Tyr Arg Lys Arg Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys His Gln Tyr Tyr Thr Tyr Pro Leu
   85 90 95
   Phe Thr Phe Gly Gln Gly Thr Lys Leu Glu Ile Lys
   100 105
<210> 47
   <211> 10
   <212> PRT
   <213> Artificial Sequence
<220>
   <223> Anti-CD33 HCDR1
<400> 47
   Gly Tyr Thr Ile Thr Asp Ser Asn Ile His
   1 5 10
<210> 48
   <211> 13
   <212> PRT
   <213> Artificial Sequence
<220>
   <223> Anti-CD33 HCDR2
<400> 48
   Tyr Ile Tyr Pro Tyr Asn Gly Gly Thr Asp Tyr Asn Gln
   1 5 10
<210> 49
   <211> 7
   <212> PRT
   <213> Artificial Sequence
<220>
   <223> Anti-CD33 HCDR3
<400> 49
   Gly Asn Pro Trp Leu Ala Tyr
   1               5
<210> 50
   <211> 116
   <212> PRT
   <213> Artificial Sequence
<220>
   <223> Anti-CD33 VH
<400> 50
<210> 51
   <211> 15
   <212> PRT
   <213> Artificial Sequence
<220>
   <223> Anti-CD33 LCDR1
<400> 51
   Arg Ala Ser Glu Ser Leu Asp Asn Tyr Gly Ile Arg Phe Leu Thr
   1 5 10 15
<210> 52
   <211> 7
   <212> PRT
   <213> Artificial Sequence
<220>
   <223> Anti-CD33 LCDR2
<400> 52
   Ala Ala Ser Asn Gln Gly Ser
   1 5
<210> 53
   <211> 9
   <212> PRT
   <213> Artificial Sequence
<220>
   <223> Anti-CD33 LCDR3
<400> 53
   Gln Gln Thr Lys Glu Val Pro Trp Ser
   1 5
<210> 54
   <211> 111
   <212> PRT
   <213> Artificial Sequence
<220>
   <223> Anti-CD133 VL
<400> 54
<210> 55
   <211> 10
   <212> PRT
   <213> Artificial Sequence
<220>
   <223> anti-Her3 HCDR1
<400> 55
   Gly Tyr Thr Phe Arg Ser Ser Tyr Ile Ser
   1               5                   10
<210> 56
   <211> 17
   <212> PRT
   <213> Artificial Sequence
<220>
   <223> anti-Her3 HCDR2
<400> 56
   Trp Ile Tyr Ala Gly Thr Gly Ser Pro Ser Tyr Asn Gln Lys Leu Gln
   1               5                   10                  15
   Gly
<210> 57
   <211> 11
   <212> PRT
   <213> Artificial Sequence
<220>
   <223> anti-Her3 HCDR3
<400> 57
   His Arg Asp Tyr Tyr Ser Asn Ser Leu Thr Tyr
   1               5                   10
<210> 58
   <211> 120
   <212> PRT
   <213> Artificial Sequence
<220>
   <223> anti-Her3 VH
<400> 58
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Arg Ser Ser
               20                  25                  30
   Tyr Ile Ser Trp Val Arg Gln Ala Pro Gly Gln Gly Leu Glu Trp Met
           35                  40                  45
   Gly Trp Ile Tyr Ala Gly Thr Gly Ser Pro Ser Tyr Asn Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg His Arg Asp Tyr Tyr Ser Asn Ser Leu Thr Tyr Trp Gly Gln
               100                 105                 110
   Gly Thr Leu Val Thr Val Ser Ser
           115                 120
<210> 59
   <211> 16
   <212> PRT
   <213> Artificial Sequence
<220>
   <223> anti-Her3 LCDR1
<400> 59
   Lys Ser Ser Gln Ser Val Leu Asn Ser Gly Asn Gln Lys Asn Tyr Leu
   1               5                   10                  15
<210> 60
   <211> 7
   <212> PRT
   <213> Artificial Sequence
<220>
   <223> anti-Her3 LCDR2
<400> 60
   Trp Ala Ser Thr Arg Glu Ser
   1               5
<210> 61
   <211> 9
   <212> PRT
   <213> Artificial Sequence
<220>
   <223> anti-Her3 LCDR3
<400> 61
   Gln Ser Asp Tyr Ser Tyr Pro Tyr Thr
   1               5
<210> 62
   <211> 113
   <212> PRT
   <213> Artificial Sequence
<220>
   <223> anti-Her3 VL
<400> 62
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Asn Ser
               20                  25                  30
   Gly Asn Gln Lys Asn Tyr Leu Thr Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Ser
                   85                  90                  95
   Asp Tyr Ser Tyr Pro Tyr Thr Phe Gly Gln Gly Thr Lys Leu Glu Ile
               100                 105                 110
   Lys
<210> 63
   <211> 5
   <212> PRT
   <213> Artificial Sequence
<220>
   <223> 5D5 HCDR1
<400> 63
   Ser Tyr Trp Leu His
   1               5
<210> 64
   <211> 17
   <212> PRT
   <213> Artificial Sequence
<220>
   <223> 5D5 HCDR2
<400> 64
   Met Ile Asp Pro Ser Asn Ser Asp Thr Arg Phe Asn Pro Asn Phe Lys
   1               5                   10                  15
   Asp
<210> 65
   <211> 10
   <212> PRT
   <213> Artificial Sequence
<220>
   <223> 5D5 HCDR3
<400> 65
   Tyr Arg Ser Tyr Val Thr Pro Leu Asp Tyr
   1               5                   10
<210> 66
   <211> 119
   <212> PRT
   <213> Artificial Sequence
<220>
   <223> 5D5 VH
<400> 66
<210> 67
   <211> 17
   <212> PRT
   <213> Artificial Sequence
<220>
   <223> 5D5 LCDR1
<400> 67
   Lys Ser Ser Gln Ser Leu Leu Tyr Thr Ser Ser Gln Lys Asn Tyr Leu
   1 5 10 15
   Ala
<210> 68
   <211> 7
   <212> PRT
   <213> Artificial Sequence
<220>
   <223> 5D5 LCDR2
<400> 68
   Trp Ala Ser Thr Arg Glu Ser
   1 5
<210> 69
   <211> 9
   <212> PRT
   <213> Artificial Sequence
<220>
   <223> 5D5 LCDR3
<400> 69
   Gln Gln Tyr Tyr Ala Tyr Pro Trp Thr
   1 5
<210> 70
   <211> 113
   <212> PRT
   <213> Artificial Sequence
<220>
   <223> 5D5 VL
<400> 70
<210> 71
   <211> 227
   <212> PRT
   <213> Homo sapiens
<400> 71
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 72
   <211> 15
   <212> PRT
   <213> Artificial Sequence
<220>
   <223> Linker
<400> 72
   Glu Pro Lys Ser Cys Gly Gly Gly Gly Ser Gly Gly Gly Gly Ser
   1               5                   10                  15
<210> 73
   <211> 16
   <212> PRT
   <213> Artificial Sequence
<220>
   <223> Linker
<400> 73
   Glu Pro Lys Ser Cys Asp Gly Gly Gly Gly Ser Gly Gly Gly Gly Ser
   1               5                   10                  15
<210> 74
   <211> 19
   <212> PRT
   <213> Artificial Sequence
<220>
   <223> Leader 1
<400> 74
   Met Asp Trp Thr Trp Arg Ile Leu Phe Leu Val Ala Ala Ala Thr Gly
   1               5                   10                  15
   Ala His Ser
<210> 75
   <211> 22
   <212> PRT
   <213> Artificial Sequence
<220>
   <223> Leader 2
<400> 75
   Met Asp Met Arg Val Pro Ala Gln Leu Leu Gly Leu Leu Leu Leu Trp
   1               5                   10                  15
   Phe Pro Gly Ala Arg Cys
               20
<210> 76
   <211> 19
   <212> PRT
   <213> Artificial Sequence
<220>
   <223> Leader 3
<400> 76
   Met Gly Trp Ser Cys Ile Ile Leu Phe Leu Val Ala Thr Ala Thr Gly
   1 5 10 15
   Val His Ser
<210> 77
   <211> 5
   <212> PRT
   <213> Artificial Sequence
<220>
   <223> V9 HCDR1
<400> 77
   Gly Tyr Thr Met Asn
   1 5
<210> 78
   <211> 17
   <212> PRT
   <213> Artificial Sequence
<220>
   <223> V9 HCDR2
<400> 78
   Leu Ile Asn Pro Tyr Lys Gly Val Ser Thr Tyr Asn Gln Lys Phe Lys
   1 5 10 15
   Asp
<210> 79
   <211> 13
   <212> PRT
   <213> Artificial Sequence
<220>
   <223> V9 HCDR3
<400> 79
   Ser Gly Tyr Tyr Gly Asp Ser Asp Trp Tyr Phe Asp Val
   1 5 10
<210> 80
   <211> 122
   <212> PRT
   <213> Artificial Sequence
<220>
   <223> V9 VH
<400> 80
<210> 81
   <211> 11
   <212> PRT
   <213> Artificial Sequence
<220>
   <223> V9 LCDR1
<400> 81
   Arg Ala Ser Gln Asp Ile Arg Asn Tyr Leu Asn
   1 5 10
<210> 82
   <211> 7
   <212> PRT
   <213> Artificial Sequence
<220>
   <223> V9 LCDR2
<400> 82
   Tyr Thr Ser Arg Leu Glu Ser
   1               5
<210> 83
   <211> 9
   <212> PRT
   <213> Artificial Sequence
<220>
   <223> V9 LCDR3
<400> 83
   Gln Gln Gly Asn Thr Leu Pro Trp Thr
   1               5
<210> 84
   <211> 107
   <212> PRT
   <213> Artificial Sequence
<220>
   <223> V9 VL
<400> 84
<210> 85
   <211> 454
   <212> PRT
   <213> Artificial Sequence
<220>
   <223> V9(VH-CL)-LC007(VL-CL)
<400> 85
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Gln Gly Thr Leu Val Thr Val Ser Ser Ala Ser Val Ala Ala Pro
           115                 120                 125
   Ser Val Phe Ile Phe Pro Pro Ser Asp Glu Gln Leu Lys Ser Gly Thr
       130                 135                 140
   Ala Ser Val Val Cys Leu Leu Asn Asn Phe Tyr Pro Arg Glu Ala Lys
   145 150 155 160
   Val Gln Trp Lys Val Asp Asn Ala Leu Gln Ser Gly Asn Ser Gln Glu
   165 170 175
   Ser Val Thr Glu Gln Asp Ser Lys Asp Ser Thr Tyr Ser Leu Ser Ser
   180 185 190
   Thr Leu Thr Leu Ser Lys Ala Asp Tyr Glu Lys His Lys Val Tyr Ala
   195 200 205
   Cys Glu Val Thr His Gln Gly Leu Ser Ser Pro Val Thr Lys Ser Phe
   210 215 220
   Asn Arg Gly Glu Cys Asp Gly Gly Gly Gly Ser Gly Gly Gly Gly Ser
   225 230 235 240
   Asp Ile Val Leu Thr Gln Ser Pro Ser Ser Leu Ser Ala Ser Leu Gly
   245 250 255
   Asp Arg Val Thr Ile Ser Cys Ser Ala Ser Gln Gly Ile Arg Asn Tyr
   260 265 270
   Leu Asn Trp Tyr Gln Gln Arg Pro Asp Gly Thr Val Lys Leu Leu Ile
   275 280 285
   Tyr Tyr Thr Ser Ser Leu His Ser Gly Val Pro Ser Arg Phe Ser Gly
   290 295 300
   Ser Gly Ser Gly Thr Asp Tyr Ser Leu Thr Ile Ser Asn Leu Glu Pro
   305 310 315 320
   Glu Asp Ile Ala Thr Tyr Tyr Cys Gln Gln Tyr Ser Lys Leu Pro Trp
   325 330 335
   Thr Phe Gly Gly Gly Thr Lys Leu Glu Ile Lys Arg Thr Val Ala Ala
   340 345 350
   Pro Ser Val Phe Ile Phe Pro Pro Ser Asp Glu Gln Leu Lys Ser Gly
   355 360 365
   Thr Ala Ser Val Val Cys Leu Leu Asn Asn Phe Tyr Pro Arg Glu Ala
   370 375 380
   Lys Val Gln Trp Lys Val Asp Asn Ala Leu Gln Ser Gly Asn Ser Gln
   385 390 395 400
   Glu Ser Val Thr Glu Gln Asp Ser Lys Asp Ser Thr Tyr Ser Leu Ser
   405 410 415
   Ser Thr Leu Thr Leu Ser Lys Ala Asp Tyr Glu Lys His Lys Val Tyr
   420 425 430
   Ala Cys Glu Val Thr His Gln Gly Leu Ser Ser Pro Val Thr Lys Ser
   435 440 445
   Phe Asn Arg Gly Glu Cys
   450
<210> 86
   <211> 227
   <212> PRT
   <213> Artificial Sequence
<220>
   <223> Fc(knob) P329G LALA
<400> 86
   Asp Lys Thr His Thr Cys Pro Pro Cys Pro Ala Pro Glu Ala Ala Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Gly Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Cys Arg Asp Glu Leu Thr Lys Asn Gln Val Ser
       130                 135                 140
   Leu Trp Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 87
   <211> 212
   <212> PRT
   <213> Artificial Sequence
<220>
   <223> V9(VL-CH1)
<400> 87
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
           130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys
       210
<210> 88
   <211> 456
   <212> PRT
   <213> Artificial Sequence
<220>
   <223> V9(VH-CL)-Fc(knob) P329G LALA
<400> 88
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Gln Gly Thr Leu Val Thr Val Ser Ser Ala Ser Val Ala Ala Pro
           115                 120                 125
   Ser Val Phe Ile Phe Pro Pro Ser Asp Glu Gln Leu Lys Ser Gly Thr
       130                 135                 140
   Ala Ser Val Val Cys Leu Leu Asn Asn Phe Tyr Pro Arg Glu Ala Lys
   145                 150                 155                 160
   Val Gln Trp Lys Val Asp Asn Ala Leu Gln Ser Gly Asn Ser Gln Glu
                   165                 170                 175
   Ser Val Thr Glu Gln Asp Ser Lys Asp Ser Thr Tyr Ser Leu Ser Ser
               180                 185                 190
   Thr Leu Thr Leu Ser Lys Ala Asp Tyr Glu Lys His Lys Val Tyr Ala
           195                 200                 205
   Cys Glu Val Thr His Gln Gly Leu Ser Ser Pro Val Thr Lys Ser Phe
       210                 215                 220
   Asn Arg Gly Glu Cys Asp Lys Thr His Thr Cys Pro Pro Cys Pro Ala
   225                 230                 235                 240
   Pro Glu Ala Ala Gly Gly Pro Ser Val Phe Leu Phe Pro Pro Lys Pro
                   245                 250                 255
   Lys Asp Thr Leu Met Ile Ser Arg Thr Pro Glu Val Thr Cys Val Val
               260                 265                 270
   Val Asp Val Ser His Glu Asp Pro Glu Val Lys Phe Asn Trp Tyr Val
           275                 280                 285
   Asp Gly Val Glu Val His Asn Ala Lys Thr Lys Pro Arg Glu Glu Gln
       290                 295                 300
   Tyr Asn Ser Thr Tyr Arg Val Val Ser Val Leu Thr Val Leu His Gln
   305                 310                 315                 320
   Asp Trp Leu Asn Gly Lys Glu Tyr Lys Cys Lys Val Ser Asn Lys Ala
                   325                 330                 335
   Leu Gly Ala Pro Ile Glu Lys Thr Ile Ser Lys Ala Lys Gly Gln Pro
               340                 345                 350
   Arg Glu Pro Gln Val Tyr Thr Leu Pro Pro Cys Arg Asp Glu Leu Thr
           355                 360                 365
   Lys Asn Gln Val Ser Leu Trp Cys Leu Val Lys Gly Phe Tyr Pro Ser
       370                 375                 380
   Asp Ile Ala Val Glu Trp Glu Ser Asn Gly Gln Pro Glu Asn Asn Tyr
   385                 390                 395                 400
   Lys Thr Thr Pro Pro Val Leu Asp Ser Asp Gly Ser Phe Phe Leu Tyr
                   405                 410                 415
   Ser Lys Leu Thr Val Asp Lys Ser Arg Trp Gln Gln Gly Asn Val Phe
               420                 425                 430
   Ser Cys Ser Val Met His Glu Ala Leu His Asn His Tyr Thr Gln Lys
           435                 440                 445
   Ser Leu Ser Leu Ser Pro Gly Lys
       450                 455
<210> 89
   <211> 442
   <212> PRT
   <213> Artificial Sequence
<220>
   <223> LC007(VH-CH1)-Fc(hole) P329G LALA
<400> 89
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
   180 185 190
   Tyr Ile Cys Asn Val Asn His Lys Pro Ser Asn Thr Lys Val Asp Lys
   195 200 205
   Lys Val Glu Pro Lys Ser Cys Asp Lys Thr His Thr Cys Pro Pro Cys
   210 215 220
   Pro Ala Pro Glu Ala Ala Gly Gly Pro Ser Val Phe Leu Phe Pro Pro
   225 230 235 240
   Lys Pro Lys Asp Thr Leu Met Ile Ser Arg Thr Pro Glu Val Thr Cys
   245 250 255
   Val Val Val Asp Val Ser His Glu Asp Pro Glu Val Lys Phe Asn Trp
   260 265 270
   Tyr Val Asp Gly Val Glu Val His Asn Ala Lys Thr Lys Pro Arg Glu
   275 280 285
   Glu Gln Tyr Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Ala Leu Gly Ala Pro Ile Glu Lys Thr Ile Ser Lys Ala Lys Gly
   325 330 335
   Gln Pro Arg Glu Pro Gln Val Cys Thr Leu Pro Pro Ser Arg Asp Glu
   340 345 350
   Leu Thr Lys Asn Gln Val Ser Leu Ser Cys Ala Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Val Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 90
   <211> 682
   <212> PRT
   <213> Artificial Sequence
<220>
   <223> LC007(VH-CH1)-V9(VH-CL)-Fc(knob) P329G LALA
<400> 90
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Gly Gly Gly Gly Ser Gly Gly Gly
       210                 215                 220
   Gly Ser Glu Val Gln Leu Val Glu Ser Gly Gly Gly Leu Val Gln Pro
   225                 230                 235                 240
   Gly Gly Ser Leu Arg Leu Ser Cys Ala Ala Ser Gly Tyr Ser Phe Thr
                   245                 250                 255
   Gly Tyr Thr Met Asn Trp Val Arg Gln Ala Pro Gly Lys Gly Leu Glu
               260                 265                 270
   Trp Val Ala Leu Ile Asn Pro Tyr Lys Gly Val Ser Thr Tyr Asn Gln
           275                 280                 285
   Lys Phe Lys Asp Arg Phe Thr Ile Ser Val Asp Lys Ser Lys Asn Thr
       290                 295                 300
   Ala Tyr Leu Gln Met Asn Ser Leu Arg Ala Glu Asp Thr Ala Val Tyr
   305                 310                 315                 320
   Tyr Cys Ala Arg Ser Gly Tyr Tyr Gly Asp Ser Asp Trp Tyr Phe Asp
                   325                 330                 335
   Val Trp Gly Gln Gly Thr Leu Val Thr Val Ser Ser Ala Ser Val Ala
               340                 345                 350
   Ala Pro Ser Val Phe Ile Phe Pro Pro Ser Asp Glu Gln Leu Lys Ser
           355                 360                 365
   Gly Thr Ala Ser Val Val Cys Leu Leu Asn Asn Phe Tyr Pro Arg Glu
       370                 375                 380
   Ala Lys Val Gln Trp Lys Val Asp Asn Ala Leu Gln Ser Gly Asn Ser
   385                 390                 395                 400
   Gln Glu Ser Val Thr Glu Gln Asp Ser Lys Asp Ser Thr Tyr Ser Leu
                   405                 410                 415
   Ser Ser Thr Leu Thr Leu Ser Lys Ala Asp Tyr Glu Lys His Lys Val
               420                 425                 430
   Tyr Ala Cys Glu Val Thr His Gln Gly Leu Ser Ser Pro Val Thr Lys
           435                 440                 445
   Ser Phe Asn Arg Gly Glu Cys Asp Lys Thr His Thr Cys Pro Pro Cys
       450                 455                 460
   Pro Ala Pro Glu Ala Ala Gly Gly Pro Ser Val Phe Leu Phe Pro Pro
   465                 470                 475                 480
   Lys Pro Lys Asp Thr Leu Met Ile Ser Arg Thr Pro Glu Val Thr Cys
                   485                 490                 495
   Val Val Val Asp Val Ser His Glu Asp Pro Glu Val Lys Phe Asn Trp
               500                 505                 510
   Tyr Val Asp Gly Val Glu Val His Asn Ala Lys Thr Lys Pro Arg Glu
           515                 520                 525
   Glu Gln Tyr Asn Ser Thr Tyr Arg Val Val Ser Val Leu Thr Val Leu
       530                 535                 540
   His Gln Asp Trp Leu Asn Gly Lys Glu Tyr Lys Cys Lys Val Ser Asn
   545                 550                 555                 560
   Lys Ala Leu Gly Ala Pro Ile Glu Lys Thr Ile Ser Lys Ala Lys Gly
                   565                 570                 575
   Gln Pro Arg Glu Pro Gln Val Tyr Thr Leu Pro Pro Cys Arg Asp Glu
               580                 585                 590
   Leu Thr Lys Asn Gln Val Ser Leu Trp Cys Leu Val Lys Gly Phe Tyr
           595                 600                 605
   Pro Ser Asp Ile Ala Val Glu Trp Glu Ser Asn Gly Gln Pro Glu Asn
       610                 615                 620
   Asn Tyr Lys Thr Thr Pro Pro Val Leu Asp Ser Asp Gly Ser Phe Phe
   625                 630                 635                 640
   Leu Tyr Ser Lys Leu Thr Val Asp Lys Ser Arg Trp Gln Gln Gly Asn
                   645                 650                 655
   Val Phe Ser Cys Ser Val Met His Glu Ala Leu His Asn His Tyr Thr
               660                 665                 670
   Gln Lys Ser Leu Ser Leu Ser Pro Gly Lys
           675                 680
<210> 91
   <211> 214
   <212> PRT
   <213> Artificial Sequence
<220>
   <223> M4-3 ML2(VL-CL)
<400> 91
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Lys Leu Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 92
   <211> 664
   <212> PRT
   <213> Artificial Sequence
<220>
   <223> V9(VL-CH1)-M4-3 ML2(VH-CH1)-Fc(knob) P329G LALA
<400> 92
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Gly Gly Gly Gly Ser Gly Gly Gly Gly Ser Gln Val
       210                 215                 220
   Gln Leu Gln Glu Ser Gly Pro Gly Leu Val Lys Pro Ser Gln Thr Leu
   225 230 235 240
   Ser Leu Thr Cys Thr Val Ser Gly Gly Ser Ile Thr Ser Gly Tyr Tyr
   245 250 255
   Trp Asn Trp Ile Arg Gln His Pro Gly Lys Gly Leu Glu Trp Ile Gly
   260 265 270
   Tyr Ile Thr Tyr Asp Gly Ser Asn Asn Tyr Asn Pro Ser Leu Lys Ser
   275 280 285
   Arg Val Thr Ile Ser Arg Asp Thr Ser Lys Asn Gln Phe Ser Leu Lys
   290 295 300
   Leu Ser Ser Val Thr Ala Ala Asp Thr Ala Val Tyr Tyr Cys Ala Asp
   305 310 315 320
   Phe Asp Tyr Trp Gly Gln Gly Thr Leu Val Thr Val Ser Ser Ala Ser
   325 330 335
   Thr Lys Gly Pro Ser Val Phe Pro Leu Ala Pro Ser Ser Lys Ser Thr
   340 345 350
   Ser Gly Gly Thr Ala Ala Leu Gly Cys Leu Val Lys Asp Tyr Phe Pro
   355 360 365
   Glu Pro Val Thr Val Ser Trp Asn Ser Gly Ala Leu Thr Ser Gly Val
   370 375 380
   His Thr Phe Pro Ala Val Leu Gln Ser Ser Gly Leu Tyr Ser Leu Ser
   385 390 395 400
   Ser Val Val Thr Val Pro Ser Ser Ser Leu Gly Thr Gln Thr Tyr Ile
   405 410 415
   Cys Asn Val Asn His Lys Pro Ser Asn Thr Lys Val Asp Lys Lys Val
   420 425 430
   Glu Pro Lys Ser Cys Asp Lys Thr His Thr Cys Pro Pro Cys Pro Ala
   435 440 445
   Pro Glu Ala Ala Gly Gly Pro Ser Val Phe Leu Phe Pro Pro Lys Pro
   450 455 460
   Lys Asp Thr Leu Met Ile Ser Arg Thr Pro Glu Val Thr Cys Val Val
   465 470 475 480
   Val Asp Val Ser His Glu Asp Pro Glu Val Lys Phe Asn Trp Tyr Val
   485 490 495
   Asp Gly Val Glu Val His Asn Ala Lys Thr Lys Pro Arg Glu Glu Gln
   500 505 510
   Tyr Asn Ser Thr Tyr Arg Val Val Ser Val Leu Thr Val Leu His Gln
   515 520 525
   Asp Trp Leu Asn Gly Lys Glu Tyr Lys Cys Lys Val Ser Asn Lys Ala
   530 535 540
   Leu Gly Ala Pro Ile Glu Lys Thr Ile Ser Lys Ala Lys Gly Gln Pro
   545 550 555 560
   Arg Glu Pro Gln Val Tyr Thr Leu Pro Pro Cys Arg Asp Glu Leu Thr
   565 570 575
   Lys Asn Gln Val Ser Leu Trp Cys Leu Val Lys Gly Phe Tyr Pro Ser
   580 585 590
   Asp Ile Ala Val Glu Trp Glu Ser Asn Gly Gln Pro Glu Asn Asn Tyr
   595 600 605
   Lys Thr Thr Pro Pro Val Leu Asp Ser Asp Gly Ser Phe Phe Leu Tyr
   610 615 620
   Ser Lys Leu Thr Val Asp Lys Ser Arg Trp Gln Gln Gly Asn Val Phe
   625 630 635 640
   Ser Cys Ser Val Met His Glu Ala Leu His Asn His Tyr Thr Gln Lys
   645 650 655
   Ser Leu Ser Leu Ser Pro Gly Lys
   660
<210> 93
   <211> 442
   <212> PRT
   <213> Artificial Sequence
<220>
   <223> M4-3 ML2(VH-CH1)-Fc(hole) P329G LALA
<400> 93
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Ala Leu Gly Ala Pro Ile Glu Lys Thr Ile Ser Lys Ala Lys Gly
   325 330 335
   Gln Pro Arg Glu Pro Gln Val Cys Thr Leu Pro Pro Ser Arg Asp Glu
   340 345 350
   Leu Thr Lys Asn Gln Val Ser Leu Ser Cys Ala Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Val Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 94
   <211> 681
   <212> PRT
   <213> Artificial Sequence
<220>
   <223> V9(VH-CL)-M4-3 ML2(VH-CH1)-Fc(knob) P329G LALA
<400> 94
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Ser Gly Tyr Tyr Gly Asp Ser Asp Trp Tyr Phe Asp Val Trp
   100 105 110
   Gly Gln Gly Thr Leu Val Thr Val Ser Ser Ala Ser Val Ala Ala Pro
   115 120 125
   Ser Val Phe Ile Phe Pro Pro Ser Asp Glu Gln Leu Lys Ser Gly Thr
   130 135 140
   Ala Ser Val Val Cys Leu Leu Asn Asn Phe Tyr Pro Arg Glu Ala Lys
   145 150 155 160
   Val Gln Trp Lys Val Asp Asn Ala Leu Gln Ser Gly Asn Ser Gln Glu
   165 170 175
   Ser Val Thr Glu Gln Asp Ser Lys Asp Ser Thr Tyr Ser Leu Ser Ser
   180 185 190
   Thr Leu Thr Leu Ser Lys Ala Asp Tyr Glu Lys His Lys Val Tyr Ala
   195 200 205
   Cys Glu Val Thr His Gln Gly Leu Ser Ser Pro Val Thr Lys Ser Phe
   210 215 220
   Asn Arg Gly Glu Cys Gly Gly Gly Gly Ser Gly Gly Gly Gly Ser Gln
   225 230 235 240
   Val Gln Leu Gln Glu Ser Gly Pro Gly Leu Val Lys Pro Ser Gln Thr
   245 250 255
   Leu Ser Leu Thr Cys Thr Val Ser Gly Gly Ser Ile Thr Ser Gly Tyr
   260 265 270
   Tyr Trp Asn Trp Ile Arg Gln His Pro Gly Lys Gly Leu Glu Trp Ile
   275 280 285
   Gly Tyr Ile Thr Tyr Asp Gly Ser Asn Asn Tyr Asn Pro Ser Leu Lys
   290 295 300
   Ser Arg Val Thr Ile Ser Arg Asp Thr Ser Lys Asn Gln Phe Ser Leu
   305 310 315 320
   Lys Leu Ser Ser Val Thr Ala Ala Asp Thr Ala Val Tyr Tyr Cys Ala
   325 330 335
   Asp Phe Asp Tyr Trp Gly Gln Gly Thr Leu Val Thr Val Ser Ser Ala
   340 345 350
   Ser Thr Lys Gly Pro Ser Val Phe Pro Leu Ala Pro Ser Ser Lys Ser
   355 360 365
   Thr Ser Gly Gly Thr Ala Ala Leu Gly Cys Leu Val Lys Asp Tyr Phe
   370 375 380
   Pro Glu Pro Val Thr Val Ser Trp Asn Ser Gly Ala Leu Thr Ser Gly
   385 390 395 400
   Val His Thr Phe Pro Ala Val Leu Gln Ser Ser Gly Leu Tyr Ser Leu
   405 410 415
   Ser Ser Val Val Thr Val Pro Ser Ser Ser Leu Gly Thr Gln Thr Tyr
   420 425 430
   Ile Cys Asn Val Asn His Lys Pro Ser Asn Thr Lys Val Asp Lys Lys
   435 440 445
   Val Glu Pro Lys Ser Cys Asp Lys Thr His Thr Cys Pro Pro Cys Pro
   450 455 460
   Ala Pro Glu Ala Ala Gly Gly Pro Ser Val Phe Leu Phe Pro Pro Lys
   465 470 475 480
   Pro Lys Asp Thr Leu Met Ile Ser Arg Thr Pro Glu Val Thr Cys Val
   485 490 495
   Val Val Asp Val Ser His Glu Asp Pro Glu Val Lys Phe Asn Trp Tyr
   500 505 510
   Val Asp Gly Val Glu Val His Asn Ala Lys Thr Lys Pro Arg Glu Glu
   515 520 525
   Gln Tyr Asn Ser Thr Tyr Arg Val Val Ser Val Leu Thr Val Leu His
   530 535 540
   Gln Asp Trp Leu Asn Gly Lys Glu Tyr Lys Cys Lys Val Ser Asn Lys
   545 550 555 560
   Ala Leu Gly Ala Pro Ile Glu Lys Thr Ile Ser Lys Ala Lys Gly Gln
   565 570 575
   Pro Arg Glu Pro Gln Val Tyr Thr Leu Pro Pro Cys Arg Asp Glu Leu
   580 585 590
   Thr Lys Asn Gln Val Ser Leu Trp Cys Leu Val Lys Gly Phe Tyr Pro
   595 600 605
   Ser Asp Ile Ala Val Glu Trp Glu Ser Asn Gly Gln Pro Glu Asn Asn
   610 615 620
   Tyr Lys Thr Thr Pro Pro Val Leu Asp Ser Asp Gly Ser Phe Phe Leu
   625 630 635 640
   Tyr Ser Lys Leu Thr Val Asp Lys Ser Arg Trp Gln Gln Gly Asn Val
   645 650 655
   Phe Ser Cys Ser Val Met His Glu Ala Leu His Asn His Tyr Thr Gln
   660 665 670
   Lys Ser Leu Ser Leu Ser Pro Gly Lys
   675 680
<210> 95
   <211> 690
   <212> PRT
   <213> Artificial Sequence
<220>
   <223> CH1A1A(VH-CH1)- V9(VH-CL)-Fc(knob) P329G LALA
<400> 95
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Gly Gly Gly Gly Ser Gly Gly Gly Gly Ser Glu Val Gln Leu Val Glu
   225 230 235 240
   Ser Gly Gly Gly Leu Val Gln Pro Gly Gly Ser Leu Arg Leu Ser Cys
   245 250 255
   Ala Ala Ser Gly Tyr Ser Phe Thr Gly Tyr Thr Met Asn Trp Val Arg
   260 265 270
   Gln Ala Pro Gly Lys Gly Leu Glu Trp Val Ala Leu Ile Asn Pro Tyr
   275 280 285
   Lys Gly Val Ser Thr Tyr Asn Gln Lys Phe Lys Asp Arg Phe Thr Ile
   290 295 300
   Ser Val Asp Lys Ser Lys Asn Thr Ala Tyr Leu Gln Met Asn Ser Leu
   305 310 315 320
   Arg Ala Glu Asp Thr Ala Val Tyr Tyr Cys Ala Arg Ser Gly Tyr Tyr
   325 330 335
   Gly Asp Ser Asp Trp Tyr Phe Asp Val Trp Gly Gln Gly Thr Leu Val
   340 345 350
   Thr Val Ser Ser Ala Ser Val Ala Ala Pro Ser Val Phe Ile Phe Pro
   355 360 365
   Pro Ser Asp Glu Gln Leu Lys Ser Gly Thr Ala Ser Val Val Cys Leu
   370 375 380
   Leu Asn Asn Phe Tyr Pro Arg Glu Ala Lys Val Gln Trp Lys Val Asp
   385 390 395 400
   Asn Ala Leu Gln Ser Gly Asn Ser Gln Glu Ser Val Thr Glu Gln Asp
   405 410 415
   Ser Lys Asp Ser Thr Tyr Ser Leu Ser Ser Thr Leu Thr Leu Ser Lys
   420 425 430
   Ala Asp Tyr Glu Lys His Lys Val Tyr Ala Cys Glu Val Thr His Gln
   435 440 445
   Gly Leu Ser Ser Pro Val Thr Lys Ser Phe Asn Arg Gly Glu Cys Asp
   450 455 460
   Lys Thr His Thr Cys Pro Pro Cys Pro Ala Pro Glu Ala Ala Gly Gly
   465 470 475 480
   Pro Ser Val Phe Leu Phe Pro Pro Lys Pro Lys Asp Thr Leu Met Ile
   485 490 495
   Ser Arg Thr Pro Glu Val Thr Cys Val Val Val Asp Val Ser His Glu
   500 505 510
   Asp Pro Glu Val Lys Phe Asn Trp Tyr Val Asp Gly Val Glu Val His
   515 520 525
   Asn Ala Lys Thr Lys Pro Arg Glu Glu Gln Tyr Asn Ser Thr Tyr Arg
   530 535 540
   Val Val Ser Val Leu Thr Val Leu His Gln Asp Trp Leu Asn Gly Lys
   545 550 555 560
   Glu Tyr Lys Cys Lys Val Ser Asn Lys Ala Leu Gly Ala Pro Ile Glu
   565 570 575
   Lys Thr Ile Ser Lys Ala Lys Gly Gln Pro Arg Glu Pro Gln Val Tyr
   580 585 590
   Thr Leu Pro Pro Cys Arg Asp Glu Leu Thr Lys Asn Gln Val Ser Leu
   595 600 605
   Trp Cys Leu Val Lys Gly Phe Tyr Pro Ser Asp Ile Ala Val Glu Trp
   610 615 620
   Glu Ser Asn Gly Gln Pro Glu Asn Asn Tyr Lys Thr Thr Pro Pro Val
   625 630 635 640
   Leu Asp Ser Asp Gly Ser Phe Phe Leu Tyr Ser Lys Leu Thr Val Asp
   645 650 655
   Lys Ser Arg Trp Gln Gln Gly Asn Val Phe Ser Cys Ser Val Met His
   660 665 670
   Glu Ala Leu His Asn His Tyr Thr Gln Lys Ser Leu Ser Leu Ser Pro
   675 680 685
   Gly Lys
   690
<210> 96
   <211> 11
   <212> PRT
   <213> Artificial Sequence
<220>
   <223> M4-3 ML2 HCDR1
<400> 96
   Gly Gly Ser Ile Thr Ser Gly Tyr Tyr Trp Asn
   1 5 10
<210> 97
   <211> 16
   <212> PRT
   <213> Artificial Sequence
<220>
   <223> M4-3 ML2 HCDR2
<400> 97
   Tyr Ile Thr Tyr Asp Gly Ser Asn Asn Tyr Asn Pro Ser Leu Lys Ser
   1 5 10 15
<210> 98
   <211> 3
   <212> PRT
   <213> Artificial Sequence
<220>
   <223> M4-3 ML2 HCDR3
<400> 98
   Phe Asp Tyr
   1
<210> 99
   <211> 112
   <212> PRT
   <213> Artificial Sequence
<220>
   <223> M4-3 ML2 VH
<400> 99
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
<210> 100
   <211> 11
   <212> PRT
   <213> Artificial Sequence
<220>
   <223> M4-3 ML2 LCDR1
<400> 100
   Arg Ala Ser Gln Gly Ile Arg Asn Tyr Leu Asn
   1               5                   10
<210> 101
   <211> 7
   <212> PRT
   <213> Artificial Sequence
<220>
   <223> M4-3 ML2 LCDR2
<400> 101
   Tyr Thr Ser Ser Leu His Ser
   1               5
<210> 102
   <211> 9
   <212> PRT
   <213> Artificial Sequence
<220>
   <223> M4-3 ML2 LCDR3
<400> 102
   Gln Gln Tyr Ser Lys Leu Pro Trp Thr
   1 5
<210> 103
   <211> 107
   <212> PRT
   <213> Artificial Sequence
<220>
   <223> M4-3 ML2 VL
<400> 103
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Tyr
   20 25 30
   Leu Asn Trp Tyr Gln Gln Lys Pro Gly Lys Ala Pro Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Lys Leu Pro Trp
   85 90 95
   Thr Phe Gly Gln Gly Thr Lys Val Glu Ile Lys
   100 105
<210> 104
   <211> 5
   <212> PRT
   <213> Artificial Sequence
<220>
   <223> anti-CD3 HCDR1
<400> 104
   Thr Tyr Ala Met Asn
   1 5
<210> 105
   <211> 19
   <212> PRT
   <213> Artificial Sequence
<220>
   <223> anti-CD3 HCDR2
<400> 105
   Arg Ile Arg Ser Lys Tyr Asn Asn Tyr Ala Thr Tyr Tyr Ala Asp Ser
   1 5 10 15
   Val Lys Asp
<210> 106
   <211> 14
   <212> PRT
   <213> Artificial Sequence
<220>
   <223> anti-CD3 HCDR3
<400> 106
   His Gly Asn Phe Gly Asn Ser Tyr Val Ser Trp Phe Ala Tyr
   1 5 10
<210> 107
   <211> 125
   <212> PRT
   <213> Artificial Sequence
<220>
   <223> anti-CD3 VH
<400> 107
   Glu Val Lys Leu Leu Glu Ser Gly Gly Gly Leu Val Gln Pro Lys Gly
   1 5 10 15
   Ser Leu Lys Leu Ser Cys Ala Ala Ser Gly Phe Thr Phe Asn Thr Tyr
   20 25 30
   Ala Met Asn Trp Val Arg Gln Ala Pro Gly Lys Gly Leu Glu Trp Val
   35 40 45
   Ala Arg Ile Arg Ser Lys Tyr Asn Asn Tyr Ala Thr Tyr Tyr Ala Asp
   50 55 60
   Ser Val Lys Asp Arg Phe Thr Ile Ser Arg Asp Asp Ser Gln Ser Ile
   65 70 75 80
   Leu Tyr Leu Gln Met Asn Asn Leu Lys Thr Glu Asp Thr Ala Met Tyr
   85 90 95
   Tyr Cys Val Arg His Gly Asn Phe Gly Asn Ser Tyr Val Ser Trp Phe
   100 105 110
   Ala Tyr Trp Gly Gln Gly Thr Leu Val Thr Val Ser Ala
   115 120 125
<210> 108
   <211> 14
   <212> PRT
   <213> Artificial Sequence
<220>
   <223> anti-CD3 LCDR1
<400> 108
   Arg Ser Ser Thr Gly Ala Val Thr Thr Ser Asn Tyr Ala Asn
   1 5 10
<210> 109
   <211> 7
   <212> PRT
   <213> Artificial Sequence
<220>
   <223> anti-CD3 LCDR2
<400> 109
   Gly Thr Asn Lys Arg Ala Pro
   1 5
<210> 110
   <211> 9
   <212> PRT
   <213> Artificial Sequence
<220>
   <223> anti-CD3 LCDR3
<400> 110
   Ala Leu Trp Tyr Ser Asn Leu Trp Val
   1 5
<210> 111
   <211> 109
   <212> PRT
   <213> Artificial Sequence
<220>
   <223> anti-CD3 VL
<400> 111
   Gln Ala Val Val Thr Gln Glu Ser Ala Leu Thr Thr Ser Pro Gly Glu
   1 5 10 15
   Thr Val Thr Leu Thr Cys Arg Ser Ser Thr Gly Ala Val Thr Thr Ser
   20 25 30
   Asn Tyr Ala Asn Trp Val Gln Glu Lys Pro Asp His Leu Phe Thr Gly
   35 40 45
   Leu Ile Gly Gly Thr Asn Lys Arg Ala Pro Gly Val Pro Ala Arg Phe
   50 55 60
   Ser Gly Ser Leu Ile Gly Asp Lys Ala Ala Leu Thr Ile Thr Gly Ala
   65 70 75 80
   Gln Thr Glu Asp Glu Ala Ile Tyr Phe Cys Ala Leu Trp Tyr Ser Asn
   85 90 95
   Leu Trp Val Phe Gly Gly Gly Thr Lys Leu Thr Val Leu
   100 105

## Description

### Field of the Invention

The present invention generally relates to bispecific antigen binding molecules. In addition, the present invention relates to polynucleotides encoding such bispecific antigen binding molecules, and to such bispecific antigen binding molecules for use in the treatment of disease.

### Background

Bi- or multispecific antibodies capable of binding two or more antigens are known in the art.

Such multispecific binding proteins can be generated by hybridoma cell fusion, chemical conjugation or recombinant DNA techniques.

Bispecific antibodies are of great interest for therapeutic applications, as they allow the simultaneous binding and inactivation of two or more target antigens, thereby obviating the need for combination therapies. Another promising application of bispecific antibodies is as engagers of immune effector cells e.g. for cellular cancer immunotherapy. For this purpose, bispecific antibodies are designed which bind to a surface antigen on target cells and, for example, to an activating component of the T cell receptor (TCR) complex. The simultaneous binding of such an antibody to both of its targets will force a temporary interaction between target cell and T cell, causing activation of any cytotoxic T lymphocyte (CTL) and subsequent lysis of the target cell.

Hence, the immune response is re-directed to the target cells, independently of peptide antigen presentation by the target cell or the specificity of the T cell as required for normal MHC-restricted activation of CTLs. In this context it is important that CTLs are only activated when a target cell is presenting the bispecific antibody to them, i.e. when the immunological synapse is mimicked, and not simply upon binding of the antibody to the T cell antigen.

A variety of recombinant multispecific antibody formats have been developed in the recent past, including, for example, tetravalent IgG-single-chain variable fragment (scFv) fusions (see e.g. Coloma & Morrison, Nat Biotechnol 15, 159-163 (1997)), tetravalent IgG-like dual-variable domain antibodies (Wu et al., Nat Biotechnol 25, 1290-1297 (2007)), or bivalent rat/mouse hybrid bispecific IgGs (see e.g. Lindhofer et al., J Immunol 155, 219-225 (1995)).

Also several bispecific formats wherein the antibody core structure (IgA, IgD, IgE, IgG or IgM) is no longer retained have been made. Examples include diabodies (see e.g. Holliger et al., Proc Natl Acad Sci USA 90, 6444-6448 (1995)), tandem scFv molecules (see e.g. Bargou et al., Science 321, 974-977 (2008)), and various derivatives thereof.

The multitude of formats that are being developed shows the great potential attributed to bispecific antibodies. The task of generating bispecific antibodies suitable for a particular purpose is, however, by no means trivial and subject to a number of considerations. For example, the valency and geometry of the antibody needs to be appropriately chosen, depending on the characteristics of the target antigens and the intended effect. As for all therapeutic antibodies, efficacy and toxicity have to be balanced, which requires i.a. minimization of immunogenicity and optimization of pharmacokinetic properties of the antibody. Also, the desirablility of Fc-mediated effects has to be considered. Furthermore, the production of bispecific antibody constructs at a clinically sufficient quantity and purity poses a major challenge, as the homodimerization of antibody heavy chains and/or the mispairing of antibody heavy and light chains of different specificities upon co-expression decreases the yield of the correctly assembled construct and results in a number of non-functional side products from which the desired bispecific antibody may be difficult to separate.

Given the increasing number of possible applications of bispecific antibodies, and the difficulties and disadvantages associated with currently available bispecific antibodies, there remains a need for novel, improved formats of such molecules.

### Summary of the Invention

In a first aspect, the invention provides a bispecific antigen binding molecule, comprising a first Fab fragment which specifically binds to a first antigen, a second Fab fragment which specifically binds to a second antigen, and an Fc domain composed of a first and a second subunit capable of stable association; wherein
a) the bispecific antigen binding molecule provides monovalent binding to the first and/or the second antigen,
b) (i) the first Fab fragment is fused at its C-terminus to the N-terminus of the second Fab fragment, which is in turn fused at its C-terminus to the N-terminus of the first Fc domain subunit, or (ii) the second Fab fragment is fused at its C-terminus to the N-terminus of the first Fab fragment, which is in turn fused at its C-terminus to the N-terminus of the first Fc domain subunit,
c) in the first and/or the second Fab fragment one of the following replacements is made: (i) the variable domains VL and VH are replaced by each other, or (ii) the constant domains CL and CH1 are replaced by each other,
   provided that not the same replacement is made in the first and the second Fab fragment, and
d) the bispecific antigen binding molecule does not comprise a single chain Fab fragment.

In one embodiment, the first Fab fragment is fused at the C-terminus of its heavy chain to the N-terminus of the heavy chain of the second Fab fragment, which is in turn fused at the C-terminus of its heavy chain to the N-terminus of the first Fc domain subunit. In a another embodiment, the second Fab fragment is fused at the C-terminus of its heavy chain to the N-terminus of the heavy chain of the first Fab fragment, which is in turn fused at the C-terminus of its heavy chain to the N-terminus of the first Fc domain subunit.

In some embodiments, additionally the Fab light chain of the first Fab fragment and the Fab light chain of the second Fab fragment are fused to each other, optionally via a peptide linker.

In one embodiment, the replacement is made in the first Fab fragment. In some embodiments, the replacement is a replacement of the variable domains VL and VH by each other. In other embodiments the replacement is a replacement of the constant domains CL and CH1 by each other.

In one embodiment the bispecific antigen binding molecule essentially consists of the first Fab fragment, the second Fab fragment, the Fc domain, and optionally one or more peptide linkers.

In particular embodiments, the bispecific antigen binding molecule comprises a third Fab fragment which specifically binds to the first or the second antigen. In one embodiment, the third Fab fragment is fused to the second Fc domain subunit. In a more specific embodiment, the third Fab fragment is fused at its C-terminus to the N-terminus of the second Fc domain subunit. In en even more specific embodiment, the third Fab fragment is fused at the C-terminus of its heavy chain to the N-terminus of the second Fc domain subunit. In one embodiment, the third Fab fragment specifically binds to the second antigen. In some embodiments the second Fab fragment, the third Fab fragment and the Fc domain are part of an immunoglobulin molecule. In a specific such embodiment, the immunoglobulin molecule is an IgG class immunoglobulin molecule, more specifically an IgG1 or IgG4 subclass immunoglobulin molecule. In one embodiment, the immunoglobulin molecule is a human immunoglobulin molecule. In one embodiment, the bispecific antigen binding molecule essentially consists of a first Fab fragment which specifically binds to the first antigen, an immunoglobulin molecule which specifically binds to the second antigen, and optionally one or more peptide linkers.

In one embodiment, the same replacement is made in Fab fragments that specifically bind to the same antigen. In a further embodiment, a replacement is made only in the first Fab fragment. In one embodiment, the bispecific antigen binding molecule provides monovalent binding to the first antigen.

In certain embodiments, the Fc domain comprises a modification promoting the association of the first and second Fc domain subunit. In a specific such embodiment, an amino acid residue in the CH3 domain of the first subunit of the Fc domain is replaced with an amino acid residue having a larger side chain volume, thereby generating a protuberance within the CH3 domain of the first subunit which is positionable in a cavity within the CH3 domain of the second subunit, and an amino acid residue in the CH3 domain of the second subunit of the Fc domain is replaced with an amino acid residue having a smaller side chain volume, thereby generating a cavity within the CH3 domain of the second subunit within which the protuberance within the CH3 domain of the first subunit is positionable. In one embodiment, the Fc domain is an IgG Fc domain, specifically an IgG1 or IgG4 Fc domain. In one embodiment the Fc domain is human. In certain embodiments, the Fc domain is engineered to to comprise an increased proportion of non-fucosylated oligosaccharides, as compared to a non-engineered Fc domain. In other embodiments, the Fc domain comprises one or more amino acid substitution that reduces the binding affinity of the Fc domain to an Fc receptor and/or effector function, wherein said amino acid substitution is at a position selected from the group of E233, L234, L235, N297, P331 and P329 (EU numbering).

According to another aspect of the invention there is provided an isolated polynucleotide encoding a bispecific antigen binding molecule of the invention.

The invention further provides a pharmaceutical composition comprising the bispecific antigen binding molecule of the invention and a pharmaceutically acceptable carrier.

In one aspect the invention provides a bispecific antigen binding molecule or a pharmaceutical composition of the invention for use as a medicament. In one aspect is provided a bispecific antigen binding molecule or a pharmaceutical composition according to the invention for use in the treatment of a disease in an individual in need thereof. In a specific embodiment the disease is cancer.

### Brief Description of the Drawings

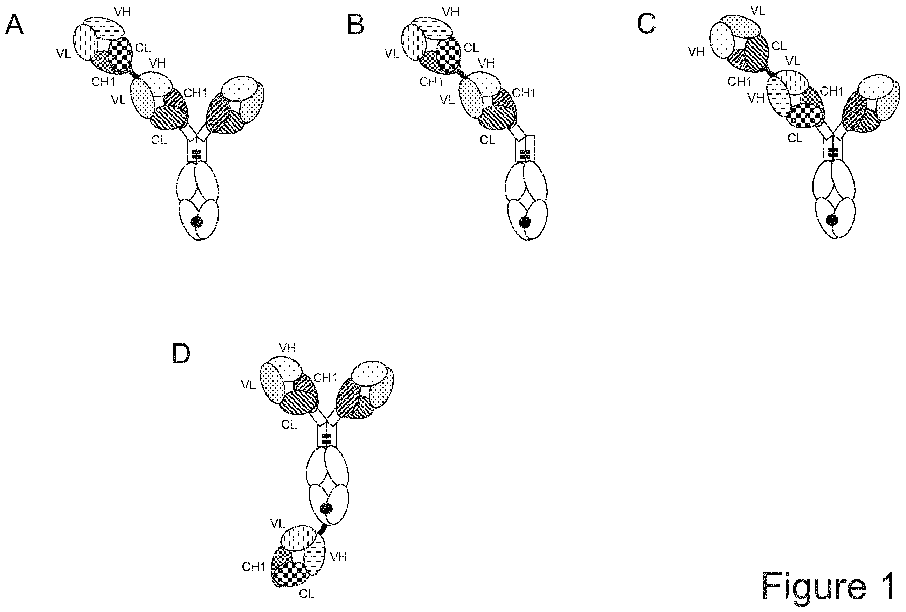

FIGURE 1. Illustration of exemplary formats of the bispecific antigen binding molecules disclosed herein. (A) "2+1" format, with Crossfab fragment of different specificity fused to N-terminus of a Fab fragment comprised in an antibody ("2+1 IgG Crossfab (N-terminal)"). (B) "1+1" format, with Crossfab fragment of different specificity fused to N-terminus of a Fab fragment comprised in an antibody lacking the second Fab fragment ("1+1 IgG Crossfab (N-terminal)") . (C) "2+1" format as in (A), wherein the order of the Crossfab fragment, and the Fab fragment to which the Crossfab fragment is fused, is inverted ("2+1" IgG Crossfab (N-terminal), inverted"). (D) "2+1" format, with Crossfab fragment of different specificity fused to C-terminus of an Fc domain subunit comprised in an antibody ("2+1 IgG Crossfab (C-terminal)"). Black dot: optional modification in the Fc domain promoting heterodimerization.

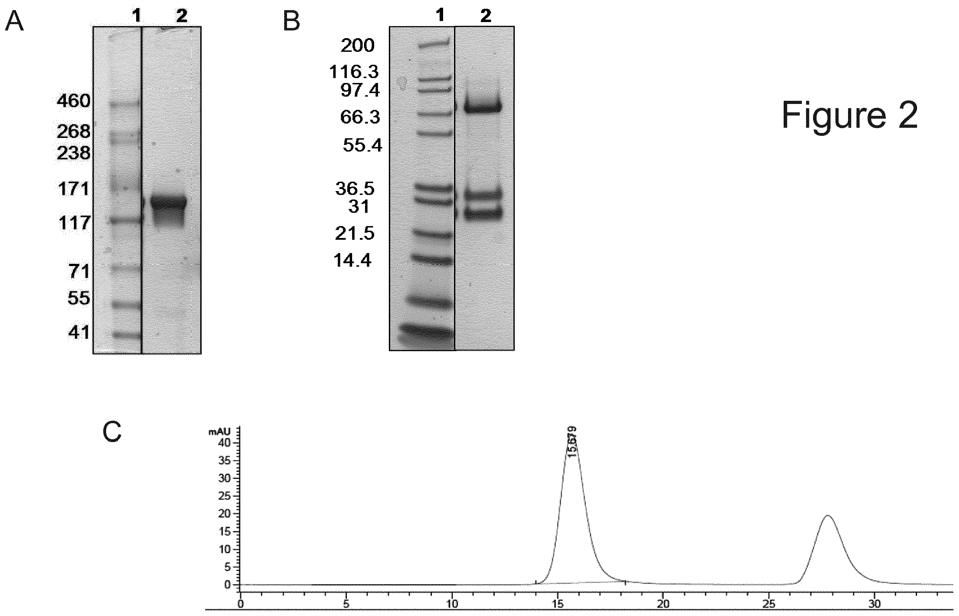

FIGURE 2. (A, B) SDS PAGE (4-12% Tris-Acetate (A) or 4-12% Bis/Tris (B), NuPage Invitrogen, Coomassie-stained) of "1+1 IgG Crossfab (N-terminal), Fc(hole) P329G LALA / Fc(knob) wt" (anti-MCSP/anti-huCD3) (see SEQ ID NOs 1, 2, 3 and 4), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG Crossfab (N-terminal), Fc(hole) P329G LALA / Fc(knob) wt" (anti-MCSP/anti-huCD3).

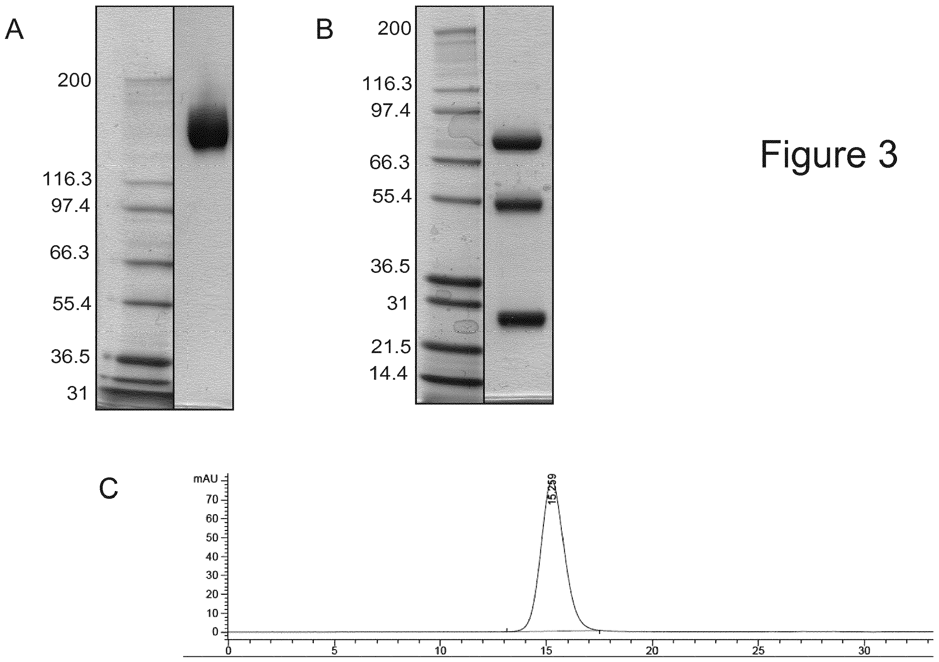

FIGURE 3. (A, B) SDS PAGE (4-12% Bis/Tris, NuPage Invitrogen, Coomassie-stained) of "2+1 IgG Crossfab (N-terminal)" (anti-MCSP/anti-huCD3) (see SEQ ID NOs 1, 3, 4 and 5), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG Crossfab (N-terminal)" (anti-MCSP/anti-huCD3).

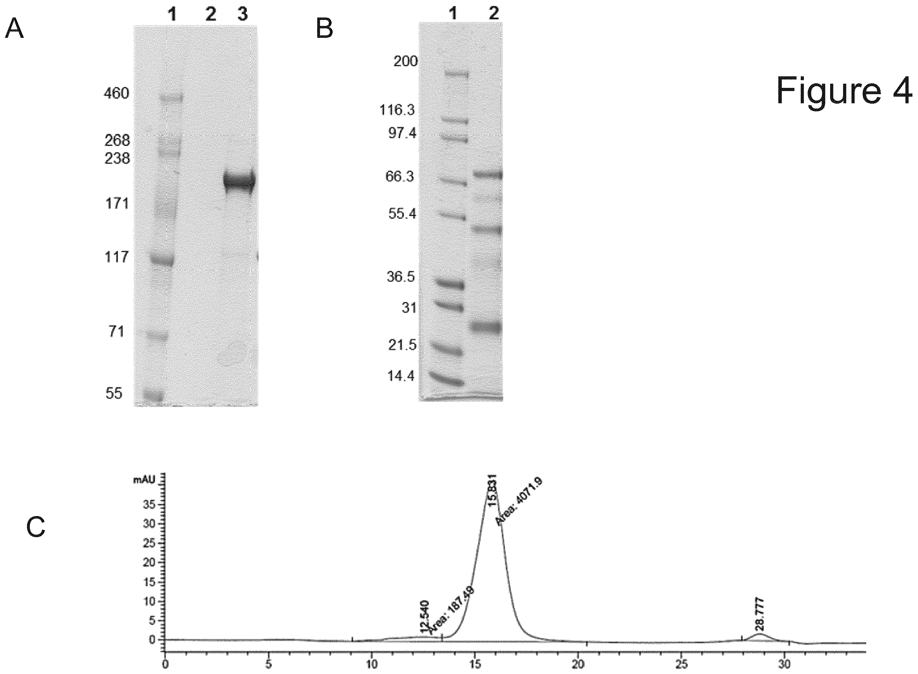

FIGURE 4. (A, B) SDS PAGE (4-12% Bis/Tris, NuPage Invitrogen, Coomassie-stained) of "2+1 IgG Crossfab (N-terminal), inverted" (anti-CEA/anti-huCD3) (see SEQ ID NOs 3, 8, 9 and 10), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG Crossfab (N-terminal), inverted" (anti-CEA/anti-huCD3).

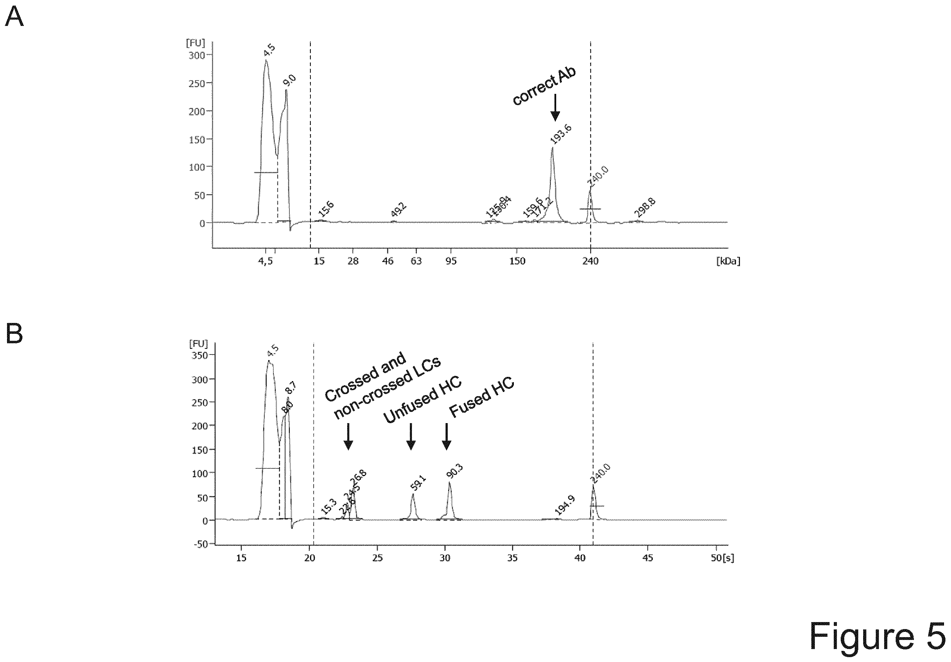

FIGURE 5. (A, B) Capillary electrophoresis (CE)-SDS gel analysis of "2+1 IgG Crossfab (C-terminal)" (anti-c-Met/anti-Her3) (see SEQ ID NOs 11, 12, 13, 14), non reduced (A) and reduced (B).

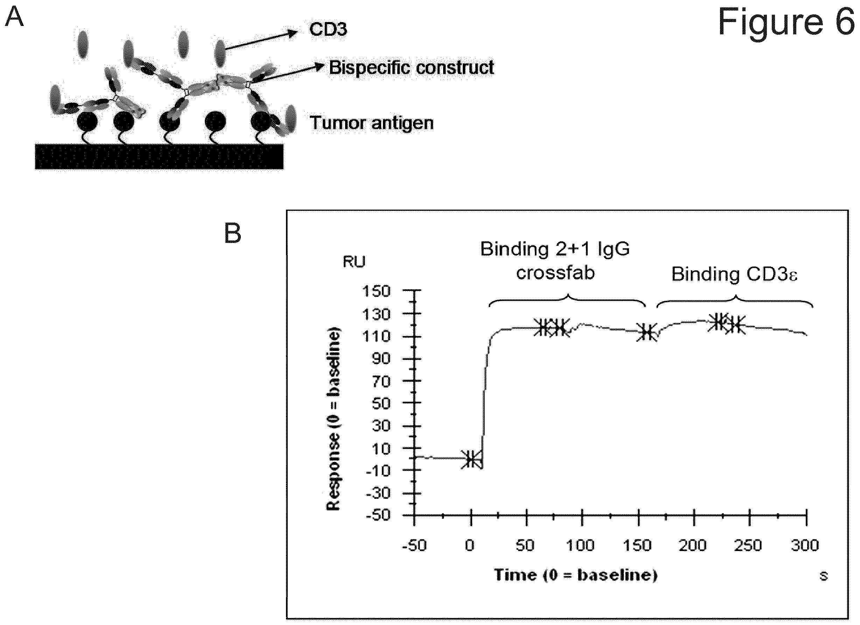

FIGURE 6. Simultaneous binding of bispecific constructs to the D3 domain of human MCSP and human CD3γ(G₄S)₅CD3ε-AcTev-Fc(knob)-Avi/Fc(hole). (A) Biacore assay setup; (B) measurement of "2+1 IgG Crossfab (N-terminal)".

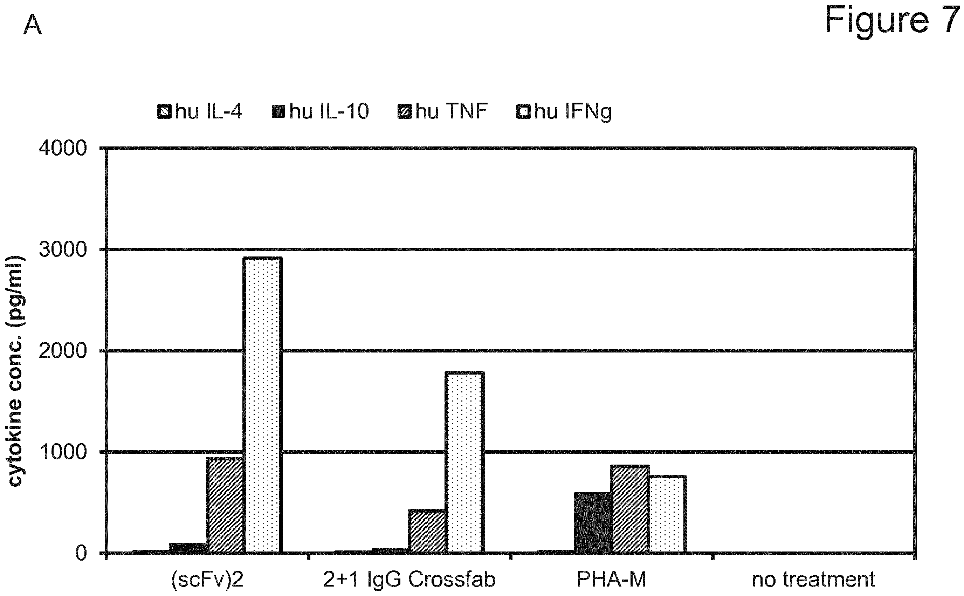

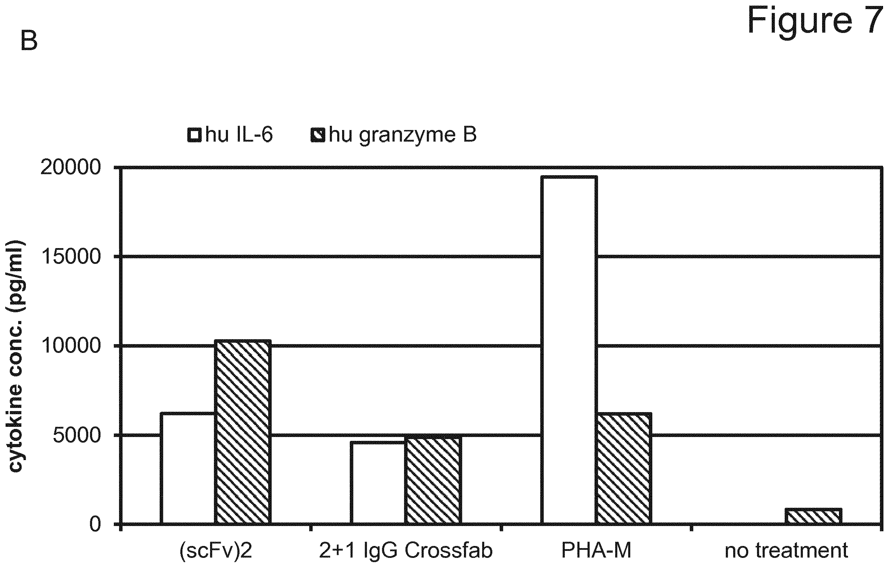

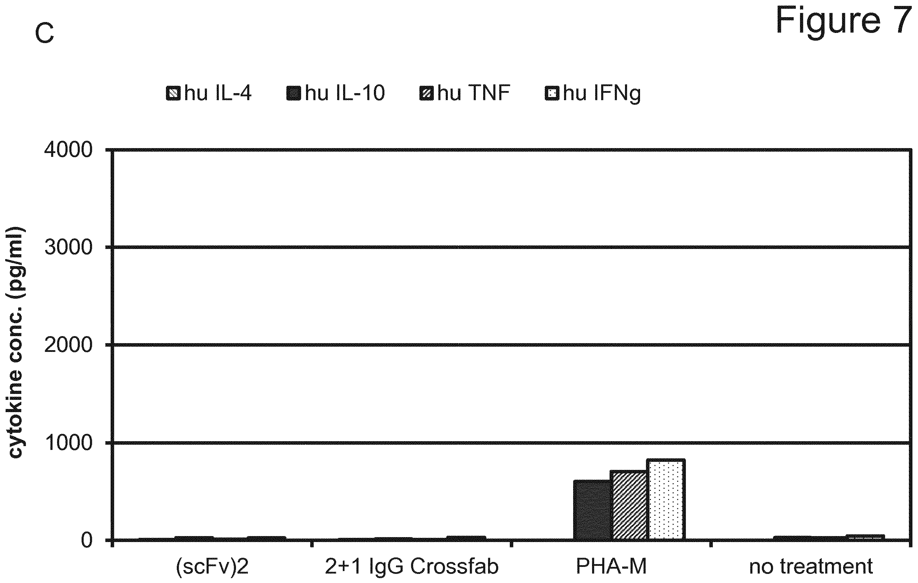

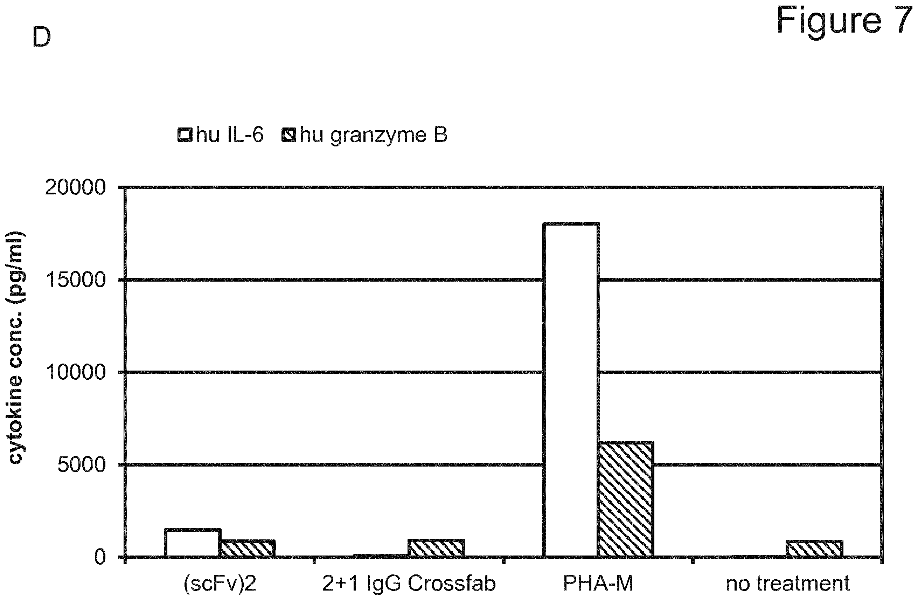

FIGURE 7. Levels of different cytokines measured in the supernatant of whole blood after treatment with 1 nM of different CD3-MCSP bispecific constructs ("2+1 IgG Crossfab (N-terminal)", "(scFv)₂") or corresponding control IgGs in the presence (A, B) or absence (C, D) of Colo-38 tumor cells for 24 hours.

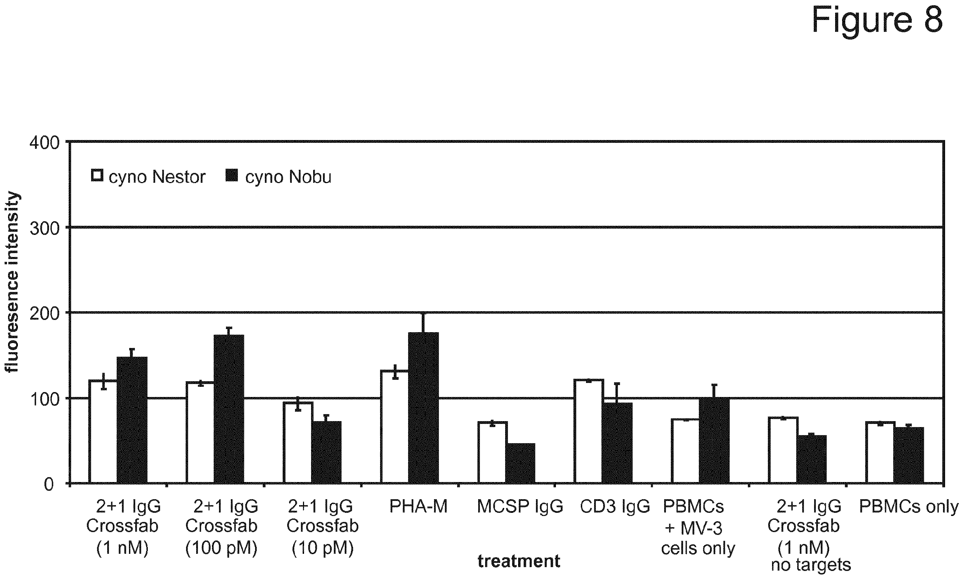

FIGURE 8. Surface expression level of the late activation marker CD25 on cynomolgus CD8⁺ T cells from two different animals (cyno Nestor, cyno Nobu) after 43 hours incubation with the indicated concentrations of the "2+1 IgG Crossfab (N-terminal)" bispecific construct (targeting cynomolgus CD3 and human MCSP), in the presence or absence of human MCSP-expressing MV-3 tumor target cells (E:T ratio = 3:1). As controls, the reference IgGs (anti-cynomolgus CD3 IgG, anti-human MCSP IgG) or the unphysiologic stimulus PHA-M were used.

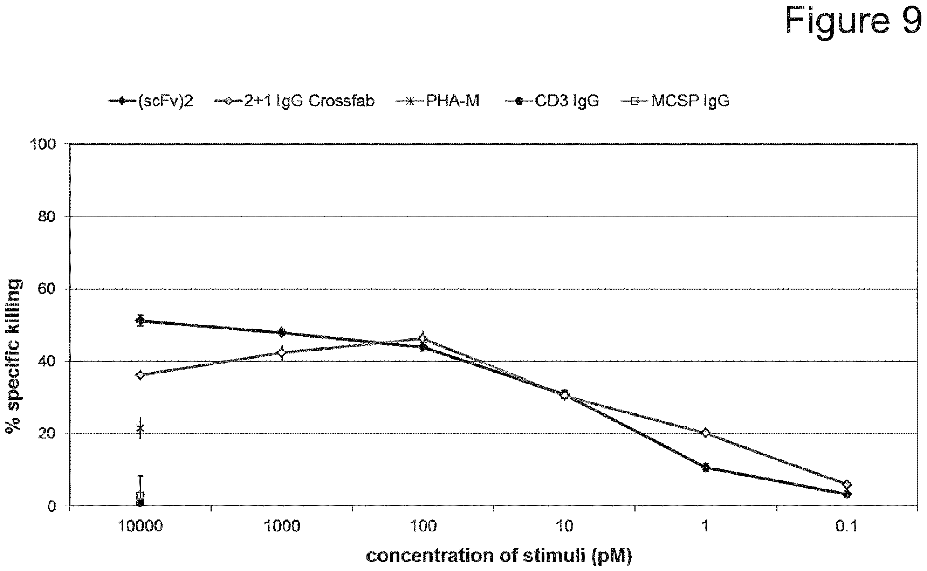

FIGURE 9. Killing (as measured by LDH release) of MDA-MB-435 tumor cells upon co-culture with human pan T cells (E:T ratio = 5:1) and activation for 20 hours by different concentrations of the "2+1 IgG Crossfab (N-terminal)" and "(scFv)₂" bispecific molecules and corresponding IgGs.

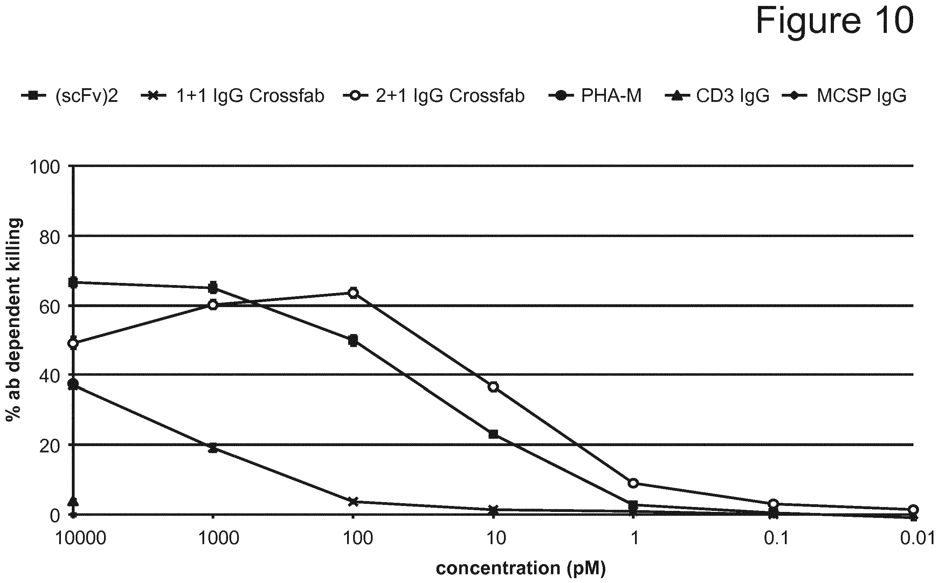

FIGURE 10. Killing (as measured by LDH release) of MDA-MB-435 tumor cells upon co-culture with human pan T cells (E:T ratio = 5:1), and activation for 21 hours by different concentrations of the bispecific constructs and corresponding IgGs. The CD3-MCSP bispecific "2+1 IgG Crossfab (N-terminal)" and "1+1 IgG Crossfab (N-terminal)" constructs, the "(scFv)₂" molecule and corresponding IgGs were compared.

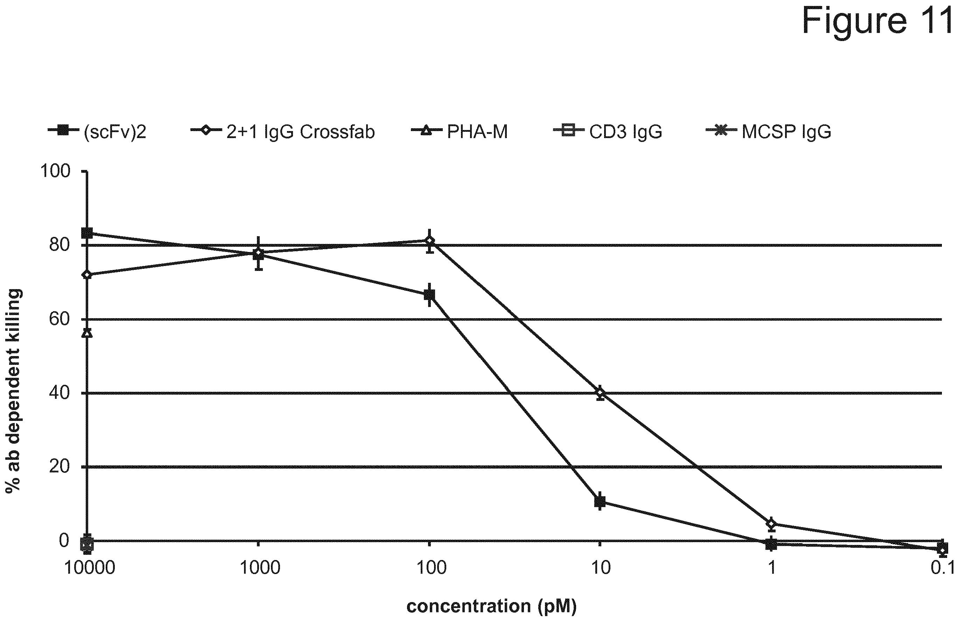

FIGURE 11. Killing (as measured by LDH release) of huMCSP-positive MV-3 melanoma cells upon co-culture with human PBMCs (E:T ratio = 10:1), treated with different CD3-MCSP bispecific constructs ("2+1 IgG Crossfab (N-terminal)" and "(scFv)₂") for -26 hours.

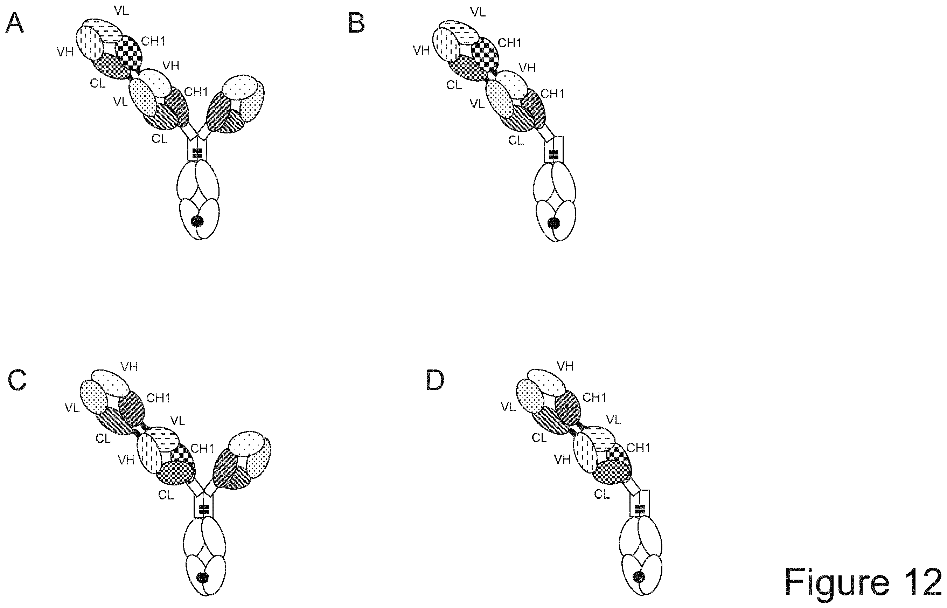

FIGURE 12. Examplary configurations of bispecific antigen binding molecules disclosed herein having a linked light chain. (A) Illustration of the "2+1 IgG Crossfab (N terminal), linked light chain" molecule. (B) Illustration of the "1+1 IgG Crossfab (N-terminal), linked light chain" molecule. (C) Illustration of the "2+1 IgG Crossfab (N-terminal), inverted, linked light chain" molecule. (D) Illustration of the "1+1 IgG Crossfab (N-terminal), inverted, linked light chain" molecule.

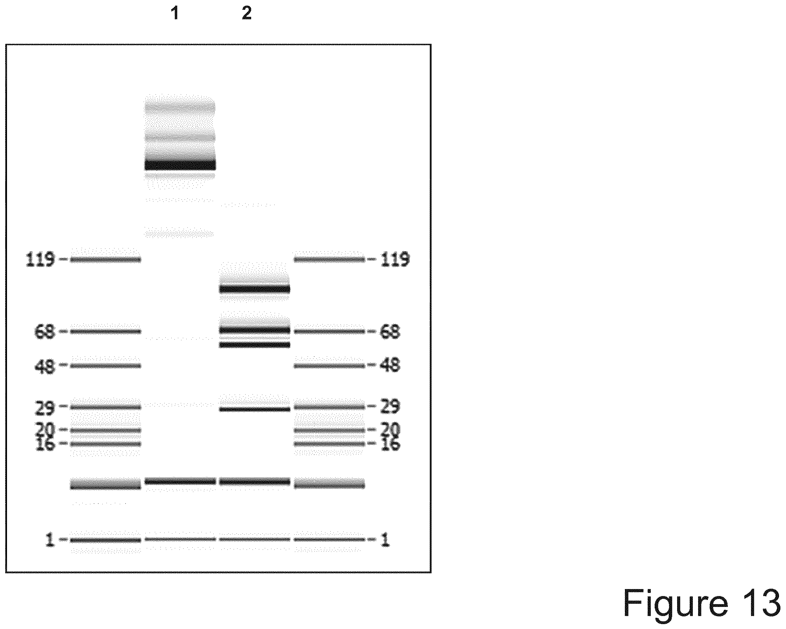

FIGURE 13. CE-SDS analyses. Electropherogram shown as SDS PAGE of "2+1 IgG Crossfab (N-terminal), linked light chain" (lane 1: reduced, lane 2: non-reduced).

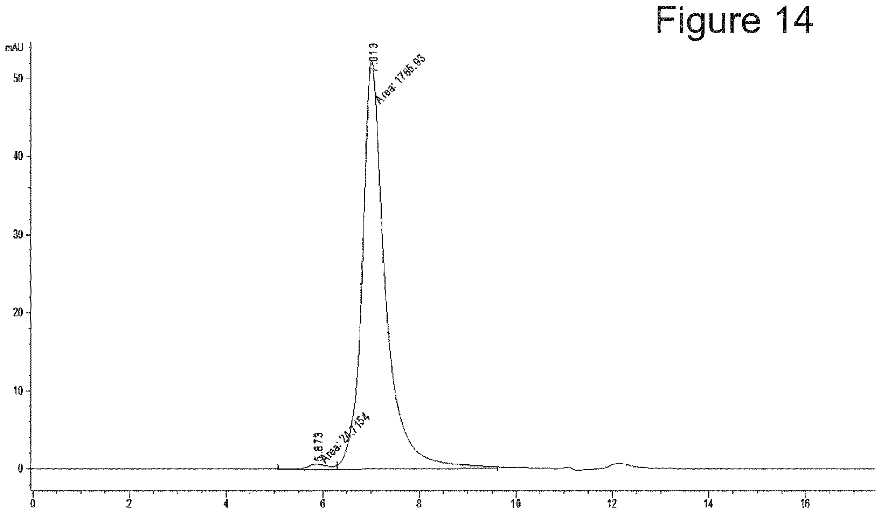

FIGURE 14. Analytical size exclusion chromatography of "2+1 IgG Crossfab (N-terminal), linked light chain" (final product). 20 µg sample "2+1 IgG Crossfab (N-terminal), linked light chain" were injected.

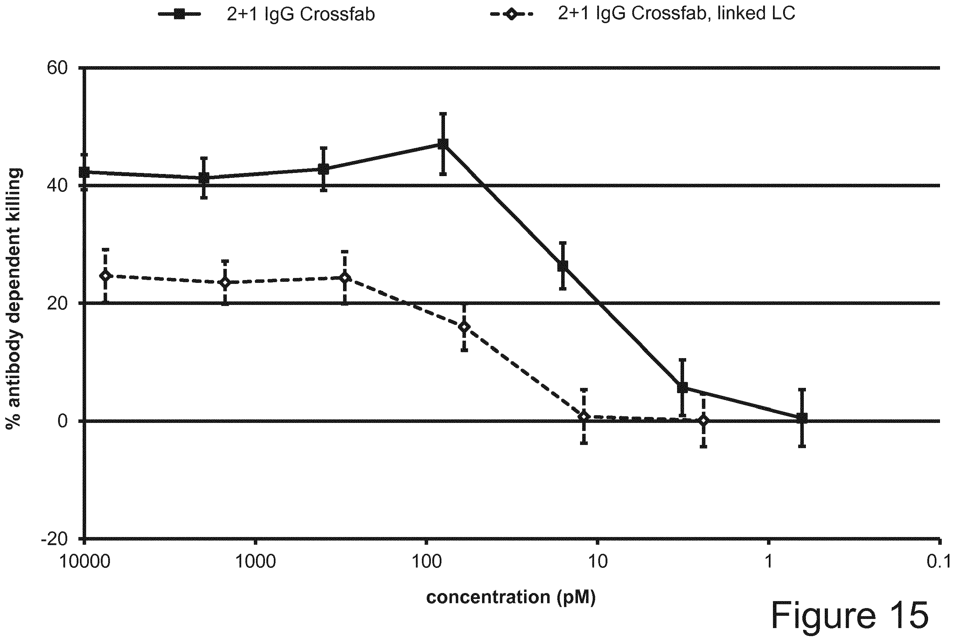

FIGURE 15. Killing (as measured by LDH release) of MCSP-positive MV-3 tumor cells upon co-culture by human PBMCs (E:T ratio = 10:1), treated with different CD3-MCSP bispecific constructs for ∼ 44 hours. Human PBMCs were isolated from fresh blood of healthy volunteers.

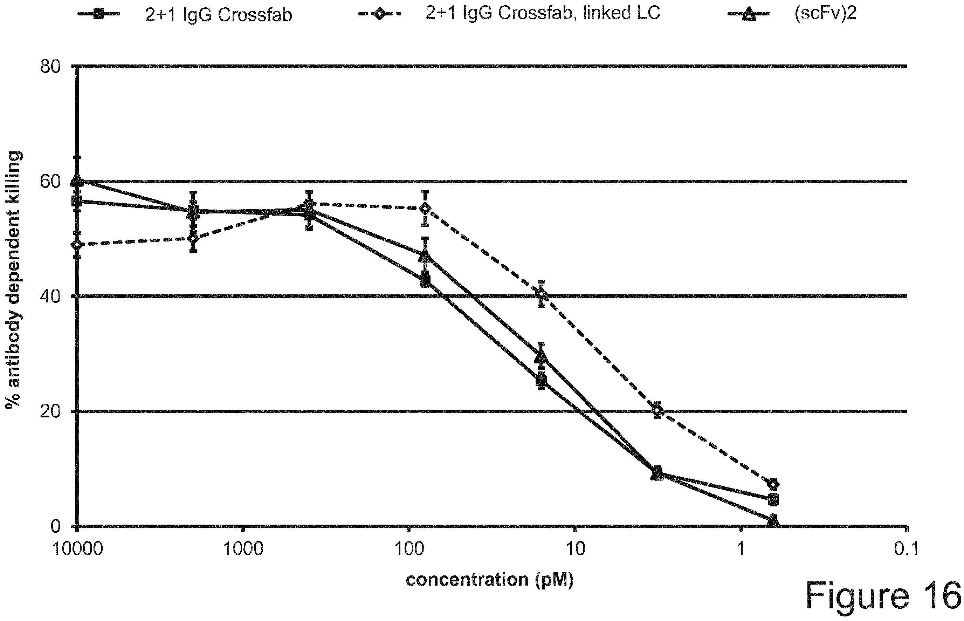

FIGURE 16. Killing (as measured by LDH release) of MCSP-positive Colo-38 tumor cells upon co-culture by human PBMCs (E:T ratio = 10:1), treated with different CD3-MCSP bispecific constructs for ∼22 hours. Human PBMCs were isolated from fresh blood of healthy volunteers.

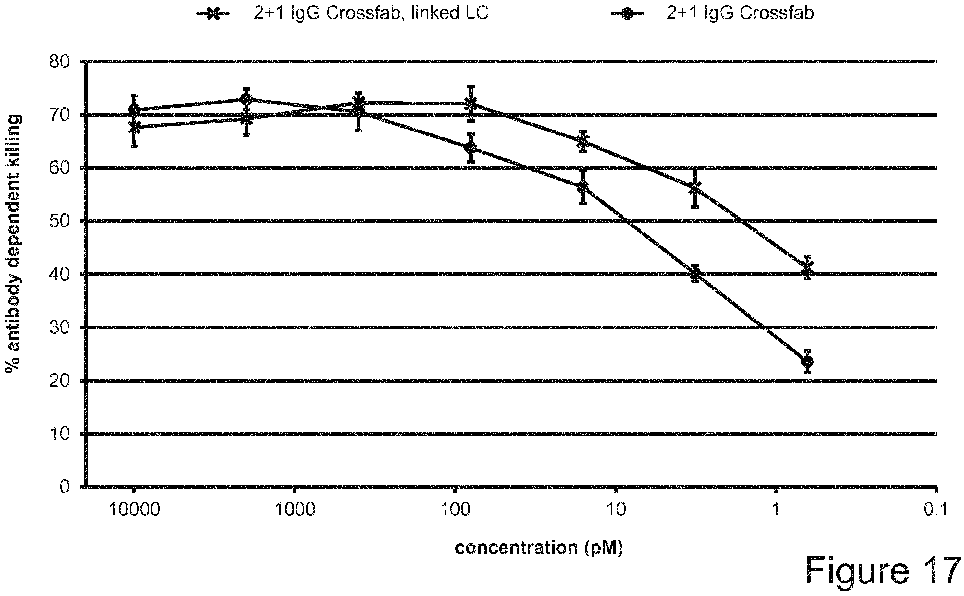

FIGURE 17. Killing (as measured by LDH release) of MCSP-positive Colo-38 tumor cells upon co-culture by human PBMCs (E:T ratio = 10:1), treated with different CD3-MCSP bispecific constructs for ∼22 hours. Human PBMCs were isolated from fresh blood of healthy volunteers.

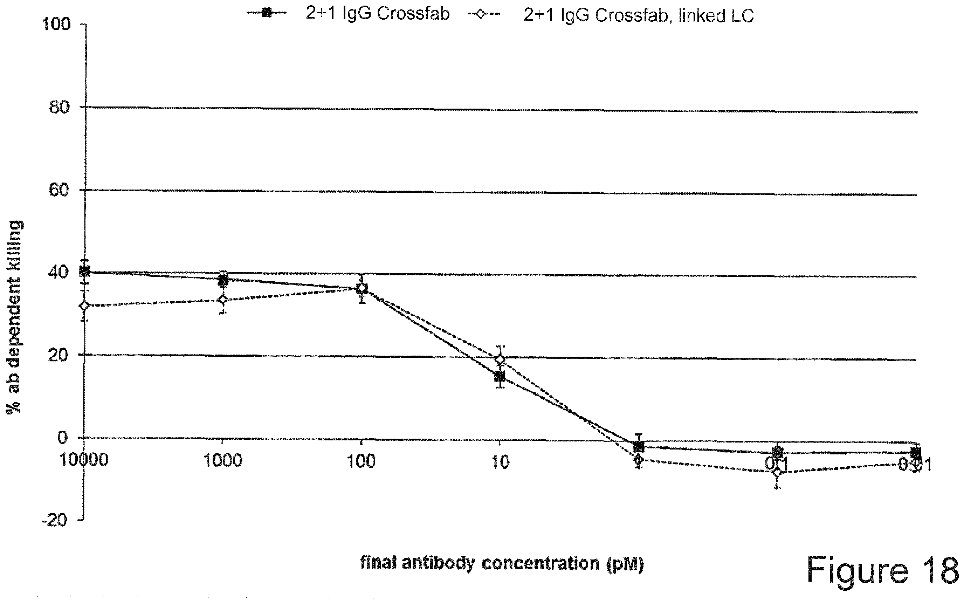

FIGURE 18. Killing (as measured by LDH release) of MCSP-positive WM266-4 cells upon co-culture by human PBMCs (E:T ratio = 10:1), treated with different CD3-MCSP bispecific constructs for ∼22 hours. Human PBMCs were isolated from fresh blood of healthy volunteers.

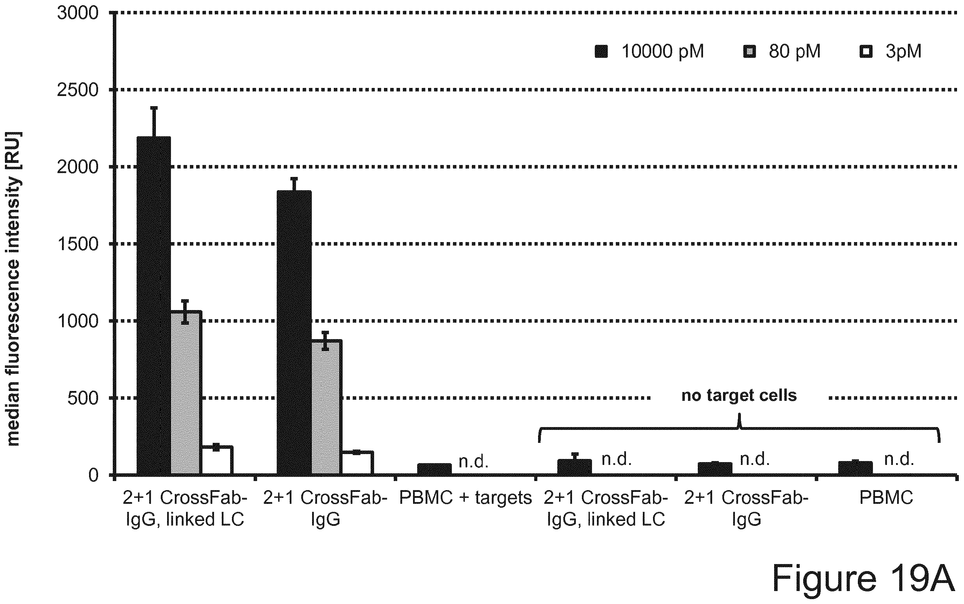

FIGURE 19. Surface expression level of the early activation marker CD69 (A) and the late activation marker CD25 (B) on human CD8⁺ T cells after 22 hours incubation with 10 nM, 80 pM or 3 pM of different CD3-MCSP bispecific constructs in the presence or absence of human MCSP-expressing Colo-38 tumor target cells (E:T ratio = 10:1).

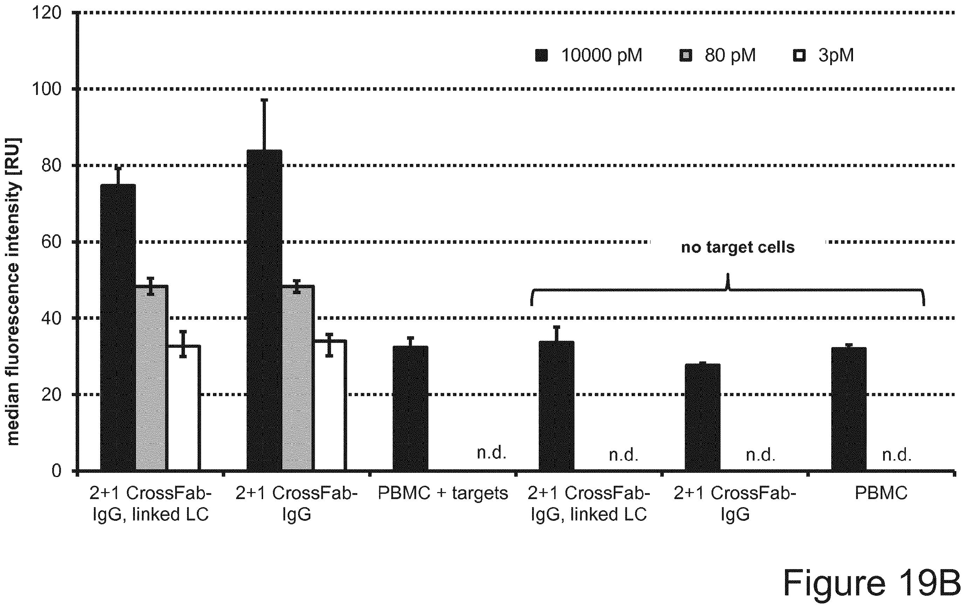

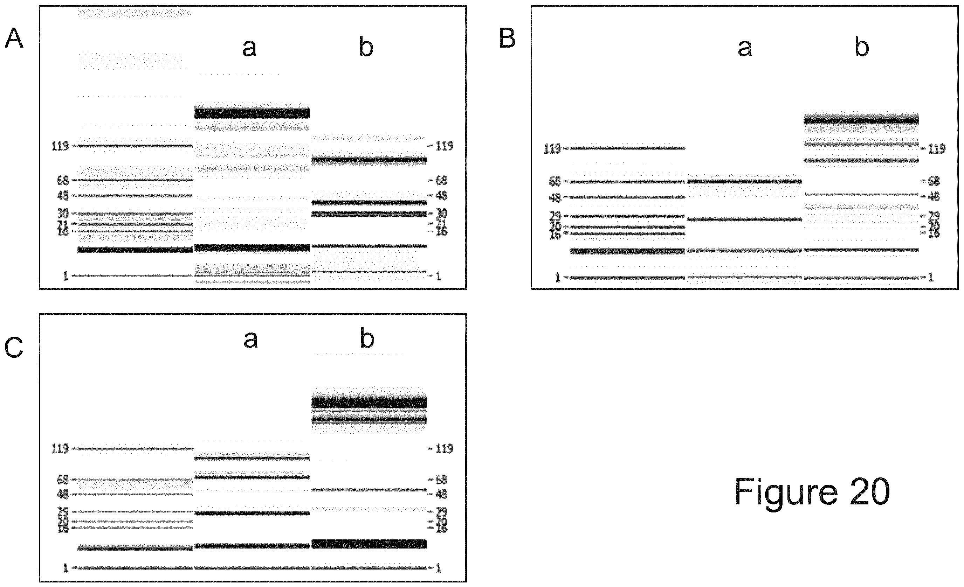

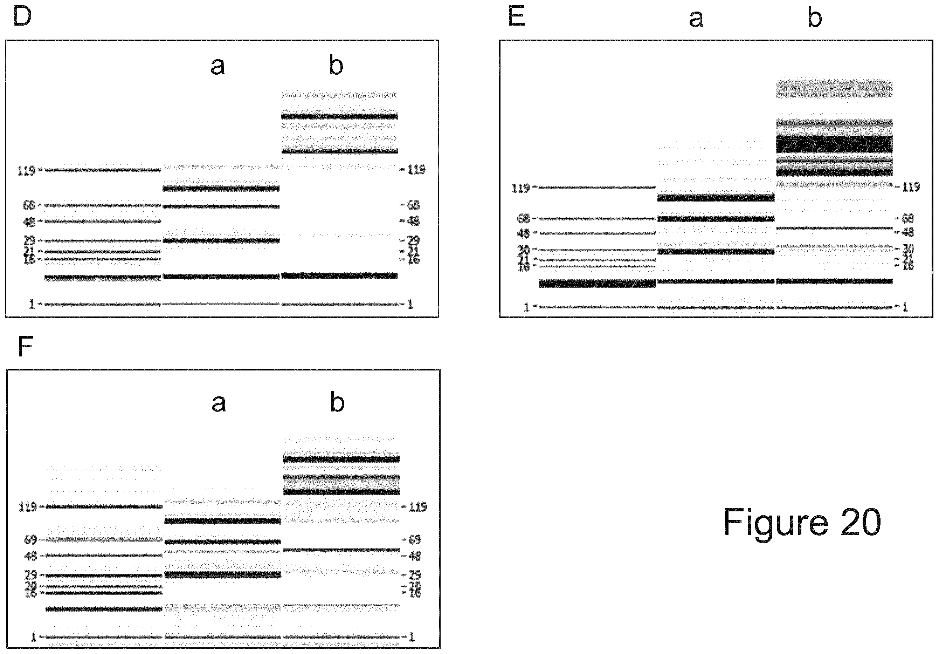

FIGURE 20. CE-SDS analyses. (A) Electropherogram shown as SDS-PAGE of 1+1 IgG Crossfab (N-terminal); VL/VH exchange (LC007/V9): a) non-reduced, b) reduced. (B) Electropherogram shown as SDS-PAGE of 1+1 CrossMab; CL/CH1 exchange (LC007/V9): a) reduced, b) non-reduced. (C) Electropherogram shown as SDS-PAGE of 2+1 IgG Crossfab (N-terminal), inverted; CL/CH1 exchange (LC007/V9): a) reduced, b) non-reduced. (D) Electropherogram shown as SDS-PAGE of 2+1 IgG Crossfab (N-terminal); VL/VH exchange (M4-3 ML2/V9): a) reduced, b) non-reduced. (E) Electropherogram shown as SDS-PAGE of 2+1 IgG Crossfab (N-terminal); CL/CH1 exchange (M4-3 ML2/V9): a) reduced, b) non-reduced. (F) Electropherogram shown as SDS-PAGE of 2+1 IgG Crossfab (N-terminal), inverted; CL/CH1 exchange (CH1A1A/V9): a) reduced, b) non-reduced.

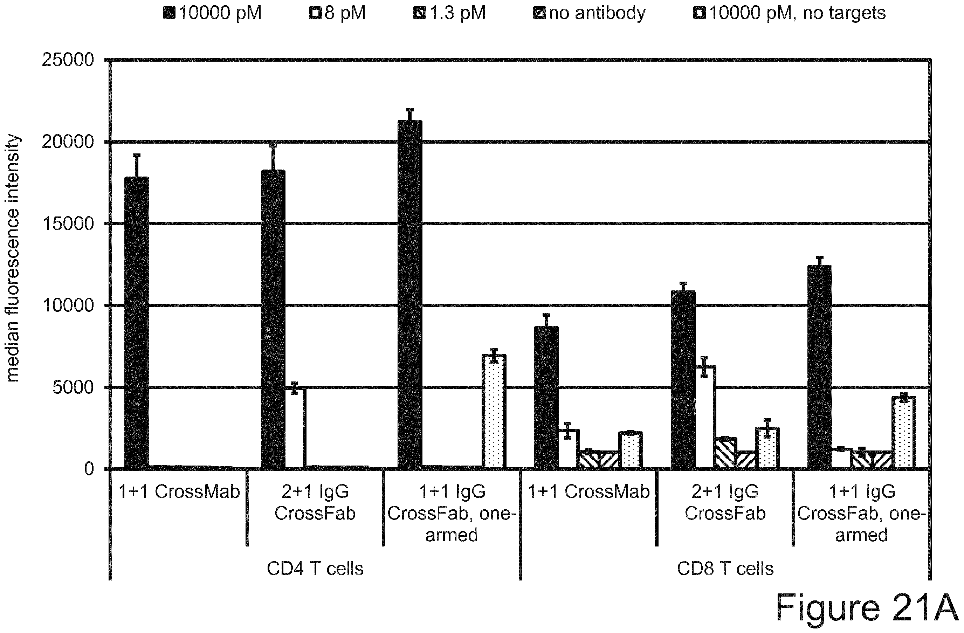

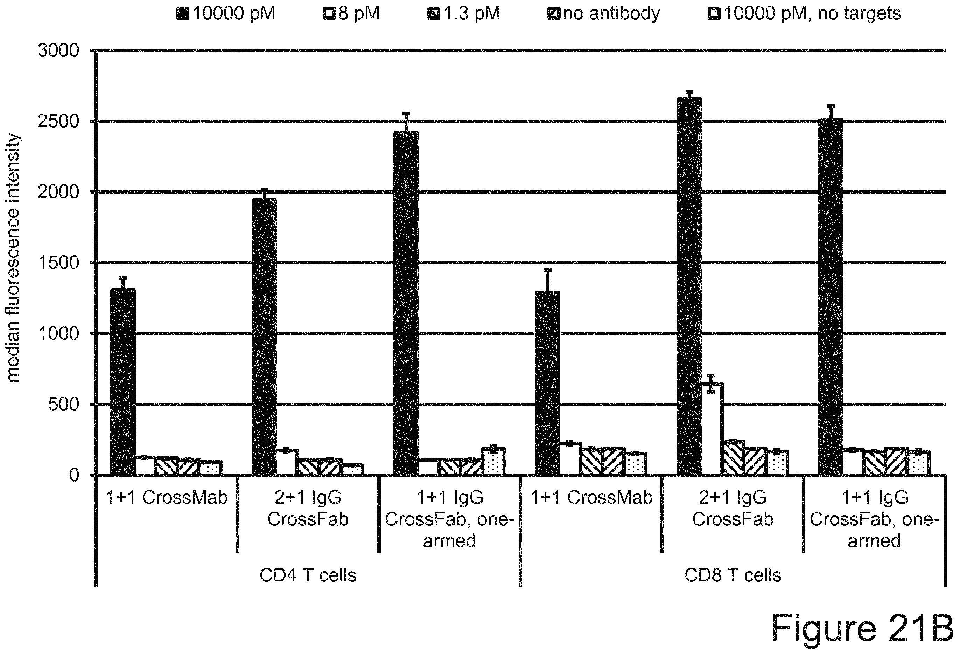

FIGURE 21. Surface expression level of the early activation marker CD69 (A) or the late activation marker CD25 (B) on human CD4⁺ or CD8⁺ T cells after 24 hours incubation with the indicated concentrations of the CD3/MCSP "1+1 CrossMab", "1+1 IgG Crossfab (N-terminal)" and "2+1 IgG Crossfab (N-terminal)" constructs. The assay was performed in the presence or absence of MV-3 target cells, as indicated.

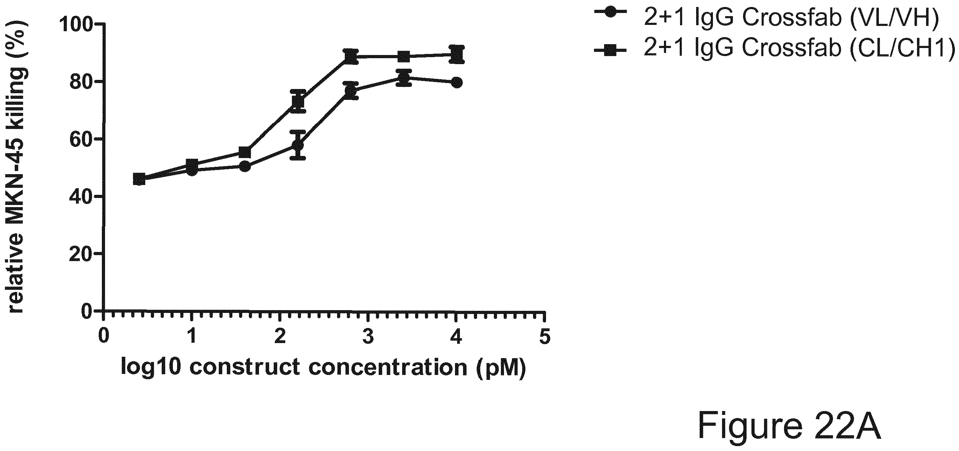

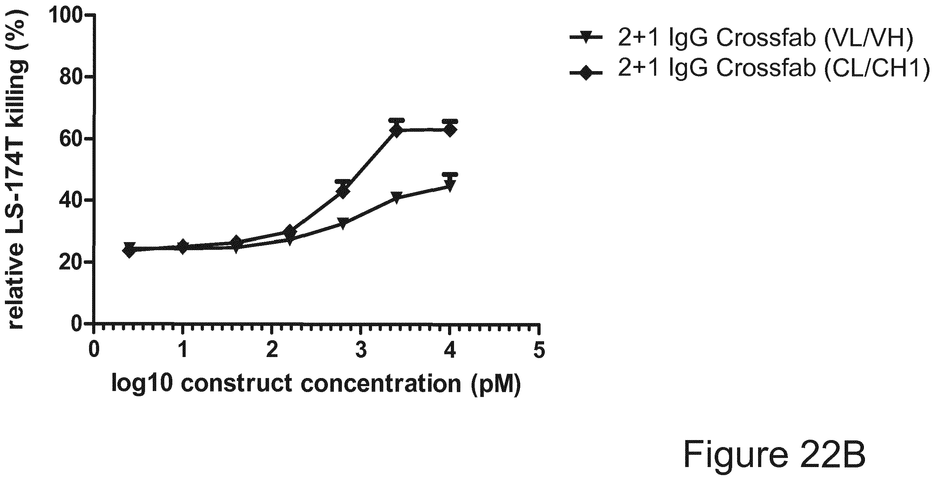

FIGURE 22. Killing (as measured by LDH release) of MKN-45 (A) or LS-174T (B) tumor cells upon co-culture with human PBMCs (E:T ratio = 10:1) and activation for 28 hours by different concentrations of the "2+1 IgG Crossfab (N-terminal), inverted (VL/VH)" versus the "2+1 IgG Crossfab (N-terminal), inverted (CL/CH1)" construct.

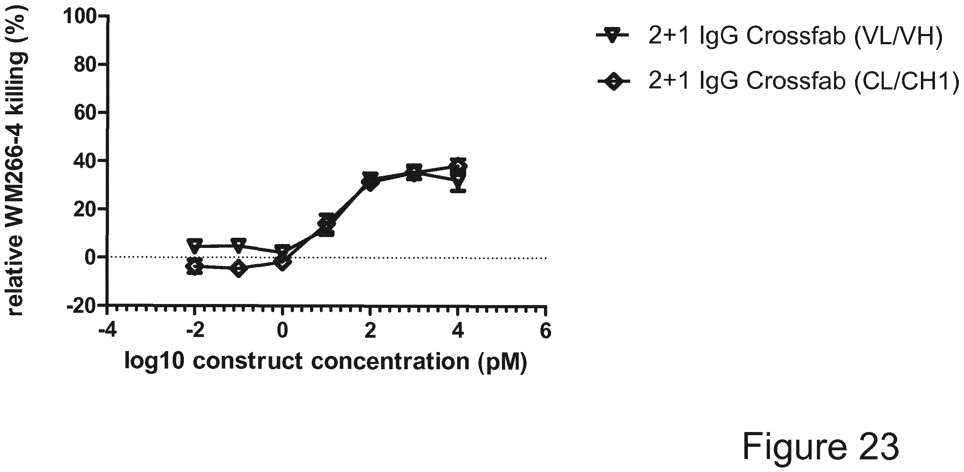

FIGURE 23. Killing (as measured by LDH release) of WM266-4 tumor cells upon co-culture with human PBMCs (E:T ratio = 10:1) and activation for 26 hours by different concentrations of the "2+1 IgG Crossfab (N-terminal) (VL/VH)" versus the "2+1 IgG Crossfab (N-terminal) (CL/CH1)" construct.

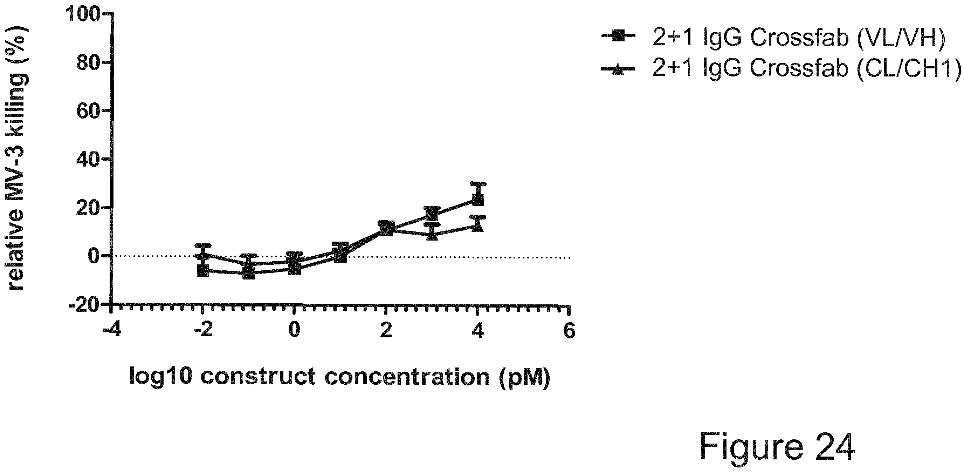

FIGURE 24. Killing (as measured by LDH release) of MV-3 tumor cells upon co-culture with human PBMCs (E:T ratio = 10:1) and activation for 27 hours by different concentrations of the "2+1 IgG Crossfab (N-terminal) (VH/VL)" versus the "2+1 IgG Crossfab (N-terminal) (CL/CH1)" constructs.

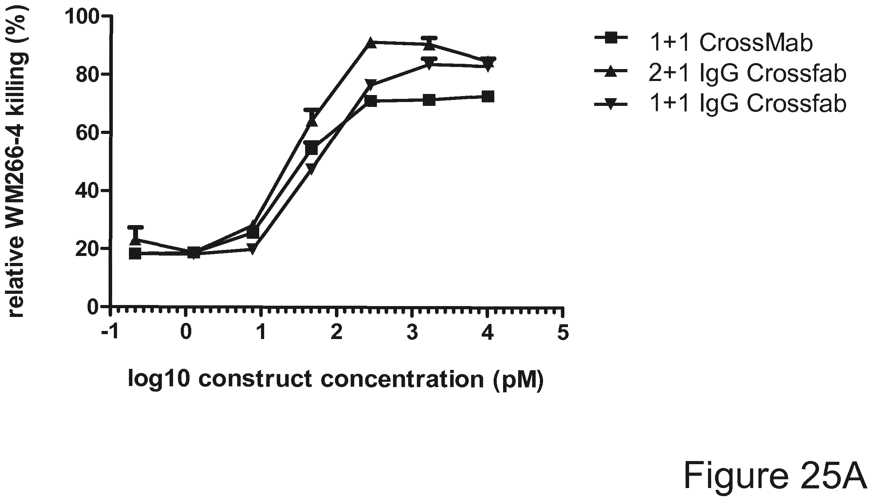

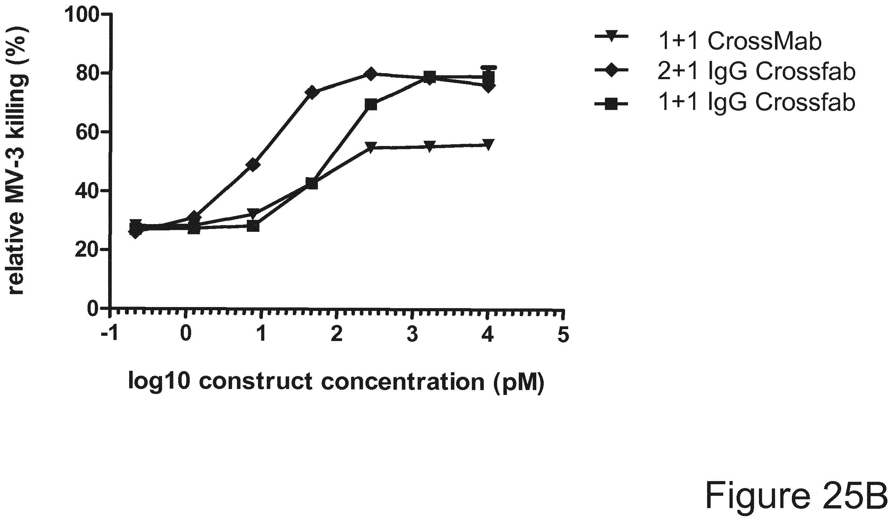

FIGURE 25. Killing (as measured by LDH release) of human MCSP-positive WM266-4 (A) or MV-3 (B) tumor cells upon co-culture with human PBMCs (E:T ratio = 10:1) and activation for 21 hours by different concentrations of the "2+1 IgG Crossfab (N-terminal)", the "1+1 CrossMab", and the "1+1 IgG Crossfab (N-terminal)", as indicated.

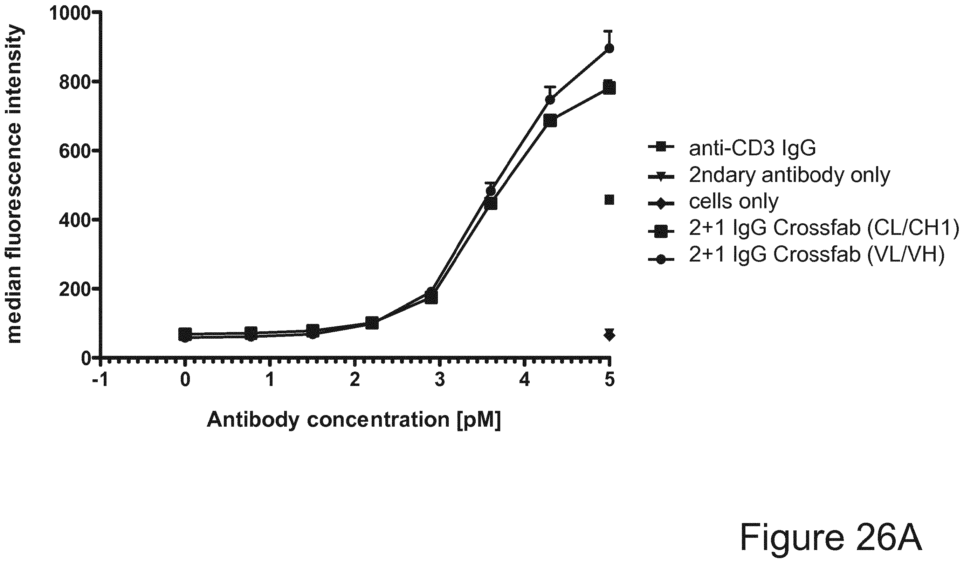

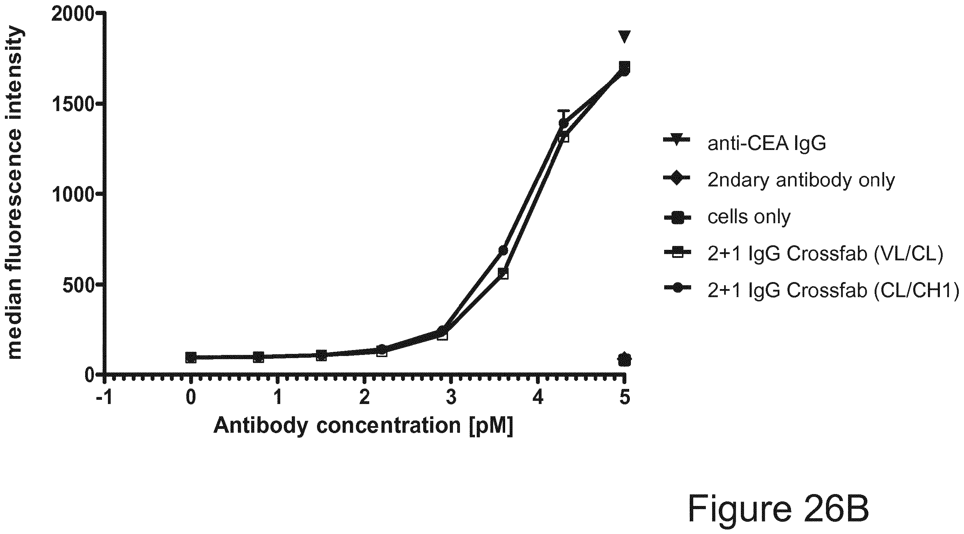

FIGURE 26. Binding of bispecific constructs to human CD3, expressed by Jurkat cells (A), or to human CEA, expressed by LS-174T cells (B) as determined by FACS. As a control, the equivalent maximum concentration of the reference IgGs and the background staining due to the labeled 2ndary antibody (goat anti-human FITC-conjugated AffiniPure F(ab')2 Fragment, Fcγ Fragment-specific, Jackson Immuno Research Lab # 109-096-098) were assessed as well.

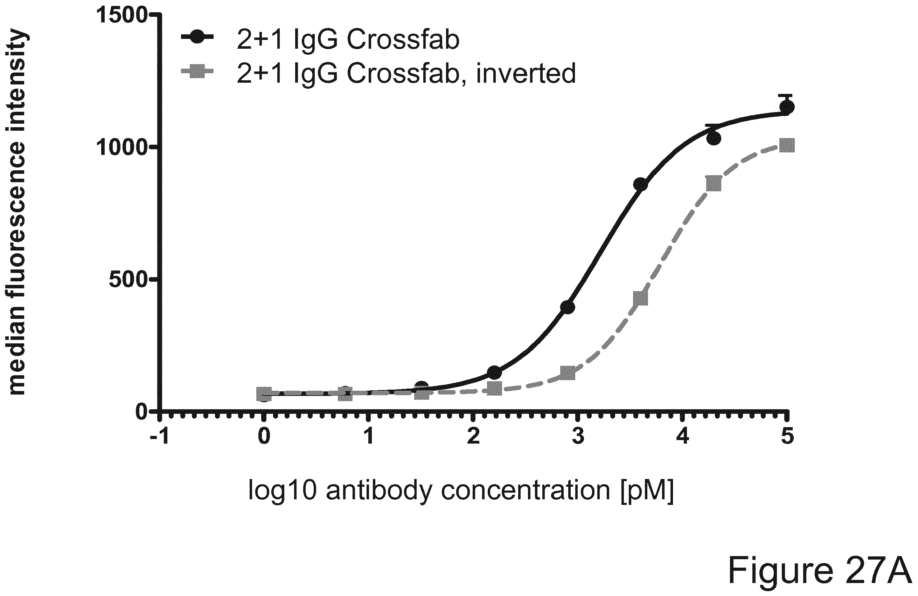

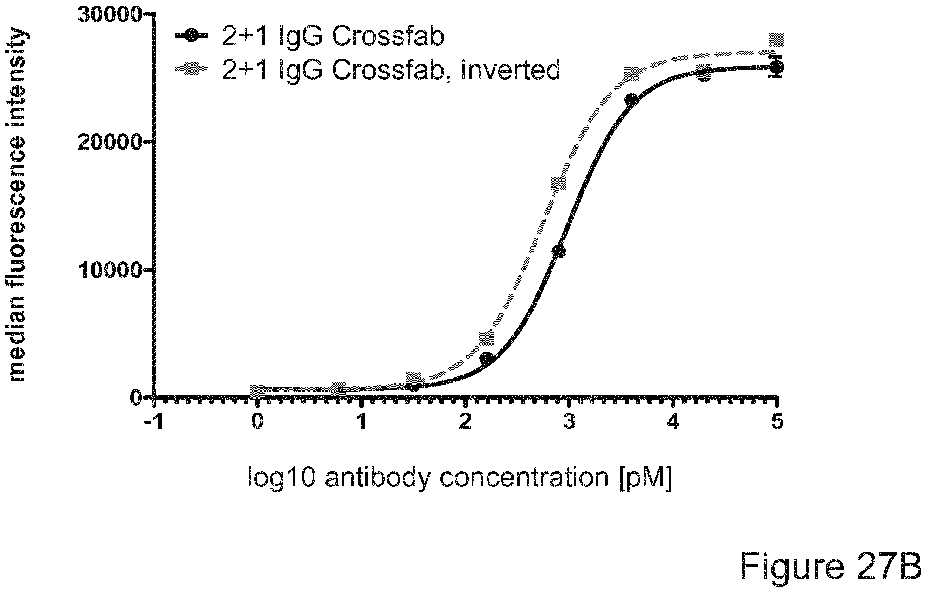

FIGURE 27. Binding of bispecific constructs to human CD3, expressed by Jurkat cells, or to human MCSP, expressed by WM266-4 tumor cells (B) as determined by FACS.

### Detailed Description

### Definitions

Terms are used herein as generally used in the art, unless otherwise defined in the following.

As used herein, the term "antigen binding molecule" refers in its broadest sense to a molecule that specifically binds an antigenic determinant. Examples of antigen binding molecules are immunoglobulins and derivatives, e.g. fragments, thereof.

The term "bispecific" means that the antigen binding molecule is able to specifically bind to two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

As used herein, the term "antigenic determinant" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex.

Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein (e.g. MCSP, FAP, CEA, EGFR, CD33, CD3, c-Met, Her3) can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants. Exemplary human proteins useful as antigens include, but are not limited to: Melanoma-associated Chondroitin Sulfate Proteoglycan (MCSP), also known as Chondroitin Sulfate Proteoglycan 4 (UniProt no. Q6UVK1, NCBI Accession no. NP_001888); Fibroblast Activation Protein (FAP), also known as Seprase (Uni Prot nos. Q12884, Q86Z29, Q99998, NCBI Accession no. NP_004451); Carcinoembroynic antigen (CEA), also known as Carcinoembryonic antigen-related cell adhesion molecule 5 (UniProt no. P06731, NCBI Accession no. NP_004354); CD33, also known as gp67 or Siglec-3 (UniProt no. P20138, NCBI Accession nos. NP_001076087, NP_001171079); Epidermal Growth Factor Receptor (EGFR), also known as ErbB-1 or Her1 (UniProt no. P0053, NCBI Accession nos. NP_958439, NP_958440), CD3, particularly the epsilon subunit of CD3 (UniProt no. P07766, NCBI Accession no. NP_000724); c-Met, also known as Hepatocyte Growth Factor Receptor (UniProt no. P08581, NCBI Accession nos. NP_000236, NP_001120972) and Her3, also known as ErbB-3 (UniProt no. P21860, NCBI Accession nos. NP_001973, NP_001005915). In certain embodiments the bispecific antigen binding molecule discosed herein binds to an epitope of a first antigen or a second antigen that is conserved among the first antigen or second antigen from different species.

By "specific binding" is meant that the binding is selective for the antigen and can be discriminated from unwanted or non-specific interactions. The ability of an antibody to bind to a specific antigenic determinant can be measured either through an enzyme-linked immunosorbent assay (ELISA) or other techniques familiar to one of skill in the art, e.g. surface plasmon resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antibody to an unrelated protein is less than about 10% of the binding of the antibody to the antigen as measured, e.g., by SPR. In certain embodiments, an antibody or a fragement thereof that binds to the antigen has a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., an antigen binding moiety and an antigen, or a receptor and its ligand). The affinity of a molecule X for its partner Y can generally be represented by the dissociation constant (K_{D}), which is the ratio of dissociation and association rate constants (k_{off} and kₒₙ, respectively). Thus, equivalent affinities may comprise different rate constants, as long as the ratio of the rate constants remains the same. Affinity can be measured by well established methods known in the art, including those described herein. A particular method for measuring affinity is Surface Plasmon Resonance (SPR).

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab fragment typically has a single antigen binding site.

As used herein, the term "antigen binding moiety" refers to a polypeptide molecule that specifically binds to an antigenic determinant. Antigen binding moieties include antibodies and fragments thereof as further defined herein. Particular antigen binding moieties include an antigen binding domain of an antibody, comprising an antibody heavy chain variable region and an antibody light chain variable region. In certain embodiments, the antigen binding moieties may comprise antibody constant regions as further defined herein and known in the art. Useful heavy chain constant regions include any of the five isotypes: α, δ, ε, γ, or µ. Useful light chain constant regions include any of the two isotypes: κ and λ.

As used herein, the terms "first" and "second" with respect to Fab fragments etc., are used for convenience of distinguishing when there is more than one of each type of moiety. Use of these terms is not intended to confer a specific order or orientation of the bispecific antigen binding molecule unless explicitly so stated.

As used herein, the term "single-chain" refers to a molecule comprising amino acid monomers linearly linked by peptide bonds. By a single-chain Fab fragment is meant a Fab molecule wherein the Fab light chain and the Fab heavy chain are connected by a peptide linker to form a single peptide chain.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by a hinge region (HR) and three constant domains (CH1, CH2, and CH3), also called a heavy chain constant region. In case of an IgE class immunoglobulin the heavy chain additionally has a CH4 domain.

Hence, an immunoglobulin heavy chain is a polypeptide consisting in N-terminal to C-terminal direction of the following domains: VH-CH1-HR-CH2-CH3-(CH4). Similarly, from N- to C-terminus, each light chain has a variable region (VL), also called a variable light domain or a light chain variable domain, followed by a constant light (CL) domain, also called a light chain constant region. Hence, an immunoglobulin light chain is a polypeptide consisting in N-terminal to C-terminal direction of the following domains: VL-CL. The heavy chain of an immunoglobulin may be assigned to one of five types, called α (IgA), δ (IgD), ε (IgE), γ (IgG), or µ (IgM), some of which may be further divided into subtypes, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ), based on the amino acid sequence of its constant domain. An immunoglobulin essentially consists of two Fab fragments and an Fc domain, linked via the immunoglobulin hinge region.

The term "antibody" herein is used in the broadest sense and encompasses various antibody structures, including but not limited to monoclonal antibodies, polyclonal antibodies, and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046. Diabodies are antibody fragments with two antigen-binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

A "Fab fragment" refers to a protein consisting of the VH and CH1 domain of the heavy chain (the "Fab heavy chain") and the VL and CL domain of the light chain (the "Fab light chain") of an immunoglobulin. A Fab fragment being fused to another protein is, in its unmodified form, fused at its heavy chain C- or N-terminus. Consequently, where the variable domains VH and VL are replaced by each other, the Fab fragment is fused at the C-terminus of the CH1 domain or the N-terminus of the VL domain. Similarly, where the constant domains CH1 and CL are replaced by each other, the Fab fragment is fused at the C-terminus of the CL domain or the N-terminus of the VH domain, and where the complete Fab heavy chain (VH-CH1) and Fab light chain (VL-CL) are replaced by each other, the Fab fragment is fused at its light chain C- or N-terminus.

By "fused" is meant that the components (e.g. a Fab fragment and an Fc domain subunit) are linked by peptide bonds, either directly or via one or more peptide linkers.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Particularly, an antigen binding domain comprises an antibody light chain variable region (VL) and an antibody heavy chain variable region (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term "hypervariable region" or "HVR", as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the complementarity determining regions (CDRs), the latter being of highest sequence variability and/or involved in antigen recognition.

With the exception of CDR1 in VH, CDRs generally comprise the amino acid residues that form the hypervariable loops. Hypervariable regions (HVRs) are also referred to as "complementarity determining regions" (CDRs), and these terms are used herein interchangeably in reference to portions of the variable region that form the antigen binding regions. This particular region has been described by Kabat et al., U.S. Dept. of Health and Human Services, Sequences of Proteins of Immunological Interest (1983) and by Chothia et al., J Mol Biol 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table 1 as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE 1. CDR Definitions¹**

| **CDR** | **Kabat** | **Chothia** | **AbM²** |
|---|---|---|---|
| V_{H} CDR1 | 31-35 | 26-32 | 26-35 |
| V_{H} CDR2 | 50-65 | 52-58 | 50-58 |
| V_{H} CDR3 | 95-102 | 95-102 | 95-102 |
| V_{L} CDR1 | 24-34 | 26-32 | 24-34 |
| V_{L} CDR2 | 50-56 | 50-52 | 50-56 |
| V_{L} CDR3 | 89-97 | 91-96 | 89-97 |

| | | | |
|---|---|---|---|
| ¹ Numbering of all CDR definitions in Table 1 is according to the numbering conventions set forth by Kabat et al. (see below). ² "AbM" with a lowercase "b" as used in Table 1 refers to the CDRs as defined by Oxford Molecular's "AbM" antibody modeling software. | | | |

Kabat et al. also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

The polypeptide sequences of the sequence listing are not numbered according to the Kabat numbering system. However, it is well within the ordinary skill of one in the art to convert the numbering of the sequences of the Sequence Listing to Kabat numbering.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The "class" of an antibody or immunoglobulin refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "Fc domain" or "Fc region" herein is used to define a C-terminal region of an immunoblobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, the C-terminal lysine (Lys447) of the Fc region may or may not be present.

Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991. A "subunit" of an Fc domain as used herein refers to one of the two polypeptides forming the dimeric Fc domain, i.e. a polypeptide comprising C-terminal constant regions of an immunoglobulin heavy chain, capable of stable self-association.

For example, a subunit of an IgG Fc domain comprises an IgG CH2 and an IgG CH3 constant region.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g. Fab fragments) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

The term "effector functions" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

As used herein, the terms "engineer, engineered, engineering", are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor, or increased association with another peptide. Amino acid sequence deletions and insertions include amino- and/or carboxy-terminal deletions and insertions of amino acids. Particular amino acid mutations are amino acid substitutions. For the purpose of altering e.g. the binding characteristics of an Fc region, non-conservative amino acid substitutions, i.e. replacing one amino acid with another amino acid having different structural and/or chemical properties, are particularly preferred.

Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc domain to glycine can be indicated as 329G, G329, G₃₂₉, P329G, or Pro329Gly.

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product.

Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide disclosed herein may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the present disclosure, as are native or recombinant polypeptides which have been separated, fractionated, or partially or substantially purified by any suitable technique.

By "isolated" nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present disclosure. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include *in vivo* or *in vitro* RNA transcripts disclosed herein, as well as positive and negative strand forms, and double-stranded forms. Isolated polynucleotides or nucleic acids disclosed herein further include such molecules produced synthetically. In addition, a polynucleotide or a nucleic acid may be or may include a regulatory element such as a promoter, ribosome binding site, or a transcription terminator.

The term "vector" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector disclosed herein comprises an expression cassette.

Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector disclosed herein comprises an expression cassette that comprises polynucleotide sequences that encode bispecific antigen binding molecules disclosed herein or fragments thereof.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages.

Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules disclosed herein. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An "activating Fc receptor" is an Fc receptor that following engagement by an Fc domain of an antibody elicits signaling events that stimulate the receptor-bearing cell to perform effector functions. Human activating Fc receptors include FcγRIIIa (CD16a), FcγRI (CD64), FcγRIIa (CD32), and FcαRI (CD89).

Antibody-dependent cell-mediated cytotoxicity (ADCC) is an immune mechanism leading to the lysis of antibody-coated target cells by immune effector cells. The target cells are cells to which antibodies or derivatives thereof comprising an Fc region specifically bind, generally via the protein part that is N-terminal to the Fc region. As used herein, the term "reduced (or increased) ADCC" is defined as either a reduction (increase) in the number of target cells that are lysed in a given time, at a given concentration of antibody in the medium surrounding the target cells, by the mechanism of ADCC defined above, and/or an increase (reduction) in the concentration of antibody in the medium surrounding the target cells, required to achieve the lysis of a given number of target cells in a given time, by the mechanism of ADCC. The reduction (increase) in ADCC is relative to the ADCC mediated by the same antibody produced by the same type of host cells, using the same standard production, purification, formulation and storage methods (which are known to those skilled in the art), but that has not been engineered. For example the reduction in ADCC mediated by an antibody comprising in its Fc domain an amino acid substitution that reduces ADCC, is relative to the ADCC mediated by the same antibody without this amino acid substitution in the Fc domain. Suitable assays to measure ADCC are well known in the art (see e.g. PCT publication no. WO 2006/082515 or PCT publication no. WO 2012/130831).

An "effective amount" of an agent refers to the amount that is necessary to result in a physiological change in the cell or tissue to which it is administered.

A "therapeutically effective amount" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "individual" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, bispecific antigen binding molecules disclosed herein are used to delay development of a disease or to slow the progression of a disease.

The term "package insert" is used to refer to instructions customarily included in commercial packages of therapeutic products, that contain information about the indications, usage, dosage, administration, combination therapy, contraindications and/or warnings concerning the use of such therapeutic products.

### Detailed Description of the Embodiments

Provided herein is a bispecific antigen binding molecule, comprising a first Fab fragment which specifically binds to a first antigen, a second Fab fragment which specifically binds to a second antigen, and an Fc domain composed of a first and a second subunit capable of stable association; wherein
a) the bispecific antigen binding molecule provides monovalent binding to the first and/or the second antigen,
b) the first Fab fragment, the second Fab fragment and the first Fc domain subunit are fused to each other, and
c) in the first and/or the second Fab fragment one of the following replacements is made: (i) the variable domains VL and VH are replaced by each other, (ii) the constant domains CL and CH1 are replaced by each other, or (iii) both the variable and constant domains VL-CL and VH-CH1 are replaced by each other,
   provided that not the same replacement is made in the first and the second Fab fragment.

### Bispecific antigen binding molecule formats

The components of the bispecific antigen binding molecule can be fused to each other in a variety of configurations. Exemplary configurations are depicted in Figure 1.

In particular embodiments, the first Fab fragment is fused at its C-terminus to the N-terminus of the second Fab fragment, which is in turn fused at its C-terminus to the N-terminus of the first Fc domain subunit (see examples in Figure 1A and 1B). In one such embodiment, the second Fab fragment is fused to the first Fc domain subunit via an immunoglobulin hinge region. In a further such embodiment, the first Fab fragment is fused to the second Fab fragment via a peptide linker.

In one embodiment, the first Fab fragment is fused at the C-terminus of its heavy chain to the N-terminus of the heavy chain of the second Fab fragment, which is in turn fused at the C-terminus of its heavy chain to the N-terminus of the first Fc domain subunit.

In other embodiments, the second Fab fragment is fused at its C-terminus to the N-terminus of the first Fab fragment, which is in turn fused at its C-terminus to the N-terminus of the first Fc domain subunit (see example in Figure 1C). In one such embodiment, the first Fab fragment is fused to the first Fc domain subunit via an immunoglobulin hinge region. In a further such embodiment, the second Fab fragment is fused to the first Fab fragment via a peptide linker. In one embodiment, the second Fab fragment is fused at the C-terminus of its heavy chain to the N-terminus of the heavy chain of the first Fab fragment, which is in turn fused at the C-terminus of its heavy chain to the N-terminus of the first Fc domain subunit.

In some embodiments wherein either the first Fab fragment is fused at the C-terminus of its heavy chain to the N-terminus of the heavy chain of the second Fab fragment which is in turn fused at the C-terminus of its heavy chain to the N-terminus of the first Fc domain subunit, or the second Fab fragment is fused at the C-terminus of its heavy chain to the N-terminus of the heavy chain of the first Fab fragment which is in turn fused at the C-terminus of its heavy chain to the N-terminus of the first Fc domain subunit, additionally the Fab light chain of the first Fab fragment and the Fab light chain of the second Fab fragment are fused to each other, optionally via a peptide linker (see examples in Figure 12).

According to these embodiments, two Fab fragments of different specificity are fused to each other, one of which is in turn fused to an Fc domain subunit. This configuration allows for a geometry (e.g. distance, angle between the Fab fragments) different from the classical bispecific immunoglobulin format with the two Fab fragments of the immunoglobulin molecule having different specificities. For example, the inventors found that this configuration is more suitable than the classical bispecific immunoglobulin format for mimicking an immunological synapse between a T cell and a target cell, as required if the bispecific antigen binding molecule is to be used for T cell engagement and re-direction (data not shown).

In other embodiments, the second Fab fragment is fused at its C-terminus to the N-terminus of the first Fc domain subunit, which is in turn fused at its C-terminus to the N-terminus of the first Fab fragment (see example in Figure 1D). In one such embodiment, the second Fab fragment is fused to the first Fc domain subunit via an immunoglobulin hinge region. In a further such embodiment, the first Fab fragment is fused to the first Fc domain subunit via a peptide linker. In one embodiment, the second Fab fragment is fused at the C-terminus of its heavy chain to the N-terminus of the first Fc domain subunit, which is in turn fused at its C-terminus to the N-terminus of the heavy chain of the first Fab fragment. According to these embodiments, two Fab fragments of different specificity are fused to the two termini of an Fc domain subunit. Again, this configuration allows for a distinct geometry which might be advantageous for particular applications. In one embodiment the bispecific antigen binding molecule essentially consists of the first Fab fragment, the second Fab fragment, the Fc domain, and optionally one or more peptide linkers.

The bispecific antigen binding molecule disclosed herein provides monovalent binding to at least one of the two antigens it binds to. Monovalent binding is important, for example in cases where internalization of the target antigen is to be expected following binding of a high affinity antigen binding molecule. In such cases, the presence of more than one antigen binding moiety specific for the target antigen may enhance internalization of the antigen, thereby reducing its availablity.

Furthermore, monovalent binding is essential where crosslinking of target antigen is not desired.

For example in bispecific antigen binding molecules for T cell engagement and re-direction, bivalent binding to an activating T cell antigen such as CD3 could lead to activation of the T cell even in the absence of target cells.

In other cases, however, bivalent binding might be desirable, for example to increase binding affinity, optimize targeting to the target site or allow crosslinking of a target antigen.

Accordingly, in particular embodiments, the bispecific antigen binding molecule comprises a third Fab fragment which specifically binds to the first or the second antigen. In one embodiment, the third Fab fragment is fused to the second Fc domain subunit. In a more specific embodiment, the third Fab fragment is fused at its C-terminus to the N-terminus of the second Fc domain subunit. In an even more specific embodiment, the third Fab fragment is fused at the C-terminus of its heavy chain to the N-terminus of the second Fc domain subunit. In one embodiment, the third Fab fragment is fused to the second Fc domain subunit via an immunoglobulin hinge region.

In one embodiment, the third Fab fragment specifically binds to the second antigen.

In some embodiments the second Fab fragment, the third Fab fragment and the Fc domain are part of an immunoglobulin molecule. In embodiments where the third Fab fragment specifically binds to the second antigen, the immunoglobulin molecule is an immunoglobulin molecule which specifically binds to the second antigen. In a specific such embodiment, the immunoglobulin molecule is an IgG class immunoglobulin molecule, more specifically an IgG1 or IgG4 subclass immunoglobulin molecule. In one specific embodiment the immunoglobulin molecule is an IgG4 molecule comprising an amino acid substitution at position S228 (EU numbering), particularly the amino acid substitution S228P. This amino acid substitution reduces in vivo Fab arm exchange of IgG₄ antibodies (see Stubenrauch et al., Drug Metabolism and Disposition 38, 84-91 (2010)). In one embodiment, the immunoglobulin molecule is a human immunoglobulin molecule. In one embodiment, the bispecific antigen binding molecule essentially consists of a first Fab fragment which specifically binds to the first antigen, an immunoglobulin molecule which specifically binds to the second antigen, and optionally one or more peptide linkers.

According to some of the above embodiments, the light chain of the first Fab fragment and the light chain of the second Fab fragment are fused to each other, optionally via a peptide linker.

Depending on the configuration of the first and the second Fab fragment, the light chain of the first Fab fragment may be fused at its C-terminus to the N-terminus of the light chain of the second Fab fragment, or the light chain of the second Fab fragment may be fused at its C-terminus to the N-terminus of the light chain of the first Fab fragment. Fusion of the light chains of the first and the second Fab fragment further reduces mispairing of unmatched Fab heavy and light chains, and also reduces the number of plasmids needed for expression of some of the bispecific antigen binding molecules disclosed herein.

According to any of the above embodiments, components of the bispecific antigen binding molecule (e.g. Fab fragments, Fc domain subunit) may be linked directly or through various linkers, particularly peptide linkers comprising one or more amino acids, typically about 2-20 amino acids, that are described herein or are known in the art. Suitable, non-immunogenic peptide linker include, for example, (G₄S)ₙ, (SG₄)ₙ, (G₄S)ₙ or G₄(SG₄)ₙ peptide linkers, wherein n is generally a number between 1 and 10, typically between 2 and 4. A particularly suitable peptide linker for fusing the light chains of the first and the second Fab fragment to each other is (G₄S)₂. Additionally, peptide linkers may comprise (a portion of) an immunoglobulin hinge region. An exemplary such linker is EPKSC(D)-(G₄S)₂ (SEQ ID NOs 72 and 73). Particularly where a Fab fragment is linked to the N-terminus of an Fc domain subunit, it may be linked via an immunoglobulin hinge region or a portion thereof, with or without an additional peptide linker.

In certain embodiments the bispecific antigen binding molecule comprises a polypeptide wherein a VL region shares a carboxy-terminal peptide bond with a CH1 region, which in turn shares a carboxy-terminal peptide bond with a peptide linker, which in turn shares a carboxy-terminal peptide bond with an immunoglobulin heavy chain (VH-CH1-HR-CH2-CH3-(CH4)). In some of these embodiments, the bispecific antigen binding molecule further comprises an antibody light chain (VL-CL) and/or a polypeptide wherein a VH region shares a carboxy terminal peptide bond with a CL region. In some of these embodiments, the bispecific antigen binding molecule further comprises a polypeptide wherein a VH region shares a carboxy-terminal peptide bond with a CL region, which in turn shares a carboxy-terminal peptide bond with a peptide linker, which in turn shares a carboxy-terminal peptide bond with a Fab light chain (VL-CL).

In other embodiments the bispecific antigen binding molecule comprises a polypeptide wherein a Fab heavy chain (VH-CH1) shares a carboxy-terminal peptide bond with a peptide linker, which in turn shares a carboxy-terminal peptide bond with a VL region, which in turn shares a carboxy-terminal peptide bond with a CH1 region, which in turn shares a carboxy-terminal peptide bond with an Fc domain subunit including an immunoglobulin hinge region (HR-CH2-CH3-(CH4)).

In some of these embodiments, the bispecific antigen binding molecule further comprises an antibody light chain (VL-CL) and/or a polypeptide wherein a VH region shares a carboxy terminal peptide bond with a CL region. In some of these embodiments, the bispecific antigen binding molecule further comprises a polypeptide wherein a Fab light chain (VL-CL) shares a carboxy-terminal peptide bond with a peptide linker, which in turn shares a carboxy-terminal peptide bond with a VH region, which in turn shares a carboxy-terminal peptide bond with a CL region.

In certain embodiments the bispecific antigen binding molecule comprises a polypeptide wherein a VH region shares a carboxy-terminal peptide bond with a CL region, which in turn shares a carboxy-terminal peptide bond with a peptide linker, which in turn shares a carboxy-terminal peptide bond with an immunoglobulin heavy chain (VH-CH1-HR-CH2-CH3-(CH4)). In some of these embodiments, the bispecific antigen binding molecule further comprises an antibody light chain (VL-CL) and/or a polypeptide wherein a VL region shares a carboxy terminal peptide bond with a CH1 region. In some of these embodiments, the bispecific antigen binding molecule further comprises a polypeptide wherein a VL region shares a carboxy-terminal peptide bond with a CH1 region, which in turn shares a carboxy-terminal peptide bond with a peptide linker, which in turn shares a carboxy-terminal peptide bond with a Fab light chain (VL-CL).

In other embodiments the bispecific antigen binding molecule comprises a polypeptide wherein a Fab heavy chain (VH-CH1) shares a carboxy-terminal peptide bond with a peptide linker, which in turn shares a carboxy-terminal peptide bond with a VH region, which in turn shares a carboxy-terminal peptide bond with a CL region, which in turn shares a carboxy-terminal peptide bond with an Fc domain subunit including an immunoglobulin hinge region (HR-CH2-CH3-(CH4)).

In some of these embodiments, the bispecific antigen binding molecule further comprises an antibody light chain (VL-CL) and/or a polypeptide wherein a VL region shares a carboxy terminal peptide bond with a CH1 region. In some of these embodiments, the bispecific antigen binding molecule further comprises a polypeptide wherein a Fab light chain (VL-CL) shares a carboxy-terminal peptide bond with a peptide linker, which in turn shares a carboxy-terminal peptide bond with a VL region, which in turn shares a carboxy-terminal peptide bond with a CH1 region.

In still other embodiments, the bispecific antigen binding molecule comprises a polypeptide wherein an immunoglobulin heavy chain ((VH-CH1-HR-CH2-CH3-(CH4)) shares a carboxy-terminal peptide bond with a peptide linker, which in turn shares a carboxy-terminal peptide bond with a VH region, which in turn shares a carboxy-terminal peptide bond with a CL region.

In one embodiment, the bispecific antigen binding molecule further comprises an immunoglobulin heavy chain ((VH-CH1-HR-CH2-CH3-(CH4)). In another embodiment, the bispecific antigen binding molecule further comprises an Fc domain subunit, optionally including an antibody hinge region ((HR)-CH2-CH3-(CH4)). In some of these embodiments, the bispecific antigen binding molecule further comprises an antibody light chain (VL-CL) and/or a polypeptide wherein a VL region shares a carboxy terminal peptide bond with a CH1 region.

### Fab fragments

The antigen binding molecule disclosed herein is bispecific, i.e. it comprises at least two antigen binding moieties capable of specific binding to two distinct antigenic determinants. In a particular embodiment, the bispecific antigen binding molecule is capable of simultaneous binding to two distinct antigenic determinants. According to the present disclosure, the antigen binding moieties are Fab fragments (i.e. antigen binding domains composed of a heavy and a light chain, each comprising a variable and a constant region). In one embodiment said Fab fragments are human. In another embodiment said Fab fragments are humanized. In yet another embodiment said Fab fragments comprise human heavy and light chain constant regions.

According to the present disclosure, at least one of the Fab fragments is a "Crossfab" fragment, wherein the variable and/or constant domains of the Fab heavy and light chain are exchanged. Such modifications prevent mispairing of heavy and light chains from different Fab fragments, thereby improving the yield and purity of the bispecific antigen binding molecule disclosed herein in recombinant production. In other words, the problem of heavy and light chain mispairing in bispecific antibody production is overcome by the exchange of heavy and light chain variable and/or constant domains within one or more Fab fragments of the bispecific antigen binding molecule, so that Fab fragments of different specificity do not have identical domain arrangement and consequently do not "interchange" light chains.

Possible replacements include the following: (i) the variable domains of the Fab heavy and light chain (VH and VL) are replaced by each other; (ii) the constant domains of the Fab heavy and light chain (CH1 and CL) are replaced by each other; or (iii) the Fab heavy and light chain (VH-CH1 and VL-CL) are replaced by each other.

To achieve the desired result, i.e. prevention of mispairing of heavy and light chains of different specificity, not the same replacement must be made in Fab fragments of different specificity. For example, in a Fab fragment which specifically binds to a first antigen, the heavy and light chain variable domains may be exchanged, while in a Fab fragment which specifically binds to a second antigen, the heavy and light chain constant region may be exchanged. As another example, in a Fab fragment which specifically binds to a first antigen, no replacement may be made, while in a Fab fragment which specifically binds to a second antigen, the heavy and light chain variable domains may be exchanged.

In a particular embodiment, the same replacement is made in Fab fragments of the same specificity (i.e. in Fab fragments which specifically bind to the same antigen). A replacement need not be made in all Fab fragments comprised in the bispecific antigen binding molecule. For example in embodiments wherein there are three Fab fragments, it is sufficient to make a replacement only in the Fab fragment having a different specificity from the other two Fab fragments. Specifically, in embodiments wherein the bispecific antigen binding molecule comprises a third Fab fragment which binds to the first antigen, a replacement is made only in the second Fab fragment. Similarly, in embodiments wherein the bispecific antigen binding molecule comprises a third Fab fragment which binds to the second antigen, a replacement is made only in the first Fab fragment.

In particular embodiments, a replacement is made in the first Fab fragment. In one such embodiment, no further replacement is made. In some embodiments, the replacement is a replacement of the variable domains VL and VH by each other. In other embodiments the replacement is a replacement of the constant domains CL and CH1 by each other. In still other embodiments, the replacement is a replacement of both the variable and constant domains VL-CL and VH-CH1 by each other.

In a particular embodiment, the bispecific antigen binding molecule provides monovalent binding to the first antigen. In one embodiment, the bispecific antigen binding molecule does not comprise a single chain Fab fragment.

In a particular aspect, provided herein is a bispecific antigen binding molecule comprising a first Fab fragment which specifically binds to a first antigen, a second Fab fragment which specifically binds to a second antigen, and an Fc domain composed of a first and a second subunit capable of stable association; wherein
a) the bispecific antigen binding molecule provides monovalent binding to the first antigen,
b) the first Fab fragment is fused at its C-terminus to the N-terminus of the second Fab fragment, which is in turn fused at its C-terminus to the N-terminus of the first Fc domain subunit,
c) in the first Fab fragment the constant domains CL and CH1 are replaced by each other, and
d) the bispecific antigen binding molecule optionally comprises a third Fab fragment which specifically binds to the second antigen and is fused at its C-terminus to the N-terminus of the second Fc domain subunit.

In another aspect, provided herein is a bispecific antigen binding molecule comprising a first Fab fragment which specifically binds to a first antigen, a second Fab fragment which specifically binds to a second antigen, and an Fc domain composed of a first and a second subunit capable of stable association; wherein
a) the bispecific antigen binding molecule provides monovalent binding to the first antigen,
b) the first Fab fragment is fused at its C-terminus to the N-terminus of the second Fab fragment, which is in turn fused at its C-terminus to the N-terminus of the first Fc domain subunit,
c) in the first Fab fragment the variable domains VL and VH are replaced by each other, and
d) the bispecific antigen binding molecule optionally comprises a third Fab fragment which specifically binds to the second antigen and is fused at its C-terminus to the N-terminus of the second Fc domain subunit.

In a further particular aspect, provided herein is a bispecific antigen binding molecule, comprising a first Fab fragment which specifically binds to a first antigen, a second Fab fragment which specifically binds to a second antigen, and an Fc domain composed of a first and a second subunit capable of stable association; wherein
a) the bispecific antigen binding molecule provides monovalent binding to the first antigen,
b) the second Fab fragment is fused at its C-terminus to the N-terminus of the first Fab fragment, which is in turn fused at its C-terminus to the N-terminus of the first Fc domain subunit,
c) in the first Fab fragment the constant domains CL and CH1 are replaced by each other, and
d) the bispecific antigen binding molecule optionally comprises a third Fab fragment which specifically binds to the second antigen and is fused at its C-terminus to the N-terminus of the second Fc domain subunit.

In a further aspect, provided herein is a bispecific antigen binding molecule, comprising a first Fab fragment which specifically binds to a first antigen, a second Fab fragment which specifically binds to a second antigen, and an Fc domain composed of a first and a second subunit capable of stable association; wherein
a) the bispecific antigen binding molecule provides monovalent binding to the first antigen,
b) the second Fab fragment is fused at its C-terminus to the N-terminus of the first Fab fragment, which is in turn fused at its C-terminus to the N-terminus of the first Fc domain subunit,
c) in the first Fab fragment the variable domains VL and VH are replaced by each other, and
d) the bispecific antigen binding molecule optionally comprises a third Fab fragment which specifically binds to the second antigen and is fused at its C-terminus to the N-terminus of the second Fc domain subunit.

In yet a further aspect, provided herein is a bispecific antigen binding molecule, comprising a first Fab fragment which specifically binds to a first antigen, a second Fab fragment which specifically binds to a second antigen, and an Fc domain composed of a first and a second subunit capable of stable association; wherein
a) the bispecific antigen binding molecule provides monovalent binding to the first antigen,
b) the second Fab fragment is fused at its C-terminus to the N-terminus of the first Fc domain subunit, which is in turn fused at its C-terminus to the N-terminus of the first Fab fragment,
c) in the first Fab fragment the constant domains CL and CH1 are replaced by each other, and
d) the bispecific antigen binding molecule optionally comprises a third Fab fragment which specifically binds to the second antigen and is fused at its C-terminus to the N-terminus of the second Fc domain subunit.

### Fc domain

The Fc domain of the bispecific antigen binding molecule consists of a pair of polypeptide chains comprising heavy chain domains of an antibody molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other. The bispecific antigen binding molecule disclosed herein comprises not more than one Fc domain.

In one embodiment the Fc domain of the bispecific antigen binding molecule is an IgG Fc domain. In a particular embodiment the Fc domain is an IgG₁ Fc domain. In another embodiment the Fc domain is an IgG₄ Fc domain. In a more specific embodiment, the Fc domain is an IgG₄ Fc domain comprising an amino acid substitution at position S228 (EU numbering), particularly the amino acid substitution S228P. This amino acid substitution reduces in vivo Fab arm exchange of IgG₄ antibodies (see Stubenrauch et al., Drug Metabolism and Disposition 38, 84-91 (2010)). In a further particular embodiment the Fc domain is human. An exemplary sequence of a human IgG₁ Fc region is given in SEQ ID NO: 71.

### Fc domain modifications promoting heterodimerization

Bispecific antigen binding molecules disclosed herein comprise different Fab fragments, fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of bispecific antigen binding molecules in recombinant production, it will thus be advantageous to introduce in the Fc domain of the bispecific antigen binding molecule a modification promoting the association of the desired polypeptides.

Accordingly, in particular embodiments, the Fc domain comprises a modification promoting the association of the first and the second Fc domain subunit. A modification may be present in the first Fc domain subunit and/or the second Fc domain subunit.

The site of most extensive protein-protein interaction between the two subunits of a human IgG Fc domain is in the CH3 domain of the Fc domain. Thus, in one embodiment said modification is in the CH3 domain of the Fc domain.

In a specific embodiment said modification is a so-called "knob-into-hole" modification, comprising a "knob" modification in one of the two subunits of the Fc domain and a "hole" modification in the other one of the two subunits of the Fc domain.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan).

Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first Fc domain subunit of the bispecific antigen binding molecule an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second Fc domain subunit an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific embodiment, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A).

In yet a further embodiment, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in formation of a disulfide bridge between the two subunits of the Fc domain, further stabilizing the dimer (Carter, J Immunol Methods 248, 7-15 (2001)).

In an alternative embodiment a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

### Fc domain modifications altering Fc receptor binding and/or effector function

In certain embodiments, the Fc domain is engineered to have altered binding affinity to an Fc receptor and/or altered effector function, as compared to a non-engineered Fc domain.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or bispecific antigen binding molecules comprising an Fc domain for Fc receptors may be evaluated using cell lines known to express particular Fc receptors, such as NK cells expressing FcγIIIa receptor.

Effector function of an Fc domain, or a bispecific antigen binding molecule comprising an Fc domain, can be measured by methods known in the art. Suitable *in vitro* assays to assess ADCC activity of a molecule of interest are described in PCT publication no. WO 2006/082515 or PCT publication no. WO 2012/130831. Useful effector cells for such assays include peripheral blood mononuclear cells (PBMC) and Natural Killer (NK) cells. Alternatively, or additionally, ADCC activity of the molecule of interest may be assessed in vivo, e.g. in a animal model such as that disclosed in Clynes et al., Proc Natl Acad Sci USA 95, 652-656 (1998).

In some embodiments binding of the Fc domain to a complement component, specifically to C1q, is altered. Accordingly, in some embodiments wherein the Fc domain is engineered to have altered effector function, said altered effector function includes altered CDC. C1q binding assays may be carried out to determine whether the bispecific antigen binding molecule is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

### a) Decreased Fc receptor binding and/or effector function

The Fc domain confers to the bispecific antigen binding molecule favorable pharmacokinetic properties, including a long serum half-life which contributes to good accumulation in the target tissue and a favorable tissue-blood distribution ratio. At the same time it may, however, lead to undesirable targeting of the bispecific antigen binding molecule to cells expressing Fc receptors rather than to the preferred antigen-bearing cells. Moreover, the activation of Fc receptor signaling pathways may lead to cytokine release and severe side effects upon systemic administration.

Accordingly, in particular embodiments, the Fc domain of the bispecific antigen binding molecule is engineered to have reduced binding affinity to an Fc receptor and/or reduced effector function, as compared to a non-engineered Fc domain. In one such embodiment the Fc domain (or the bispecific antigen binding molecule comprising said Fc domain) exhibits less than 50%, preferably less than 20%, more preferably less than 10% and most preferably less than 5% of the binding affinity to an Fc receptor, as compared to a non-engineered Fc domain (or a bispecific antigen binding molecule comprising a non-engineered Fc domain), and/or less than 50%, preferably less than 20%, more preferably less than 10% and most preferably less than 5% of the effector function, as compared to a non-engineered Fc domain domain (or a bispecific antigen binding molecule comprising a non-engineered Fc domain). In one embodiment, the Fc domain domain (or the bispecific antigen binding molecule comprising said Fc domain) does not substantially bind to an Fc receptor and/or induce effector function. In a particular embodiment the Fc receptor is an Fcγ receptor. In one embodiment the Fc receptor is a human Fc receptor. In one embodiment the Fc receptor is an activating Fc receptor. In a specific embodiment the Fc receptor is an activating human Fcγ receptor, more specifically human FcγRIIIa, FcγRI or FcγRIIa, most specifically human FcγRIIIa. In one embodiment the effector function is one or more selected from the group of CDC, ADCC, ADCP, and cytokine secretion. In a particular embodiment the effector function is ADCC. In one embodiment the Fc domain domain exhibits substantially similar binding affinity to neonatal Fc receptor (FcRn), as compared to a non-engineered Fc domain. Substantially similar binding to FcRn is achieved when the Fc domain (or the bispecific antigen binding molecule comprising said Fc domain) exhibits greater than about 70%, particularly greater than about 80%, more particularly greater than about 90% of the binding affinity of a non-engineered Fc domain (or the bispecific antigen binding molecule comprising a non-engineered Fc domain) to FcRn.

In certain embodiments, the Fc domain of the bispecific antigen binding molecule comprises one or more amino acid mutation that reduces the binding affinity of the Fc domain to an Fc receptor and/or effector function. Typically, the same one or more amino acid mutation is present in each of the two subunits of the Fc domain. In one embodiment the amino acid mutation reduces the binding affinity of the Fc domain to an Fc receptor. In one embodiment the amino acid mutation reduces the binding affinity of the Fc domain to an Fc receptor by at least 2-fold, at least 5-fold, or at least 10-fold. In embodiments where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In a particular embodiment the Fc receptor is an Fcγ receptor. In some embodiments the Fc receptor is a human Fc receptor. In some embodiments the Fc receptor is an activating Fc receptor. In a specific embodiment the Fc receptor is an activating human Fcγ receptor, more specifically human FcγRIIIa, FcγRI or FcγRIIa, most specifically human FcγRIIIa. Preferably, binding to each of these receptors is reduced. In some embodiments binding affinity to a complement component, specifically binding affinity to C1q, is also reduced. In one embodiment binding affinity to neonatal Fc receptor (FcRn) is not reduced.

In certain embodiments the Fc domain of the bispecific antigen binding molecule is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced crosslinking of target-bound antibodies, reduced dendritic cell maturation, or reduced T cell priming. In one embodiment the reduced effector function is one or more selected from the group of reduced CDC, reduced ADCC, reduced ADCP, and reduced cytokine secretion. In a particular embodiment the reduced effector function is reduced ADCC. In one embodiment the reduced ADCC is less than 20% of the ADCC induced by a non-engineered Fc domain (or a bispecific antigen binding molecule comprising a non-engineered Fc domain).

In one embodiment the amino acid mutation that reduces the binding affinity of the Fc domain to an Fc receptor and/or effector function is an amino acid substitution. In one embodiment the Fc domain comprises an amino acid substitution at a position selected from the group of E233, L234, L235, N297, P331 and P329. In a more specific embodiment the Fc domain comprises an amino acid substitution at a position selected from the group of L234, L235 and P329. In some embodiments the Fc domain comprises the amino acid substitutions L234A and L235A. In one such embodiment, the Fc domain is an IgG₁ Fc domain, particularly a human IgG₁ Fc domain. In one embodiment the Fc domain comprises an amino acid substitution at position P329. In a more specific embodiment the amino acid substitution is P329A or P329G, particularly P329G. In one embodiment the Fc domain comprises an amino acid substitution at position P329 and a further amino acid substitution at a position selected from E233, L234, L235, N297 and P331. In a more specific embodiment the further amino acid substitution is E233P, L234A, L235A, L235E, N297A, N297D or P331S. In particular embodiments the Fc domain comprises amino acid substitutions at positions P329, L234 and L235. In more particular embodiments the Fc domain comprises the amino acid mutations L234A, L235A and P329G ("P329G LALA"). In one such embodiment, the Fc domain is an IgG₁ Fc domain, particularly a human IgG₁ Fc domain. The "P329G LALA" combination of amino acid substitutions almost completely abolishes Fcγ receptor binding of a human IgG₁ Fc domain, as described in PCT publication no. WO 2012/130831. WO 2012/130831 also describes methods of preparing such mutant Fc domains and methods for determining its properties such as Fc receptor binding or effector functions.

IgG₄ antibodies exhibit reduced binding affinity to Fc receptors and reduced effector functions as compared to IgG₁ antibodies. Hence, in some embodiments the Fc domain of the bispecific antigen binding molecules disclosed herein is an IgG₄ Fc domain, particularly a human IgG₄ Fc domain. In one embodiment the IgG₄ Fc domain comprises amino acid substitutions at position S228, specifically the amino acid substitution S228P. To further reduce its binding affinity to an Fc receptor and/or its effector function, in one embodiment the IgG₄ Fc domain comprises an amino acid substitution at position L235, specifically the amino acid substitution L235E. In another embodiment, the IgG₄ Fc domain comprises an amino acid substitution at position P329, specifically the amino acid substitution P329G. In a particular embodiment, the IgG₄ Fc domain comprises amino acid substitutions at positions S228, L235 and P329, specifically amino acid substitutions S228P, L235E and P329G. Such IgG₄ Fc domain mutants and their Fcγ receptor binding properties are described in PCT publication no. WO 2012/130831.

In a particular embodiment the Fc domain exhibiting reduced binding affinity to an Fc receptor and/or reduced effector function, as compared to a native IgG₁ Fc domain, is a human IgG₁ Fc domain comprising the amino acid substitutions L234A, L235A and optionally P329G, or a human IgG₄ Fc domain comprising the amino acid substitutions S228P, L235E and optionally P329G.

In certain embodiments N-glycosylation of the Fc domain has been eliminated. In one such embodiment the Fc domain comprises an amino acid mutation at position N297, particularly an amino acid substitution replacing asparagine by alanine (N297A) or aspartic acid (N297D).

In addition to the Fc domains described hereinabove and in PCT publication no. WO 2012/130831, Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

### b) Increased Fc receptor binding and/or effector function

Conversely, there may be situations where it is desirable to maintain or even enhance Fc receptor binding and/or effector functions of the bispecific antigen binding molecules, for example when the bispecific antigen binding molecule is targeted to a highly specific tumor antigen. Hence, in certain embodiments the Fc domain of the bispecific antigen binding molecules disclosed herein is engineered to have increased binding affinity to an Fc receptor. Increased binding affinity may be an increase in the binding affinity of the Fc domain to the Fc receptor by at least 2-fold, at least 5-fold, or at least 10-fold. In one embodiment the Fc receptor is an activating Fc receptor.

In a specific embodiment the Fc receptor is an Fcγ receptor, particularly a human Fcγ receptor.

In one embodiment the Fc receptor is selected from the group of FcγRIIIa, FcγRI and FcγRIIa.

In a particular embodiment the Fc receptor is FcγRIIIa.

In one such embodiment the Fc domain is engineered to have an altered oligosaccharide structure compared to a non-engineered Fc domain. In a particular such embodiment the Fc domain comprises an increased proportion of non-fucosylated oligosaccharides, compared to a non-engineered Fc domain. In a more specific embodiment, at least about 50%, more particularly at least about 70%, of the N-linked oligosaccharides in the Fc domain of the bispecific antigen binding molecule are non-fucosylated. The non-fucosylated oligosaccharides may be of the hybrid or complex type. In another specific embodiment the Fc domain comprises an increased proportion of bisected oligosaccharides, compared to a non-engineered Fc domain. In a more specific embodiment, at least about 35%, particularly at least about 50%, more particularly at least about 70%, of the N-linked oligosaccharides in the Fc domain of the bispecific antigen binding molecule are bisected. The bisected oligosaccharides may be of the hybrid or complex type. In yet another specific embodiment the Fc domain comprises an increased proportion of bisected, non-fucosylated oligosaccharides, compared to a non-engineered Fc domain. In a more specific embodiment, at least about 15%, more particularly at least about 25%, at least about 35% or at least about 50%, of the N-linked oligosaccharides in the Fc domain of the bispecific antigen binding molecule are bisected, non-fucosylated. The bisected, non-fucosylated oligosaccharides may be of the hybrid or complex type.

The oligosaccharide structures in the bispecific antigen binding molecule Fc domain can be analysed by methods well known in the art, e.g. by MALDI TOF mass spectrometry as described in Umana et al., Nat Biotechnol 17, 176-180 (1999) or Ferrara et al., Biotechn Bioeng 93, 851-861 (2006). The percentage of non-fucosylated oligosaccharides is the amount of oligosaccharides lacking fucose residues, relative to all oligosaccharides attached to Asn 297 (e.g. complex, hybrid and high mannose structures) and identified in an N-glycosidase F treated sample by MALDI TOF MS. Asn 297 refers to the asparagine residue located at about position 297 in the Fc domain (EU numbering of Fc region residues); however, Asn297 may also be located about ± 3 amino acids upstream or downstream of position 297, i.e., between positions 294 and 300, due to minor sequence variations in immunoglobulins. The percentage of bisected, or bisected non-fucosylated, oligosaccharides is determined analogously.

Modification of the glycosylation in the Fc domain of the bispecific antigen binding molecule may result from production of the bispecific antigen binding molecule in a host cell that has been manipulated to express altered levels of one or more polypeptides having glycosyltransferase activity.

In one embodiment the Fc domain of the bispecific antigen binding molecule is engineered to have an altered oligosaccharide structure, as compared to a non-engineered Fc domain, by producing the bispecific antigen binding molecule in a host cell having altered activity of one or more glycosyltransferase. Glycosyltransferases include for example β(1,4)-N-acetylglucosaminyltransferase III (GnTIII), β(1,4)-galactosyltransferase (GalT), β(1,2)-N-acetylglucosaminyltransferase I (GnTI), β(1,2)-N-acetylglucosaminyltransferase II (GnTII) and α(1,6)-fucosyltransferase. In a specific embodiment the Fc domain of the bispecific antigen binding molecule is engineered to comprise an increased proportion of non-fucosylated oligosaccharides, as compared to a non-engineered Fc domain, by producing the bispecific antigen binding molecule in a host cell having increased β(1,4)-N-acetylglucosaminyltransferase III (GnTIII) activity. In an even more specific embodiment the host cell additionally has increased α-mannosidase II (ManII) activity. The glycoengineering methodology that can be used for glycoengineering bispecific antigen binding molecules disclosed herein has been described in greater detail in Umana et al., Nat Biotechnol 17, 176-180 (1999); Ferrara et al., Biotechn Bioeng 93, 851-861 (2006); WO 99/54342 (U.S. Pat. No. 6,602,684; EP 1071700); WO 2004/065540 (U.S. Pat. Appl. Publ. No. 2004/0241817; EP 1587921), WO 03/011878 (U.S. Pat. Appl. Publ. No. 2003/0175884).

Generally, any type of cultured cell line, including the cell lines discussed herein, can be used to generate cell lines for the production of bispecific antigen binding molecules with altered glycosylation pattern. Particular cell lines include CHO cells, BHK cells, NS0 cells, SP2/0 cells, YO myeloma cells, P3X63 mouse myeloma cells, PER cells, PER.C6 cells or hybridoma cells, and other mammalian cells. In certain embodiments, the host cells have been manipulated to express increased levels of one or more polypeptides having β(1,4)-N-acetylglucosaminyltransferase III (GnTIII) activity. In certain embodiments the host cells have been further manipulated to express increased levels of one or more polypeptides having α-mannosidase II (ManII) activity. In a specific embodiment, the polypeptide having GnTIII activity is a fusion polypeptide comprising the catalytic domain of GnTIII and the Golgi localization domain of a heterologous Golgi resident polypeptide. Particularly, said Golgi localization domain is the Golgi localization domain of mannosidase II. Methods for generating such fusion polypeptides and using them to produce antibodies with increased effector functions are disclosed in Ferrara et al., Biotechn Bioeng 93, 851-861 (2006) and WO 2004/065540.

The host cells which contain a coding sequence of a bispecific antigen binding molecule disclosed herein and/or a coding sequence of a polypeptide having glycosyltransferase activity, and which express the biologically active gene products, may be identified e.g. by DNA-DNA or DNA-RNA hybridization, the presence or absence of "marker" gene functions, assessing the level of transcription as measured by the expression of the respective mRNA transcripts in the host cell, or detection of the gene product as measured by immunoassay or by its biological activity - methods which are well known in the art. GnTIII or Man II activity can be detected e.g. by employing a lectin which binds to biosynthesis products of GnTIII or ManII, respectively. An example for such a lectin is the E₄-PHA lectin which binds preferentially to oligosaccharides containing bisecting GlcNAc. Biosynthesis products (i.e. specific oligosaccharide structures) of polypeptides having GnTIII or ManII activity can also be detected by mass spectrometric analysis of oligosaccharides released from glycoproteins produced by cells expressing said polypeptides. Alternatively, a functional assay which measures the increased effector function and/or increased Fc receptor binding, mediated by bispecific antigen binding molecules produced by the cells engineered with the polypeptide having GnTIII or ManII activity may be used.

In another embodiment the Fc domain is engineered to comprise an increased proportion of non-fucosylated oligosaccharides, as compared to a non-engineered Fc domain, by producing the bispecific antigen binding molecule in a host cell having decreased α(1,6)-fucosyltransferase activity. A host cell having decreased α(1,6)-fucosyltransferase activity may be a cell in which the α(1,6)-fucosyltransferase gene has been disrupted or otherwise deactivated, e.g. knocked out (see Yamane-Ohnuki et al., Biotech Bioeng 87, 614 (2004); Kanda et al., Biotechnol Bioeng 94(4), 680-688 (2006); Niwa et al., J Immunol Methods 306, 151-160 (2006)).

Other examples of cell lines capable of producing defucosylated bispecific antigen binding molecules include Lec13 CHO cells deficient in protein fucosylation (Ripka et al., Arch Biochem Biophys 249, 533-545 (1986); US Pat. Appl. No. US 2003/0157108; and WO 2004/056312, especially at Example 11). The bispecific antigen binding molecules disclosed herein can alternatively be glycoengineered to have reduced fucose residues in the Fc domain according to the techniques disclosed in EP 1 176 195 A1, WO 03/084570, WO 03/085119 and U.S. Pat. Appl. Pub. Nos. 2003/0115614, 2004/093621, 2004/110282, 2004/110704, 2004/132140, US Pat. No. 6,946,292 (Kyowa), e.g. by reducing or abolishing the activity of a GDP-fucose transporter protein in the host cells used for bispecific antigen binding molecule production.

Glycoengineered bispecific antigen binding molecules disclosed herein may also be produced in expression systems that produce modified glycoproteins, such as those taught in WO 2003/056914 (GlycoFi, Inc.) or in WO 2004/057002 and WO 2004/024927 (Greenovation).

In one embodiment the Fc domain of the bispecific antigen binding molecule is engineered to have increased effector function, compared to a non-engineered Fc domain. The increased effector function can include, but is not limited to, one or more of the following: increased complement dependent cytotoxicity (CDC), increased antibody-dependent cell-mediated cytotoxicity (ADCC), increased antibody-dependent cellular phagocytosis (ADCP), increased cytokine secretion, increased immune complex-mediated antigen uptake by antigen-presenting cells, increased binding to NK cells, increased binding to macrophages, increased binding to monocytes, increased binding to polymorphonuclear cells, increased direct signaling inducing apoptosis, increased crosslinking of target-bound antibodies, increased dendritic cell maturation, or increased T cell priming.

In one embodiment the increased effector function is one or more selected from the group of increased CDC, increased ADCC, increased ADCP, and increased cytokine secretion. In a particular embodiment the increased effector function is increased ADCC. In one embodiment ADCC induced by an engineered Fc domain (or a bispecific antigen binding molecule comprising an engineered Fc domain) is a least 2-fold increased as compared to ADCC induced by a non-engineered Fc domain (or a bispecific antigen binding molecule comprising a non-engineered Fc domain).

### Antigens

The bispecific antigen binding molecules disclosed herein may bind to a variety of antigens. In certain embodiments the first and/or second antigen is an antigen associated with a pathological condition, such as an antigen presented on a tumor cell, on a virus-infected cell, or at a site of inflammation. Suitable antigens include cell surface antigens (for example, but not limited to, cell surface receptors), antigens free in blood serum, and/or antigens in the extracellular matrix.

In particular embodiments the antigen is a human antigen.

Non-limiting examples of antigens include tumor antigens such as MAGE, MART-1/Melan-A, gp100, Dipeptidyl peptidase IV (DPPIV), adenosine deaminase-binding protein (ADAbp), cyclophilin b, Colorectal associated antigen (CRC)-C017-1A/GA733, Carcinoembryonic Antigen (CEA) and its immunogenic epitopes CAP-1 and CAP-2, etv6, aml1, Prostate Specific Antigen (PSA) and its immunogenic epitopes PSA-1, PSA-2, and PSA-3, prostate-specific membrane antigen (PSMA), T-cell receptor/CD3-zeta chain, MAGE-family of tumor antigens (e.g., MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, Her3, p21ras, RCAS1, α-fetoprotein, E-cadherin, α-catenin, β-catenin and γ-catenin, p120ctn, gp100 Pmel117, PRAME, NY-ESO-1, cdc27, adenomatous polyposis coli protein (APC), fodrin, Connexin 37, Ig-idiotype, p15, gp75, GM2 and GD2 gangliosides, Smad family of tumor antigens, lmp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2; ECM antigens such as syndecan, heparanase, integrins, osteopontin, link, cadherins, laminin, laminin type EGF, lectin, fibronectin and its alternatively spliced domains (e.g. the Extra Domain B), notch, various forms of tenascin (e.g. tenascin C) and its alternatively spliced domains (e.g. the A1 or A2 domain of tenascin-C), and matrixin; Fibroblast Activation Protein (FAP), Epidermal Growth Factor Receptor (EGFR), CD2 (T-cell surface antigen), CD3 (heteromultimer associated with the TCR), CD19, CD22 (B-cell receptor), CD23 (low affinity IgE receptor), CD25 (IL-2 receptor α chain), CD30 (cytokine receptor), CD33 (myeloid cell surface antigen), CD40 (tumor necrosis factor receptor), IL-6R (IL6 receptor), CD20, Melanoma-associated Chondroitin Sulfate Proteoglycan (MCSP), Insulin-like growth factor-1 receptor (IGF-1R), and PDGFβR (β platelet-derived growth factor receptor).

In a specific embodiment the first and second antigen are selected from the group of Fibroblast Activation Protein (FAP), Melanoma-associated Chondroitin Sulfate Proteoglycan (MCSP), Epidermal Growth Factor Receptor (EGFR), Her3, c-Met, Carcinoembryonic Antigen (CEA), CD33 and CD3.

In a particular embodiment the first antigen is CD3, particularly human or cynomolgus CD3, most particularly human CD3. In some embodiments, the first antigen is the epsilon subunit of CD3. In one embodiment, the first Fab fragment can compete with monoclonal antibody H2C (described in PCT publication no. WO2008/119567) for binding an epitope of CD3. In a particular embodiment, the first Fab fragment can compete with monoclonal antibody SP34 (described in Pessano et al., EMBO J 4, 337-340 (1985)) for binding an epitope of CD3. In another embodiment, the first Fab fragment can compete with monoclonal antibody V9 (described in Rodrigues et al., Int J Cancer Suppl 7, 45-50 (1992) and US patent no. 6,054,297) for binding an epitope of CD3. In yet another embodiment, the first Fab fragment can compete with monoclonal antibody FN18 (described in Nooij et al., Eur J Immunol 19, 981-984 (1986)) for binding an epitope of CD3. In one embodiment, the first Fab fragment is specific for CD3 and comprises the heavy chain CDR1 of SEQ ID NO: 77, the heavy chain CDR2 of SEQ ID NO: 78, the heavy chain CDR3 of SEQ ID NO: 79, the light chain CDR1 of SEQ ID NO: 81, the light chain CDR2 of SEQ ID NO: 82, and the light chain CDR3 of SEQ ID NO: 83. In a further embodiment, the Fab fragment that is specific for CD3 comprises a heavy chain variable region sequence that is at least about 80%, 85%, 90%, 95%, 96%, 97%, 98%, 99% or 100% identical to SEQ ID NO: 80 and a light chain variable region sequence that is at least about 80%, 85%, 90%, 95%, 96%, 97%, 98%, 99% or 100% identical to SEQ ID NO: 84, or variants thereof that retain functionality. In another embodiment, the first Fab fragment is specific for CD3 and comprises the heavy chain CDR1 of SEQ ID NO: 104, the heavy chain CDR2 of SEQ ID NO: 105, the heavy chain CDR3 of SEQ ID NO: 106, the light chain CDR1 of SEQ ID NO: 108, the light chain CDR2 of SEQ ID NO: 109, and the light chain CDR3 of SEQ ID NO: 110. In a further embodiment, the Fab fragment that is specific for CD3 comprises a heavy chain variable region sequence that is at least about 80%, 85%, 90%, 95%, 96%, 97%, 98%, 99% or 100% identical to SEQ ID NO: 107 and a light chain variable region sequence that is at least about 80%, 85%, 90%, 95%, 96%, 97%, 98%, 99% or 100% identical to SEQ ID NO: 111, or variants thereof that retain functionality.

In a particular embodiment, the bispecific antigen binding molecule is capable of simultaneous binding to a target cell antigen, particularly a tumor cell antigen, and CD3. In one embodiment, the bispecific antigen binding molecule is capable of crosslinking a T cell and a target cell by simultaneous binding to a target cell antigen and CD3. In an even more particular embodiment, such simultaneous binding results in lysis of the target cell, particularly a tumor cell. In one embodiment, such simultaneous binding results in activation of the T cell. In other embodiments, such simultaneous binding results in a cellular response of a T lymphocyte, particularly a cytotoxic T lymphocyte, selected from the group of: proliferation, differentiation, cytokine secretion, cytotoxic effector molecule release, cytotoxic activity, and expression of activation markers. In one embodiment, binding of the bispecific antigen binding molecule to CD3 without simultaneous binding to the target cell antigen does not result in T cell activation.

In one embodiment, the bispecific antigen binding molecule is capable of re-directing cytotoxic activity of a T cell to a target cell. In a particular embodiment, said re-direction is independent of MHC-mediated peptide antigen presentation by the target cell and and/or specificity of the T cell.

Particularly, a T cell according to any of the embodiments of the present disclosure is a cytotoxic T cell. In some embodiments the T cell is a CD4⁺ or a CD8⁺ T cell, particularly a CD8⁺ T cell.

In one embodiment, the first antigen is c-Met, particularly human c-Met. In one embodiment, the first Fab fragment can compete with monoclonal antibody 5D5 (described e.g. in US patent no. 7,476,724) for binding an epitope of c-Met. In one embodiment, the first Fab fragment is specific for c-Met and comprises the heavy chain CDR1 of SEQ ID NO: 63, the heavy chain CDR2 of SEQ ID NO: 64, the heavy chain CDR3 of SEQ ID NO: 65, the light chain CDR1 of SEQ ID NO: 67, the light chain CDR2 of SEQ ID NO: 68, and the light chain CDR3 of SEQ ID NO: 69.

In a further embodiment, the Fab fragment that is specific for c-Met comprises a heavy chain variable region sequence that is at least about 80%, 85%, 90%, 95%, 96%, 97%, 98%, 99% or 100% identical to SEQ ID NO: 66 and a light chain variable region sequence that is at least about 80%, 85%, 90%, 95%, 96%, 97%, 98%, 99% or 100% identical to SEQ ID NO: 70, or variants thereof that retain functionality.

In particular embodiments the second antigen is a tumor-associated antigen, specficially an antigen presented on a tumor cell or a cell of the tumor stroma. In one such embodiment the second antigen is selected from the group of Fibroblast Activation Protein (FAP), Melanoma-associated Chondroitin Sulfate Proteoglycan (MCSP), Epidermal Growth Factor Receptor (EGFR), Her3, CD33, and Carcinoembryonic Antigen (CEA).

In one embodiment the second antigen is Melanoma-associated Chondroitin Sulfate Proteoglycan (MCSP). In another embodiment second and optionally the third Fab fragment can compete with monoclonal antibody LC007 (see SEQ ID NOs 18 and 22) for binding to an epitope of MCSP. In one embodiment, the Fab fragment that is specific for MCSP comprises the heavy chain CDR1 of SEQ ID NO: 15, the heavy chain CDR2 of SEQ ID NO: 16, the heavy chain CDR3 of SEQ ID NO: 17, the light chain CDR1 of SEQ ID NO: 19, the light chain CDR2 of SEQ ID NO: 20, and the light chain CDR3 of SEQ ID NO: 21. In a further embodiment, the Fab fragment that is specific for MCSP comprises a heavy chain variable region sequence that is at least about 80%, 85%, 90%, 95%, 96%, 97%, 98%, 99% or 100% identical to SEQ ID NO: 18 and a light chain variable region sequence that is at least about 80%, 85%, 90%, 95%, 96%, 97%, 98%, 99% or 100% identical to SEQ ID NO: 22, or variants thereof that retain functionality. In another embodiment second and optionally the third Fab fragment can compete with monoclonal antibody M4-3 ML2 (see SEQ ID NOs 99 and 103) for binding to an epitope of MCSP. In one embodiment, the Fab fragment that is specific for MCSP comprises the heavy chain CDR1 of SEQ ID NO: 96, the heavy chain CDR2 of SEQ ID NO: 97, the heavy chain CDR3 of SEQ ID NO: 98, the light chain CDR1 of SEQ ID NO: 100, the light chain CDR2 of SEQ ID NO: 101, and the light chain CDR3 of SEQ ID NO: 102. In a further embodiment, the Fab fragment that is specific for MCSP comprises a heavy chain variable region sequence that is at least about 80%, 85%, 90%, 95%, 96%, 97%, 98%, 99% or 100% identical to SEQ ID NO: 99 and a light chain variable region sequence that is at least about 80%, 85%, 90%, 95%, 96%, 97%, 98%, 99% or 100% identical to SEQ ID NO: 103, or variants thereof that retain functionality.

In yet another embodiment the bispecific antigen binding molecule comprises the polypeptide sequence of SEQ ID NO: 1, the polypeptide sequence of SEQ ID NO: 2, the polypeptide sequence of SEQ ID NO: 3 and the polypeptide sequence of SEQ ID NO: 4, or variants thereof that retain functionality. In a further embodiment the bispecific antigen binding molecule comprises the polypeptide sequence of SEQ ID NO: 1, the polypeptide sequence of SEQ ID NO: 3, the polypeptide sequence of SEQ ID NO: 4 and the polypeptide sequence of SEQ ID NO: 5, or variants thereof that retain functionality. In yet another embodiment the bispecific antigen binding molecule comprises the polypeptide sequence of SEQ ID NO: 4, the polypeptide sequence of SEQ ID NO: 5, the polypeptide sequence of SEQ ID NO: 6 and the polypeptide sequence of SEQ ID NO: 7, or variants thereof that retain functionality. In still another embodiment the bispecific antigen binding molecule comprises the polypeptide sequence of SEQ ID NO: 4, the polypeptide sequence of SEQ ID NO: 5, the polypeptide sequence of SEQ ID NO: 1 and the polypeptide sequence of SEQ ID NO: 85, or variants thereof that retain functionality.

In a further embodiment the bispecific antigen binding molecule comprises the polypeptide sequence of SEQ ID NO: 1, the polypeptide sequence of SEQ ID NO: 3, the polypeptide sequence of SEQ ID NO: 4 and the polypeptide sequence of SEQ ID NO: 86, or variants thereof that retain functionality. In still a further embodiment the bispecific antigen binding molecule comprises the polypeptide sequence of SEQ ID NO: 4, the polypeptide sequence of SEQ ID NO: 87, the polypeptide sequence of SEQ ID NO: 89 and the polypeptide sequence of SEQ ID NO: 90, or variants thereof that retain functionality. In a further embodiment the bispecific antigen binding molecule comprises the polypeptide sequence of SEQ ID NO: 3, the polypeptide sequence of SEQ ID NO: 91, the polypeptide sequence of SEQ ID NO: 92 and the polypeptide sequence of SEQ ID NO: 93, or variants thereof that retain functionality. In still another embodiment the bispecific antigen binding molecule comprises the polypeptide sequence of SEQ ID NO: 87, the polypeptide sequence of SEQ ID NO: 91, the polypeptide sequence of SEQ ID NO: 93 and the polypeptide sequence of SEQ ID NO: 94, or variants thereof that retain functionality.

In one embodiment the second antigen is Carcinoembryonic Antigen (CEA). In another embodiment the second and optionally the third Fab fragment can compete with monoclonal antibody CH1A1A for binding to an epitope of CEA. See PCT publication WO 2011/023787. In one embodiment, the Fab fragment that is specific for CEA comprises the heavy chain CDR1 of SEQ ID NO: 39, the heavy chain CDR2 of SEQ ID NO: 40, the heavy chain CDR3 of SEQ ID NO: 41, the light chain CDR1 of SEQ ID NO: 43, the light chain CDR2 of SEQ ID NO: 44, and the light chain CDR3 of SEQ ID NO: 45. In a further embodiment, the Fab fragment that is specific for CEA comprises a heavy chain variable region sequence that is at least about 80%, 85%, 90%, 95%, 96%, 97%, 98%, 99% or 100% identical to SEQ ID NO: 42 and a light chain variable region sequence that is at least about 80%, 85%, 90%, 95%, 96%, 97%, 98%, 99% or 100% identical to SEQ ID NO: 46, or variants thereof that retain functionality.

In yet another embodiment the bispecific antigen binding molecule comprises the polypeptide sequence of SEQ ID NO: 3, the polypeptide sequence of SEQ ID NO: 8, the polypeptide sequence of SEQ ID NO: 9 and the polypeptide sequence of SEQ ID NO: 10, or variants thereof that retain functionality. In still another embodiment the bispecific antigen binding molecule comprises the polypeptide sequence of SEQ ID NO: 9, the polypeptide sequence of SEQ ID NO: 10, the polypeptide sequence of SEQ ID NO: 87 and the polypeptide sequence of SEQ ID NO: 95, or variants thereof that retain functionality.

In one embodiment the second antigen is Her3. In another embodiment the second and optionally the third Fab fragment can compete with monoclonal antibody Mab 205.10 for binding to an epitope of Her3. See PCT publication no. WO 2011/076683. In one embodiment, the Fab fragment that is specific for Her3 comprises the heavy chain CDR1 of SEQ ID NO: 55, the heavy chain CDR2 of SEQ ID NO: 56, the heavy chain CDR3 of SEQ ID NO: 57, the light chain CDR1 of SEQ ID NO: 59, the light chain CDR2 of SEQ ID NO: 60, and the light chain CDR3 of SEQ ID NO: 61. In a further embodiment, the Fab fragment that is specific for Her3 comprises a heavy chain variable region sequence that is at least about 80%, 85%, 90%, 95%, 96%, 97%, 98%, 99% or 100% identical to SEQ ID NO: 58 and a light chain variable region sequence that is at least about 80%, 85%, 90%, 95%, 96%, 97%, 98%, 99% or 100% identical to SEQ ID NO: 62, or variants thereof that retain functionality.

In yet another embodiment the bispecific antigen binding molecule comprises the polypeptide sequence of SEQ ID NO: 11, the polypeptide sequence of SEQ ID NO: 12, the polypeptide sequence of SEQ ID NO: 13 and the polypeptide sequence of SEQ ID NO: 14, or variants thereof that retain functionality.

In one embodiment the second antigen is epidermal growth factor receptor (EGFR). In another embodiment the second and optionally the third Fab fragment can compete with monoclonal antibody GA201 for binding to an epitope of EGFR. See PCT publication WO 2006/082515. In one embodiment, the Fab fragment that is specific for EGFR comprises the heavy chain CDR1 of SEQ ID NO: 23, the heavy chain CDR2 of SEQ ID NO: 24, the heavy chain CDR3 of SEQ ID NO: 25, the light chain CDR1 of SEQ ID NO: 27, the light chain CDR2 of SEQ ID NO: 28, and the light chain CDR3 of SEQ ID NO: 29. In a further embodiment, the Fab fragment that is specific for EGFR comprises a heavy chain variable region sequence that is at least about 80%, 85%, 90%, 95%, 96%, 97%, 98%, 99% or 100% identical to SEQ ID NO: 26 and a light chain variable region sequence that is at least about 80%, 85%, 90%, 95%, 96%, 97%, 98%, 99% or 100% identical to SEQ ID NO: 30, or variants thereof that retain functionality.

In one embodiment the second antigen is fibroblast activation protein (FAP). In another embodiment the second and optionally the third Fab fragment can compete with monoclonal antibody 3F2 for binding to an epitope of FAP. See PCT publication WO 2012/020006. In one embodiment, the Fab fragment that is specific for FAP comprises the heavy chain CDR1 of SEQ ID NO: 31, the heavy chain CDR2 of SEQ ID NO: 32, the heavy chain CDR3 of SEQ ID NO: 33, the light chain CDR1 of SEQ ID NO: 35, the light chain CDR2 of SEQ ID NO: 36, and the light chain CDR3 of SEQ ID NO: 37. In a further embodiment, the Fab fragment that is specific for FAP comprises a heavy chain variable region sequence that is at least about 80%, 85%, 90%, 95%, 96%, 97%, 98%, 99% or 100% identical to SEQ ID NO: 34 and a light chain variable region sequence that is at least about 80%, 85%, 90%, 95%, 96%, 97%, 98%, 99% or 100% identical to SEQ ID NO: 38, or variants thereof that retain functionality.

In one embodiment the second antigen is CD33. In one embodiment, the Fab fragment that is specific for CD33 comprises the heavy chain CDR1 of SEQ ID NO: 47, the heavy chain CDR2 of SEQ ID NO: 48, the heavy chain CDR3 of SEQ ID NO: 49, the light chain CDR1 of SEQ ID NO: 51, the light chain CDR2 of SEQ ID NO: 52, and the light chain CDR3 of SEQ ID NO: 53.

In a further embodiment, the Fab fragment that is specific for CD33 comprises a heavy chain variable region sequence that is at least about 80%, 85%, 90%, 95%, 96%, 97%, 98%, 99% or 100% identical to SEQ ID NO: 50 and a light chain variable region sequence that is at least about 80%, 85%, 90%, 95%, 96%, 97%, 98%, 99% or 100% identical to SEQ ID NO: 54, or variants thereof that retain functionality.

### Polynucleotides

Further provided herein are isolated polynucleotides encoding a bispecific antigen binding molecule as described herein or a fragment thereof. The polynucleotides encoding bispecific antigen binding molecules described herein may be expressed as a single polynucleotide that encodes the entire bispecific antigen binding molecule or as multiple (e.g., two or more) polynucleotides that are co-expressed. Polypeptides encoded by polynucleotides that are co-expressed may associate through, e.g., disulfide bonds or other means to form a functional bispecific antigen binding molecule. For example, the light chain portion of a Fab fragment may be encoded by a separate polynucleotide from the portion of the bispecific antigen binding molecule comprising the heavy chain portion of the Fab fragment, an Fc domain subunit and optionally (part of) another Fab fragment. When co-expressed, the heavy chain polypeptides will associate with the light chain polypeptides to form the Fab fragment. In another example, the portion of the bispecific antigen binding molecule comprising one of the two Fc domain subunits and optionally (part of) one or more Fab fragments could be encoded by a separate polynucleotide from the portion of the bispecific antigen binding molecule comprising the the other of the two Fc domain subunits and optionally (part of) a Fab fragment. When co-expressed, the Fc domain subunits will associate to form the Fc domain.

In one embodiment, an isolated polynucleotide disclosed herein encodes the first Fc domain subunit, the heavy chain of the second Fab fragment and the heavy chain of the first Fab fragment. In a more specific embodiment, the isolated polynucleotide encodes a polypeptide wherein a VL region shares a carboxy-terminal peptide bond with a CH1 region, which in turn shares a carboxy-terminal peptide bond with a peptide linker, which in turn shares a carboxy-terminal peptide bond with an immunoglobulin heavy chain (VH-CH1-HR-CH2-CH3-(CH4)). In another specific embodiment the isolated polynucleotide encodes a polypeptide wherein a Fab heavy chain (VH-CH1) shares a carboxy-terminal peptide bond with a peptide linker, which in turn shares a carboxy-terminal peptide bond with a VL region, which in turn shares a carboxy-terminal peptide bond with a CH1 region, which in turn shares a carboxy-terminal peptide bond with an Fc domain subunit including an immunoglobulin hinge region (HR-CH2-CH3-(CH4)).

In yet another specific embodiment, the isolated polynucleotide encodes a polypeptide wherein a VH region shares a carboxy-terminal peptide bond with a CL region, which in turn shares a carboxy-terminal peptide bond with a peptide linker, which in turn shares a carboxy-terminal peptide bond with an immunoglobulin heavy chain (VH-CH1-HR-CH2-CH3-(CH4)). In still another specific embodiment the isolated polynucleotide encodes a polypeptide wherein a Fab heavy chain (VH-CH1) shares a carboxy-terminal peptide bond with a peptide linker, which in turn shares a carboxy-terminal peptide bond with a VH region, which in turn shares a carboxy-terminal peptide bond with a CL region, which in turn shares a carboxy-terminal peptide bond with an Fc domain subunit including an immunoglobulin hinge region (HR-CH2-CH3-(CH4)).

In yet another specific embodiment, the isolated polynucleotide encodes a polypeptide wherein an immunoglobulin heavy chain ((VH-CH1-HR-CH2-CH3-(CH4)) shares a carboxy-terminal peptide bond with a peptide linker, which in turn shares a carboxy-terminal peptide bond with a VH region, which in turn shares a carboxy-terminal peptide bond with a CL region.

In further embodiments, an isolated polynucleotide disclosed herein encodes the second Fc domain subunit and optionally the heavy chain of a third Fab fragment. In a specific embodiment, the isolated polynucleotide encodes an immunoglobulin heavy chain ((VH-CH1-HR-CH2-CH3-(CH4)). In another specific embodiment, the isolated polynucleotide encodes an Fc domain subunit, optionally including an antibody hinge region ((HR)-CH2-CH3-(CH4)).

In still further embodiments, an isolated polynucleotide disclosed herein encodes one or more light chain comprised in the bispecific antigen binding molecule. In a specific embodiment, the isolated polynucleotide encodes an immunoglobulin light chain (VL-CL). In another specific embodiment, the isolated polynucleotide encodes a polypeptide wherein a VL region shares a carboxy-terminal peptide bond with a CH1 region. In yet another specific embodiment, the isolated polynucleotide encodes a polypeptide wherein a VH region shares a carboxy-terminal peptide bond with a CL region. In still another specific embodiment, the isolated polynucleotide encodes a polypeptide wherein a VH region shares a carboxy-terminal peptide bond with a CL region, which in turn shares a carboxy-terminal peptide bond with a Fab light chain (VL-CL). In yet another specific embodiment, the isolated polynucleotide encodes a polypeptide wherein a Fab light chain (VL-CL) shares a carboxy-terminal peptide bond with a VH region, which in turn shares a carboxy-terminal peptide bond with a CL region.

In another embodiment, the present disclosure is directed to an isolated polynucleotide encoding a bispecific antigen binding molecule disclosed herein or a fragment thereof, wherein the polynucleotide comprises a sequence that encodes a variable region sequence as shown in SEQ ID NOs 18, 22, 26, 30, 34, 38, 42, 46, 50, 54, 58, 62, 66, 70, 80, 84, 99, 103, 107 and 111. In another embodiment, the present disclosure is directed to an isolated polynucleotide encoding a bispecific antigen binding molecule or fragment thereof, wherein the polynucleotide comprises a sequence that encodes a polypeptide sequence as shown in SEQ ID NOs 1, 2, 3, 4, 5, 6, 7, 8, 9, 10, 11, 12, 13, 14, 85, 86, 87, 88, 89, 90, 91, 92, 93, 94 and 95. In another embodiment, the disclosure is directed to an isolated polynucleotide encoding a bispecific antigen binding molecule disclosed herein or a fragment thereof, wherein the polynucleotide comprises a sequence that encodes a variable region sequence that is at least about 80%, 85%, 90%, 95%, 96%, 97%, 98%, or 99% identical to an amino acid sequence in SEQ ID NOs 18, 22, 26, 30, 34, 38, 42, 46, 50, 54, 58, 62, 66, 70, 80, 84, 99, 103, 107 or 111. In another embodiment, the present disclosure is directed to an isolated polynucleotide encoding a bispecific antigen binding molecule or fragment thereof, wherein the polynucleotide comprises a sequence that encodes a polypeptide sequence that is at least 80%, 85%, 90%, 95%, 96%, 97%, 98%, or 99% identical to an amino acid sequence in SEQ ID NOs 1, 2, 3, 4, 5, 6, 7, 8, 9, 10, 11, 12, 13, 14, 85, 86, 87, 88, 89, 90, 91, 92, 93, 94 or 95. The present disclosure encompasses an isolated polynucleotide encoding a bispecific antigen binding molecule disclosed herein or a fragment thereof, wherein the polynucleotide comprises a sequence that encodes the variable region sequence of SEQ ID NOs 18, 22, 26, 30, 34, 38, 42, 46, 50, 54, 58, 62, 66, 70, 80, 84, 99, 103, 107 or 111 with conservative amino acid substitutions. The disclosure also encompasses an isolated polynucleotide encoding a bispecific antigen binding molecule or fragment thereof, wherein the polynucleotide comprises a sequence that encodes the polypeptide sequence of SEQ ID NOs 1, 2, 3, 4, 5, 6, 7, 8, 9, 10, 11, 12, 13, 14, 85, 86, 87, 88, 89, 90, 91, 92, 93, 94 or 95 with conservative amino acid substitutions.

In certain embodiments the polynucleotide or nucleic acid is DNA. In other embodiments, a polynucleotide disclosed herein is RNA, for example, in the form of messenger RNA (mRNA). RNA disclosed herein may be single stranded or double stranded.

### Recombinant Methods

Bispecific antigen binding molecules disclosed herein may be obtained, for example, by solid-state peptide synthesis (e.g. Merrifield solid phase synthesis) or recombinant production. For recombinant production one or more polynucleotide encoding the bispecific antigen binding molecule (fragment), e.g., as described above, is isolated and inserted into one or more vectors for further cloning and/or expression in a host cell. Such polynucleotide may be readily isolated and sequenced using conventional procedures. In one embodiment a vector, preferably an expression vector, comprising one or more of the polynucleotides disclosed herein is provided.

Methods which are well known to those skilled in the art can be used to construct expression vectors containing the coding sequence of a bispecific antigen binding molecule (fragment) along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the bispecific antigen binding molecule (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region.

Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector disclosed herein may encode one or more polypeptides, which are post- or co-translationally separated into the final proteins via proteolytic cleavage. In addition, a vector, polynucleotide, or nucleic acid disclosed herein may encode heterologous coding regions, either fused or unfused to a polynucleotide encoding the bispecific antigen binding molecule (fragment) disclosed herein, or variant or derivative thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions disclosed herein may be associated with additional coding regions which encode secretory or signal peptides, which direct the secretion of a polypeptide encoded by a polynucleotide disclosed herein. For example, if secretion of the bispecific antigen binding molecule is desired, DNA encoding a signal sequence may be placed upstream of the nucleic acid encoding a bispecific antigen binding molecule disclosed herein or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated.

Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, *e.g.* an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase. Exemplary amino acid sequences of secretory signal peptides are given in SEQ ID NOs 74-76.

DNA encoding a short protein sequence that could be used to facilitate later purification (e.g. a histidine tag) or assist in labeling the bispecific antigen binding molecule may be included within or at the ends of the bispecific antigen binding molecule (fragment) encoding polynucleotide.

In a further embodiment, a host cell comprising one or more polynucleotides as disclosed herein is provided. In certain embodiments a host cell comprising one or more vectors as disclosed herein is provided. The polynucleotides and vectors may incorporate any of the features, singly or in combination, described herein in relation to polynucleotides and vectors, respectively. In one such embodiment a host cell comprises (e.g. has been transformed or transfected with) a vector comprising a polynucleotide that encodes (part of) a bispecific antigen binding molecule disclosed herein. As used herein, the term "host cell" refers to any kind of cellular system which can be engineered to generate the bispecific antigen binding molecules disclosed herein or fragments thereof. Host cells suitable for replicating and for supporting expression of bispecific antigen binding molecules are well known in the art. Such cells may be transfected or transduced as appropriate with the particular expression vector and large quantities of vector containing cells can be grown for seeding large scale fermenters to obtain sufficient quantities of the bispecific antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr⁻ CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell).

Standard technologies are known in the art to express foreign genes in these systems. Cells expressing a polypeptide comprising either the heavy or the light chain of an antigen binding domain such as an antibody, may be engineered so as to also express the other of the antibody chains such that the expressed product is an antibody that has both a heavy and a light chain.

In one embodiment, a method of producing a bispecific antigen binding molecule as disclosed herein is provided, wherein the method comprises culturing a host cell comprising a polynucleotide encoding the bispecific antigen binding molecule, as provided herein, under conditions suitable for expression of the bispecific antigen binding molecule, and recovering the bispecific antigen binding molecule from the host cell (or host cell culture medium).

The components of the bispecific antigen binding molecule are genetically fused to each other. Bispecific antigen binding molecule can be designed such that its components are fused directly to each other or indirectly through a linker sequence. The composition and length of the linker may be determined in accordance with methods well known in the art and may be tested for efficacy. Examples of linker sequences between different components of bispecific antigen binding molecules are found in the sequences provided herein. Additional sequences may also be included to incorporate a cleavage site to separate the individual components of the fusion if desired, for example an endopeptidase recognition sequence.

In certain embodiments the Fab fragments forming part of the bispecific antigen binding molecules comprise at least an antibody variable region capable of binding an antigenic determinant. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of antibody, antibody fragment, antigen binding domain or variable region can be used in the bispecific antigen binding molecules disclosed herein. Non-limiting antibodies, antibody fragments, antigen binding domains or variable regions useful herein can be of murine, primate, or human origin. If the bispecific antigen binding molecule is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain embodiments, the Fab fragments useful herein are engineered to have enhanced binding affinity according to, for example, the methods disclosed in U.S. Pat. Appl. Publ. No. 2004/0132066. The ability of the bispecific antigen binding molecule disclosed herein to bind to a specific antigenic determinant can be measured either through an enzyme-linked immunosorbent assay (ELISA) or other techniques familiar to one of skill in the art, e.g. surface plasmon resonance technique (analyzed on a BIACORE T100 system) (Liljeblad, et al., Glyco J 17, 323-329 (2000)), and traditional binding assays (Heeley, Endocr Res 28, 217-229 (2002)).

Competition assays may be used to identify an antibody, antibody fragment, antigen binding domain or variable domain that competes with a reference antibody for binding to a particular antigen. In certain embodiments, such a competing antibody binds to the same epitope (e.g. a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen is incubated in a solution comprising a first labeled antibody that binds to the antigen and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

Bispecific antigen binding molecules prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art.

For affinity chromatography purification an antibody, ligand, receptor or antigen can be used to which the bispecific antigen binding molecule binds. For example, for affinity chromatography purification of bispecific antigen binding molecules disclosed herein, a matrix with protein A or protein G may be used. Sequential Protein A or G affinity chromatography and size exclusion chromatography can be used to isolate a bispecific antigen binding molecule essentially as described in the Examples. The purity of the bispecific antigen binding molecule can be determined by any of a variety of well known analytical methods including gel electrophoresis, high pressure liquid chromatography, and the like. For example, the heavy chain fusion proteins expressed as described in the Examples were shown to be intact and properly assembled as demonstrated by reducing SDS-PAGE (see e.g. Figure 3). Three bands were resolved at approximately Mr 25,000, Mr 50,000 and Mr 75,000, corresponding to the predicted molecular weights of the bispecific antigen binding molecule light chains, heavy chain and heavy chain/Fab heavy chain fusion protein, respectively.

### Assays

Bispecific antigen binding molecules provided herein may be identified, screened for, or characterized for their physical/chemical properties and/or biological activities by various assays known in the art.

### Affinity assays

The affinity of the bispecific antigen binding molecule for an Fc receptor or a target antigen can be determined in accordance with the methods set forth in the Examples by surface plasmon resonance (SPR), using standard instrumentation such as a BIAcore instrument (GE Healthcare), and receptors or target proteins such as may be obtained by recombinant expression.

Alternatively, binding of bispecific antigen binding molecules for different receptors or target antigens may be evaluated using cell lines expressing the particular receptor or target antigen, for example by flow cytometry (FACS). A specific illustrative and exemplary embodiment for measuring binding affinity is described in the following and in the Examples below.

According to one embodiment, K_{D} is measured by surface plasmon resonance using a BIACORE® T100 machine (GE Healthcare) at 25 °C.

To analyze the interaction between the Fc-portion and Fc receptors, His-tagged recombinant Fc-receptor is captured by an anti-Penta His antibody (Qiagen) immobilized on CM5 chips and the bispecific constructs are used as analytes. Briefly, carboxymethylated dextran biosensor chips (CM5, GE Healthcare) are activated with N-ethyl-N'-(3-dimethylaminopropyl)-carbodiimide hydrochloride (EDC) and N-hydroxysuccinimide (NHS) according to the supplier's instructions.

Anti Penta-His antibody is diluted with 10 mM sodium acetate, pH 5.0, to 40 µg/ml before injection at a flow rate of 5 µl/min to achieve approximately 6500 response units (RU) of coupled protein. Following the injection of the ligand, 1 M ethanolamine is injected to block unreacted groups. Subsequently the Fc-receptor is captured for 60 s at 4 or 10 nM. For kinetic measurements, four-fold serial dilutions of the bispecific construct (range between 500 nM and 4000 nM) are injected in HBS-EP (GE Healthcare, 10 mM HEPES, 150 mM NaCl, 3 mM EDTA, 0.05 % Surfactant P20, pH 7.4) at 25 °C at a flow rate of 30 µl/min for 120 s.

To determine the affinity to the target antigen, bispecific constructs are captured by an anti human Fab specific antibody (GE Healthcare) that is immobilized on an activated CM5-sensor chip surface as described for the anti Penta-His antibody. The final amount of coupled protein is is approximately 12000 RU. The bispecific constructs are captured for 90 s at 300 nM. The target antigens are passed through the flow cells for 180 s at a concentration range from 250 to 1000 nM with a flowrate of 30 µl/min. The dissociation is monitored for 180 s.

Bulk refractive index differences are corrected for by subtracting the response obtained on reference flow cell. The steady state response was used to derive the dissociation constant K_{D} by non-linear curve fitting of the Langmuir binding isotherm. Association rates (kₒₙ) and dissociation rates (k_{off}) are calculated using a simple one-to-one Langmuir binding model (BIACORE® T100 Evaluation Software version 1.1.1) by simultaneously fitting the association and dissociation sensorgrams. The equilibrium dissociation constant (K_{D}) is calculated as the ratio k_{off}/kₒₙ. See, e.g., Chen et al., J Mol Biol 293, 865-881 (1999).

### Activity assays

Biological activity of the bispecific antigen binding molecules disclosed herein can be measured by various assays known in the art, including those described in the Examples. Biological activities may for example include the induction of proliferation of T cells, the induction of signaling in T cells, the induction of expression of activation markers in T cells, the induction of cytokine secretion by T cells, the inhibition of signaling in target cells such as tumor cells or cells of the tumor stroma, the inhibition of proliferation of target cells, the induction of lysis of target cells, and the induction of tumor regression and/or the improvement of survival.

### Compositions, Formulations, and Routes of Administration

In a further aspect, provided herein are pharmaceutical compositions comprising any of the bispecific antigen binding molecules provided herein, e.g., for use in any of the below therapeutic methods. In one embodiment, a pharmaceutical composition comprises any of the bispecific antigen binding molecules provided herein and a pharmaceutically acceptable carrier.

In another embodiment, a pharmaceutical composition comprises any of the bispecific antigen binding molecules provided herein and at least one additional therapeutic agent, e.g., as described below.

Further provided is a method of producing a bispecific antigen binding molecule as disclosed herein in a form suitable for administration in vivo, the method comprising (a) obtaining a bispecific antigen binding molecule as disclosed herein, and (b) formulating the bispecific antigen binding molecule with at least one pharmaceutically acceptable carrier, whereby a preparation of bispecific antigen binding molecule is formulated for administration in vivo.

Pharmaceutical compositions disclosed herein comprise a therapeutically effective amount of one or more bispecific antigen binding molecule dissolved or dispersed in a pharmaceutically acceptable carrier. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one bispecific antigen binding molecule and optionally an additional active ingredient will be known to those of skill in the art in light of the present disclosure, as exemplified by Remington's Pharmaceutical Sciences, 18th Ed. Mack Printing Company, 1990. Moreover, for animal (e.g., human) administration, it will be understood that preparations should meet sterility, pyrogenicity, general safety and purity standards as required by FDA Office of Biological Standards or corresponding authorities in other countries. Preferred compositions are lyophilized formulations or aqueous solutions. As used herein, "pharmaceutically acceptable carrier" includes any and all solvents, buffers, dispersion media, coatings, surfactants, antioxidants, preservatives (*e.g*. antibacterial agents, antifungal agents), isotonic agents, absorption delaying agents, salts, preservatives, antioxidants, proteins, drugs, drug stabilizers, polymers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

The composition may comprise different types of carriers depending on whether it is to be administered in solid, liquid or aerosol form, and whether it need to be sterile for such routes of administration as injection. Bispecific antigen binding molecules disclosed herein (and any additional therapeutic agent) can be administered intravenously, intradermally, intraarterially, intraperitoneally, intralesionally, intracranially, intraarticularly, intraprostatically, intrasplenically, intrarenally, intrapleurally, intratracheally, intranasally, intravitreally, intravaginally, intrarectally, intratumorally, intramuscularly, intraperitoneally, subcutaneously, subconjunctivally, intravesicularlly, mucosally, intrapericardially, intraumbilically, intraocularally, orally, topically, locally, by inhalation (e.g. aerosol inhalation), injection, infusion, continuous infusion, localized perfusion bathing target cells directly, via a catheter, via a lavage, in cremes, in lipid compositions (e.g. liposomes), or by other method or any combination of the forgoing as would be known to one of ordinary skill in the art (see, for example, Remington's Pharmaceutical Sciences, 18th Ed. Mack Printing Company, 1990). Parenteral administration, in particular intravenous injection, is most commonly used for administering polypeptide molecules such as the bispecific antigen binding molecules disclosed herein.

Parenteral compositions include those designed for administration by injection, e.g. subcutaneous, intradermal, intralesional, intravenous, intraarterial intramuscular, intrathecal or intraperitoneal injection. For injection, the bispecific antigen binding molecules disclosed herein may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the bispecific antigen binding molecules may be in powder form for constitution with a suitable vehicle, e.g., sterile pyrogen-free water, before use. Sterile injectable solutions are prepared by incorporating the bispecific antigen binding molecules disclosed herein in the required amount in the appropriate solvent with various of the other ingredients enumerated below, as required. Sterility may be readily accomplished, e.g., by filtration through sterile filtration membranes.

Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable carriers include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

In addition to the compositions described previously, the bispecific antigen binding molecules may also be formulated as a depot preparation. Such long acting formulations may be administered by implantation (for example subcutaneously or intramuscularly) or by intramuscular injection. Thus, for example, the bispecific antigen binding molecules may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the bispecific antigen binding molecules disclosed herein may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The bispecific antigen binding molecules may be formulated into a composition in a free acid or base, neutral or salt form. Pharmaceutically acceptable salts are salts that substantially retain the biological activity of the free acid or base. These include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

### Therapeutic Methods and Compositions

Any of the bispecific antigen binding molecules provided herein may be used in therapeutic methods. Bispecific antigen binding molecules as disclosed herein can be used for example in the treatment of cancers.

For use in therapeutic methods, bispecific antigen binding molecules disclosed herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, bispecific antigen binding molecules as disclosed herein for use as a medicament are provided. In further aspects, bispecific antigen binding molecules as disclosed herein for use in treating a disease are provided. In certain embodiments, bispecific antigen binding molecules as disclosed herein for use in a method of treatment are provided. In one embodiment, a bispecific antigen binding molecule as described herein is provided for use in the treatment of a disease in an individual in need thereof. In certain embodiments, a bispecific antigen binding molecule is provided for use in a method of treating an individual having a disease comprising administering to the individual a therapeutically effective amount of the bispecific antigen binding molecule. In certain embodiments the disease to be treated is a proliferative disorder. In a particular embodiment the disease is cancer. In certain embodiments the method further comprises administering to the individual a therapeutically effective amount of at least one additional therapeutic agent, e.g., an anti-cancer agent if the disease to be treated is cancer. An "individual" according to any of the above embodiments is a mammal, preferably a human.

In a further aspect, provided herein is the use of a bispecific antigen binding molecule as disclosed herein in the manufacture or preparation of a medicament. In one embodiment the medicament is for the treatment of a disease in an individual in need thereof. In a further embodiment, the medicament is for use in a method of treating a disease comprising administering to an individual having the disease a therapeutically effective amount of the medicament. In certain embodiments the disease to be treated is a proliferative disorder. In a particular embodiment the disease is cancer. In one embodiment, the method further comprises administering to the individual a therapeutically effective amount of at least one additional therapeutic agent, e.g., an anti-cancer agent if the disease to be treated is cancer. An "individual" according to any of the above embodiments may be a mammal, preferably a human.

In a further aspect, provided herein is a method for treating a disease. In one embodiment, the method comprises administering to an individual having such disease a therapeutically effective amount of a bispecific antigen binding molecule as disclosed herein. In one embodiment a composition is administered to said individual, comprising the bispecific antigen binding molecule disclosed herein in a pharmaceutically acceptable form. In certain embodiments the disease to be treated is a proliferative disorder. In a particular embodiment the disease is cancer.

In certain embodiments the method further comprises administering to the individual a therapeutically effective amount of at least one additional therapeutic agent, e.g., an anti-cancer agent if the disease to be treated is cancer. An "individual" according to any of the above embodiments may be a mammal, preferably a human.

In certain embodiments the disease to be treated is a proliferative disorder, particularly cancer.

Non-limiting examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that can be treated using a bispecific antigen binding molecule disclosed herein include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases.

In certain embodiments the cancer is chosen from the group consisting of renal cell cancer, skin cancer, lung cancer, colorectal cancer, breast cancer, brain cancer, head and neck cancer. A skilled artisan readily recognizes that in many cases the bispecific antigen binding molecule may not provide a cure but may only provide partial benefit. In some embodiments, a physiological change having some benefit is also considered therapeutically beneficial. Thus, in some embodiments, an amount of bispecific antigen binding molecule that provides a physiological change is considered an "effective amount" or a "therapeutically effective amount". The subject, patient, or individual in need of treatment is typically a mammal, more specifically a human.

In some embodiments, an effective amount of a bispecific antigen binding molecule disclosed herein is administered to a cell. In other embodiments, a therapeutically effective amount of a bispecific antigen binding molecule disclosed herein is administered to an individual for the treatment of disease.

For the prevention or treatment of disease, the appropriate dosage of a bispecific antigen binding molecule disclosed herein (when used alone or in combination with one or more other additional therapeutic agents) will depend on the type of disease to be treated, the route of administration, the body weight of the patient, the type of bispecific antigen binding molecule, the severity and course of the disease, whether the bispecific antigen binding molecule is administered for preventive or therapeutic purposes, previous or concurrent therapeutic interventions, the patient's clinical history and response to the bispecific antigen binding molecule, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The bispecific antigen binding molecule is suitably administered to the patient at one time or over a series of treatments. Depending on the type and severity of the disease, about 1 µg/kg to 15 mg/kg (e.g. 0.1 mg/kg - 10 mg/kg) of bispecific antigen binding molecule can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the bispecific antigen binding molecule would be in the range from about 0.005 mg/kg to about 10 mg/kg. In other non-limiting examples, a dose may also comprise from about 1 microgram/kg body weight, about 5 microgram/kg body weight, about 10 microgram/kg body weight, about 50 microgram/kg body weight, about 100 microgram/kg body weight, about 200 microgram/kg body weight, about 350 microgram/kg body weight, about 500 microgram/kg body weight, about 1 milligram/kg body weight, about 5 milligram/kg body weight, about 10 milligram/kg body weight, about 50 milligram/kg body weight, about 100 milligram/kg body weight, about 200 milligram/kg body weight, about 350 milligram/kg body weight, about 500 milligram/kg body weight, to about 1000 mg/kg body weight or more per administration, and any range derivable therein. In non-limiting examples of a derivable range from the numbers listed herein, a range of about 5 mg/kg body weight to about 100 mg/kg body weight, about 5 microgram/kg body weight to about 500 milligram/kg body weight, etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the bispecific antigen binding molecule). An initial higher loading dose, followed by one or more lower doses may be administered. However, other dosage regimens may be useful. The progress of this therapy is easily monitored by conventional techniques and assays.

The bispecific antigen binding molecules disclosed herein will generally be used in an amount effective to achieve the intended purpose. For use to treat or prevent a disease condition, the bispecific antigen binding molecules disclosed herein, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from *in vitro* assays, such as cell culture assays. A dose can then be formulated in animal models to achieve a circulating concentration range that includes the IC₅₀ as determined in cell culture. Such information can be used to more accurately determine useful doses in humans.

Initial dosages can also be estimated from *in vivo* data, *e.g*., animal models, using techniques that are well known in the art. One having ordinary skill in the art could readily optimize administration to humans based on animal data.

Dosage amount and interval may be adjusted individually to provide plasma levels of the bispecific antigen binding molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the bispecific antigen binding molecules may not be related to plasma concentration. One having skill in the art will be able to optimize therapeutically effective local dosages without undue experimentation.

A therapeutically effective dose of the bispecific antigen binding molecules described herein will generally provide therapeutic benefit without causing substantial toxicity. Toxicity and therapeutic efficacy of a bispecific antigen binding molecule can be determined by standard pharmaceutical procedures in cell culture or experimental animals. Cell culture assays and animal studies can be used to determine the LD₅₀ (the dose lethal to 50% of a population) and the ED₅₀ (the dose therapeutically effective in 50% of a population). The dose ratio between toxic and therapeutic effects is the therapeutic index, which can be expressed as the ratio LD₅₀/ED₅₀.

Bispecific antigen binding molecules that exhibit large therapeutic indices are preferred. In one embodiment, the bispecific antigen binding molecule disclosed herein exhibits a high therapeutic index. The data obtained from cell culture assays and animal studies can be used in formulating a range of dosages suitable for use in humans. The dosage lies preferably within a range of circulating concentrations that include the ED₅₀ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1).

The attending physician for patients treated with bispecific antigen binding molecules disclosed herein would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

### Other Agents and Treatments

The bispecific antigen binding molecules disclosed herein may be administered in combination with one or more other agents in therapy. For instance, a bispecific antigen binding molecule disclosed herein may be co-administered with at least one additional therapeutic agent. The term "therapeutic agent" encompasses any agent administered to treat a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is an immunomodulatory agent, a cytostatic agent, an inhibitor of cell adhesion, a cytotoxic agent, an activator of cell apoptosis, or an agent that increases the sensitivity of cells to apoptotic inducers. In a particular embodiment, the additional therapeutic agent is an anti-cancer agent, for example a microtubule disruptor, an antimetabolite, a topoisomerase inhibitor, a DNA intercalator, an alkylating agent, a hormonal therapy, a kinase inhibitor, a receptor antagonist, an activator of tumor cell apoptosis, or an antiangiogenic agent.

Such other agents are suitably present in combination in amounts that are effective for the purpose intended. The effective amount of such other agents depends on the amount of bispecific antigen binding molecule used, the type of disorder or treatment, and other factors discussed above. The bispecific antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the bispecific antigen binding molecule disclosed herein can occur prior to, simultaneously, and/or following, administration of the additional therapeutic agent and/or adjuvant. Bispecific antigen binding molecules disclosed herein can also be used in combination with radiation therapy.

### Articles of Manufacture

In another aspect, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc.

The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a bispecific antigen binding molecule as disclosed herein. The label or package insert indicates that the composition is used for treating the condition of choice. Moreover, the article of manufacture may comprise (a) a first container with a composition contained therein, wherein the composition comprises a bispecific antigen binding molecule as disclosed herein; and (b) a second container with a composition contained therein, wherein the composition comprises a further cytotoxic or otherwise therapeutic agent. The article of manufacture in this embodiment may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Examples

The following are examples of methods and compositions disclosed herein. It is understood that various other embodiments may be practiced, given the general description provided above.

### General methods

### Recombinant DNA Techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturers' instructions. General information regarding the nucleotide sequences of human immunoglobulins light and heavy chains is given in: Kabat, E.A. et al., (1991) Sequences of Proteins of Immunological Interest, 5th ed., NIH Publication No. 91-3242.

### DNA Sequencing

DNA sequences were determined by double strand sequencing.

### Gene Synthesis

Desired gene segments where required were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells. SEQ ID NOs 74-76 give exemplary leader peptides.

### Isolation of primary human pan T cells from PBMCs

Peripheral blood mononuclear cells (PBMCs) were prepared by Histopaque density centrifugation from enriched lymphocyte preparations (buffy coats) obtained from local blood banks or from fresh blood from healthy human donors. Briefly, blood was diluted with sterile PBS and carefully layered over a Histopaque gradient (Sigma, H8889). After centrifugation for 30 minutes at 450 x g at room temperature (brake switched off), part of the plasma above the PBMC containing interphase was discarded. The PBMCs were transferred into new 50 ml Falcon tubes and tubes were filled up with PBS to a total volume of 50 ml. The mixture was centrifuged at room temperature for 10 minutes at 400 x g (brake switched on). The supernatant was discarded and the PBMC pellet washed twice with sterile PBS (centrifugation steps at 4°C for 10 minutes at 350 x g). The resulting PBMC population was counted automatically (ViCell) and stored in RPMI1640 medium, containing 10% FCS and 1% L-alanyl-L-glutamine (Biochrom, K0302) at 37°C, 5% CO₂ in the incubator until assay start.

T cell enrichment from PBMCs was performed using the Pan T Cell Isolation Kit II (Miltenyi Biotec #130-091-156), according to the manufacturer's instructions. Briefly, the cell pellets were diluted in 40 µl cold buffer per 10 million cells (PBS with 0.5% BSA, 2 mM EDTA, sterile filtered) and incubated with 10 µl Biotin-Antibody Cocktail per 10 million cells for 10 min at 4°C. 30 µl cold buffer and 20 µl Anti-Biotin magnetic beads per 10 million cells were added, and the mixture incubated for another 15 min at 4°C. Cells were washed by adding 10-20x the current volume and a subsequent centrifugation step at 300 x g for 10 min. Up to 100 million cells were resuspended in 500 µl buffer. Magnetic separation of unlabeled human pan T cells was performed using LS columns (Miltenyi Biotec #130-042-401) according to the manufacturer's instructions. The resulting T cell population was counted automatically (ViCell) and stored in AIM-V medium at 37°C, 5% CO₂ in the incubator until assay start (not longer than 24 h).

### Isolation of primary human naive T cells from PBMCs

Peripheral blood mononuclar cells (PBMCs) were prepared by Histopaque density centrifugation from enriched lymphocyte preparations (buffy coats) obtained from local blood banks or from fresh blood from healthy human donors. T-cell enrichment from PBMCs was performed using the Naive CD8⁺ T cell isolation Kit from Miltenyi Biotec (#130-093-244), according to the manufacturer's instructions, but skipping the last isolation step of CD8⁺ T cells (also see description for the isolation of primary human pan T cells).

### Isolation of primary cynomolgus PBMCs from heparinized blood

Peripheral blood mononuclar cells (PBMCs) were prepared by density centrifugation from fresh blood from healthy cynomolgus donors, as follows: Heparinized blood was diluted 1:3 with sterile PBS, and Lymphoprep medium (Axon Lab #1114545) was diluted to 90% with sterile PBS. Two volumes of the diluted blood were layered over one volume of the diluted density gradient and the PBMC fraction was separated by centrifugation for 30 min at 520 x g, without brake, at room temperature. The PBMC band was transferred into a fresh 50 ml Falcon tube and washed with sterile PBS by centrifugation for 10 min at 400 x g at 4°C. One low-speed centrifugation was performed to remove the platelets (15 min at 150 x g, 4°C), and the resulting PBMC population was automatically counted (ViCell) and immediately used for further assays.

### Target cells

For the assessment of MCSP-targeting bispecific antigen binding molecules, the following tumor cell lines were used: the human melanoma cell line WM266-4 (ATCC #CRL-1676), derived from a metastatic site of a malignant melanoma and expressing high levels of human MCSP; and the human melanoma cell line MV-3 (a kind gift from The Radboud University Nijmegen Medical Centre), expressing medium levels of human MCSP.

For the assessment of CEA-targeting bispecific antigen binding molecules, the following tumor cell lines were used: the human gastric cancer cell line MKN45 (DSMZ #ACC 409), expressing very high levels of human CEA; the human female Caucasian colon adenocarcinoma cell line LS-174T (ECACC #87060401), expressing medium to low levels of human CEA; the human epithelioid pancreatic carcinoma cell line Panc-1 (ATCC #CRL-1469), expressing (very) low levels of human CEA; and a murine colon carcinoma cell line MC38-huCEA, that was engineered in-house to stably express human CEA.

In addition, a human T cell leukaemia cell line, Jurkat (ATCC #TIB-152), was used to assess binding of different bispecific constructs to human CD3 on cells.

### Example 1

### Preparation, purification and characterization of bispecific antigen binding molecules

The heavy and light chain variable region DNA sequences were subcloned in frame with either the constant heavy chain or the constant light chain pre-inserted into the respective recipient mammalian expression vector. The antibody expression was driven by an MPSV promoter and a synthetic polyA signal sequence is located at the 3' end of the CDS. In addition each vector contained an EBV OriP sequence.

The molecules were produced by co-transfecting HEK293 EBNA cells with the mammalian expression vectors. Exponentially growing HEK293 EBNA cells were transfected using the calcium phosphate method. Alternatively, HEK293 EBNA cells growing in suspension were transfected using polyethylenimine (PEI). For preparation of "1+1 IgG Crossfab" constructs, cells were transfected with the corresponding expression vectors in a 1:1:1:1 ratio ("vector second heavy chain" : "vector first light chain" : "vector light chain Crossfab" : "vector first heavy chain-heavy chain Crossfab"). For preparation of "2+1 IgG Crossfab" constructs cells were transfected with the corresponding expression vectors in a 1:2:1:1 ratio ("vector second heavy chain" : "vector light chain" : "vector first heavy chain-heavy chain Crossfab" : "vector light chain Crossfab". For preparation of the "2+1 IgG Crossfab (N-terminal), linked light chain" construct, cells were transfected with the corresponding expression vectors in a 1:1:1:1 ratio ("vector heavy chain" : "vector light chain" : "vector heavy chain (CrossFab-Fab-Fc)" : "vector linked light chain"). For preparation of the "1+1 CrossMab" construct, cells were transfected with the corresponding expression vectors in a 1:1:1:1 ratio ("vector first heavy chain" : "vector second heavy chain" : "vector first light chain" : "vector second light chain").

For transfection using calcium phosphate cells were grown as adherent monolayer cultures in T-flasks using DMEM culture medium supplemented with 10 % (v/v) FCS, and transfected when they were between 50 and 80 % confluent. For the transfection of a T150 flask, 15 million cells were seeded 24 hours before transfection in 25 ml DMEM culture medium supplemented with FCS (at 10% v/v final), and cells were placed at 37°C in an incubator with a 5% CO₂ atmosphere overnight. For each T150 flask to be transfected, a solution of DNA, CaCl₂ and water was prepared by mixing 94 µg total plasmid vector DNA divided in the corresponding ratio, water to a final volume of 469 µl and 469 µl of a 1 M CaCl₂ solution. To this solution, 938 µl of a 50 mM HEPES, 280 mM NaCl, 1.5 mM Na₂HPO₄ solution at pH 7.05 were added, mixed immediately for 10 s and left to stand at room temperature for 20 s. The suspension was diluted with 10 ml of DMEM supplemented with 2 % (v/v) FCS, and added to the T150 in place of the existing medium. Subsequently, additional 13 ml of transfection medium were added. The cells were incubated at 37°C, 5% CO₂ for about 17 to 20 hours, then medium was replaced with 25 ml DMEM, 10 % FCS. The conditioned culture medium was harvested approximately 7 days post-media exchange by centrifugation for 15 min at 210 x g, sterile filtered (0.22 • m filter), supplemented with sodium azide to a final concentration of 0.01 % (w/v), and kept at 4°C.

For transfection using polyethylenimine (PEI) HEK293 EBNA cells were cultivated in suspension in serum free CD CHO culture medium. For the production in 500 ml shake flasks, 400 million HEK293 EBNA cells were seeded 24 hours before transfection. For transfection cells were centrifuged for 5 min at 210 x g, and supernatant was replaced by 20 ml pre-warmed CD CHO medium. Expression vectors were mixed in 20 ml CD CHO medium to a final amount of 200 µg DNA. After addition of 540 µl PEI, the mixture was vortexed for 15 s and subsequently incubated for 10 min at room temperature. Afterwards cells were mixed with the DNA/PEI solution, transferred to a 500 ml shake flask and incubated for 3 hours at 37°C in an incubator with a 5% CO₂ atmosphere. After the incubation time 160 ml F17 medium was added and cells were cultivated for 24 hours. One day after transfection 1 mM valproic acid and 7% Feed 1 (Lonza) were added. After a cultivation of 7 days, supernatant was collected for purification by centrifugation for 15 min at 210 x g, the solution was sterile filtered (0.22 µm filter), supplemented with sodium azide to a final concentration of 0.01 % w/v, and kept at 4°C.

The secreted proteins were purified from cell culture supernatants by Protein A affinity chromatography, followed by a size exclusion chromatography step.

For affinity chromatography supernatant was loaded on a HiTrap ProteinA HP column (CV = 5 ml, GE Healthcare) equilibrated with 25 ml 20 mM sodium phosphate, 20 mM sodium citrate, pH 7.5 or 40 ml 20 mM sodium phosphate, 20 mM sodium citrate, 0.5 M sodium chloride, pH 7.5. Unbound protein was removed by washing with at least ten column volumes 20 mM sodium phosphate, 20 mM sodium citrate, 0.5 M sodium chloride pH 7.5, followed by an additional wash step using six column volumes 10 mM sodium phosphate, 20 mM sodium citrate, 0.5 M sodium chloride pH 5.45. Subsequently, the column was washed with 20 ml 10 mM MES, 100 mM sodium chloride, pH 5.0, and target protein was eluted in six column volumes 20 mM sodium citrate, 100 mM sodium chloride, 100 mM glycine, pH 3.0. Alternatively, target protein was eluted using a gradient over 20 column volumes from 20 mM sodium citrate, 0.5 M sodium chloride, pH 7.5 to 20 mM sodium citrate, 0.5 M sodium chloride, pH 2.5. The protein solution was neutralized by adding 1/10 of 0.5 M sodium phosphate, pH 8. The target protein was concentrated and filtrated prior to loading on a HiLoad Superdex 200 column (GE Healthcare) equilibrated with 25 mM potassium phosphate, 125 mM sodium chloride, 100 mM glycine solution of pH 6.7. For the purification of 1+1 IgG Crossfab the column was equilibrated with 20 mM histidine, 140 mM sodium chloride solution of pH 6.0.

The protein concentration of purified protein samples was determined by measuring the optical density (OD) at 280 nm, using the molar extinction coefficient calculated on the basis of the amino acid sequence. Purity and molecular weight of the bispecific constructs were analyzed by SDS-PAGE in the presence and absence of a reducing agent (5 mM 1,4-dithiotreitol) and staining with Coomassie (SimpleBlue™ SafeStain from Invitrogen), using the NuPAGE® PreCast gel system (Invitrogen, USA) according to the manufacturer's instructions (4-12% Tris-Acetate gels or 4-12% Bis-Tris). Alternatively, purity and molecular weight of molecules were analyzed by CE-SDS analyses in the presence and absence of a reducing agent, using the Caliper LabChip GXII system (Caliper Lifescience) according to the manufacturer's instructions.

The aggregate content of the protein samples was analyzed using a Superdex 200 10/300GL analytical size-exclusion chromatography column (GE Healthcare) in 2 mM MOPS, 150 mM NaCl, 0.02% (w/v) NaN₃, pH 7.3 running buffer at 25°C. Alternatively, the aggregate content of antibody samples was analyzed using a TSKgel G3000 SW XL analytical size-exclusion column (Tosoh) in 25 mM K₂HPO₄, 125 mM NaCl, 200 mM L-arginine monohydrocloride, 0.02% (w/v) NaN₃, pH 6.7 running buffer at 25°C.

The "2+1 IgG Crossfab (C-terminal)" construct (see SEQ ID NOs 11, 12, 13 and 14) was prepared using plasmid vectors having a genomic gene organization, including intron sequences, a CMV promoter and a polyadenylation signal from the gene of bovine growth hormone. The bispecific construct was transiently expressed in HEK293F cells by simultaneous transfection of required plasmids via lipofection using different plasmid ratios. Cell were grown in F17 medium. Supernatant was collected 5-7 days after transfection. Harvested cell-culture supernatant was sterile filtrated through a 0.2 µm pore-size membrane (Millipore) prior to purification. For purification, the bispecific molecule was captured on a MabSelectSure resin (GE Healthcare), washed with 1x PBS and eluted with 20 mM sodium-citrate at pH 3.0. The molecule was further purified by size exclusion chromatography using a Superdex™ 200 GL (Amersham Bioscience) column equilibrated with 20 mM histidine, 140 mM NaCl, pH 6.0. Characterization (antibody integrity assessment) of the bispecific molecule was done using Capillary electrophoresis (CE-SDS) analysis, using microfluidic Labchip technology (Caliper). 5 µl of protein solution was prepared for CE-SDS analysis using the HT Protein Express Reagent Kit according to the manufacturer's instructions and analysed on a LabChip GXII system using a HT Protein Express Chip.

Figures 2-5 show the results of the SDS PAGE and analytical size exclusion chromatography and Table 2 shows the yields, aggregate content after Protein A and final monomer content of the preparations of the different bispecific constructs. Importantly, the bispecific IgG Crossfab constructs showed a 10 to 20 fold reduced aggregate content after Protein A affinity chromatography as compared to corresponding bispecific constructs comprising a single chain Fab fragment instead of a Crossfab fragment (data not shown).

Figure 13 shows the result of the CE-SDS analyses of the anti-CD3/anti-MCSP bispecific "2+1 IgG Crossfab (N-terminal), linked light chain" construct (see SEQ ID NOs 1, 4, 5 and 85). 2 µg sample was used for analyses. Figure 14 shows the result of the analytical size exclusion chromatography of the final product (20 µg sample injected).

Figure 20 shows the results of the CE-SDS analyses of the 1+1 IgG Crossfab (N-terminal); VL/VH exchange (LC007/V9), the 1+1 CrossMab; CL/CH1 exchange (LC007/V9), the 2+1 IgG Crossfab (N-terminal), inverted; CL/CH1 exchange (LC007/V9), the 2+1 IgG Crossfab (N-terminal); VL/VH exchange (M4-3 ML2/V9), the 2+1 IgG Crossfab (N-terminal); CL/CH1 exchange (M4-3 ML2/V9), and the 2+1 IgG Crossfab (N-terminal), inverted; CL/CH1 exchange (CH1A1A/V9), and Table 2 shows the yields, aggregate content after Protein A and final monomer content of the preparations of the different bispecific constructs.

**TABLE 2. Yields, aggregate content after Protein A and final and monomer content.**

| **Construct** | **Yield [mg/l]** | **Aggregates after Protein A [%]** | **HMW [%]** | **LMW [%]** | **Monomer [%]** |
|---|---|---|---|---|---|
| 2+1 IgG Crossfab (N-terminal); VL/VH exchange (MCSP (LC007)/huCD3) | 12.8 | 2.2 | 0 | 0 | 100 |
| 2+1 IgG Crossfab (N-terminal); VL/VH exchange (MCSP (LC007)/cyCD3) | 3.2 | 5.7 | 0.4 | 0 | 99.6 |
| 1+1 IgG Crossfab (N-terminal); VL/VH exchange (MCSP (LC007)/huCD3) | 9.8 | 0 | 0 | 0 | 100 |
| 2+1 IgG Crossfab (N-terminal), inverted; VL/VH exchange (CEA/huCD3) | 0.34 | 13.04 | 4.4 | 0 | 95.6 |
| 2+1 IgG Crossfab (C-terminal); CL/CH1 exchange (c-Met/Her3) | 15 | 14 | | | |
| 2+1 IgG Crossfab (N-terminal), linked light chain; VL/VH exchange (MCSP (LC007)/huCD3) | 0.54 | 40 | 1.4 | 0 | 98.6 |
| 1+1 IgG Crossfab (N-terminal); VL/VH exchange (MCSP (LC007)/huCD3) | 6.61 | 8.5 | 0 | 0 | 100 |
| 1+1 CrossMab; CL/CH1 exchange (MCSP (LC007)/huCD3) | 6.91 | 10.5 | 1.3 | 1.7 | 97 |
| 2+1 IgG Crossfab (N-terminal), inverted; CL/CH1 exchange (MCSP (LC007)/huCD3 | 9.45 | 6.1 | 0.8 | 0 | 99.2 |
| 2+1 IgG Crossfab (N-terminal); VL/VH exchange (MCSP (M4-3 ML2)/huCD3) | 36.6 | 0 | 9.5 | 35.3 | 55.2 |
| 2+1 IgG Crossfab (N-terminal); CL/CH1 exchange (MCSP(M4-3 ML2)/huCD3) | 2.62 | 12 | 2.8 | 0 | 97.2 |
| 2+1 IgG Crossfab (N-terminal), inverted; CL/CH1 exchange (CEA/huCD3) | 12.7 | 43 | 0 | 0 | 100 |

### Example 2

### Simultaneous binding of bispecific constructs to both target antigens

Simultaneous binding to of the "2+1 IgG Crossfab (N-terminal)" construct (SEQ ID NOs 1, 3, 4, 5) to human MCSP and human CD3ε was analyzed by surface plasmon resonance (Figure 6).

All surface plasmon resonance (SPR) experiments are performed on a Biacore T100 at 25°C with HBS-EP as running buffer (0.01 M HEPES pH 7.4, 0.15 M NaCl, 3 mM EDTA, 0.005% Surfactant P20, Biacore, Freiburg/Germany).

Analysis of simultaneous binding of the bispecific construct to the tumor antigen and the human CD3ε was performed by direct coupling of 1650 resonance units (RU) of biotinylated D3 domain of MCSP on a sensor chip SA using the standard coupling procedure. The assay setup is shown in Figure 6A.

The "2+1 IgG Crossfab (N-terminal)" construct was captured for 60 s at 200 nM. Human CD3γ(G₄S)₅CD3ε-AcTev-Fc(knob)-Avi/Fc(hole) was subsequently passed at a concentration of 2000 nM and a flow rate of 40 µl/min for 60 s. Bulk refractive index differences were corrected for by subtracting the response obtained on a reference flow cell where the recombinant CD3ε was flown over a surface with immobilized D3 domain of MCSP without captured bispecific construct.

As shown in Figure 6B, the construct was able to bind the tumor antigen and the CD3 simultaneously. The binding level (RU) after injection of human CD3ε was higher than the binding level achieved after injection of the construct alone reflecting that both tumor antigen and the human CD3ε are bound to the bispecific construct.

### Example 3

### T cell activation by bispecific constructs in the presence and absence of target cells

### Cytokine release

The purified "2+1 IgG Crossfab (N-terminal)" construct (SEQ ID NOs 1, 3, 4, 5) and the "(scFv)₂"molecule, both targeting human MCSP and human CD3, were analyzed for their ability to induce T cell-mediated *de novo* secretion of cytokines in the presence or absence of tumor target cells.

Briefly, 280 µl whole blood from a healthy donor were plated per well of a deep-well 96-well plate. 30 000 Colo-38 tumor target cells, expressing human MCSP, as well as the two bispecific constructs and IgG controls were added at 1 nM final concentration. The cells were incubated for 24 h at 37°C, 5% CO₂ and then centrifuged for 5 min at 350 x g. The supernatant was transferred into a new deep-well 96-well-plate for the subsequent analysis. The CBA analysis was performed according to manufacturer's instructions for FACS CantoII, using the combination of the following CBA Flex Sets: human granzyme B (BD #560304), human IFN-γ Flex Set (BD #558269), human TNF Flex Set (BD #558273), human IL-10 Flex Set (BD #558274), human IL-6 Flex Set (BD #558276), human IL-4 Flex Set (BD #558272), human IL-2 Flex Set (BD #558270).

Figure 7 shows the levels of the different cytokine measured in the supernatant. The main cytokine secreted in the presence of Colo-38 tumor cells was IL-6, followed by IFN-γ. In addition, also the levels of granzyme B strongly increased upon activation of T cells in the presence of target cells. In general, both bispecific constructs induced high levels of cytokine secretion in the presence of target cells (Figure 7, A and B), but not in the absence of target cells (Figure 7, C and D). There was no significant secretion of Th2 cytokines (IL-10 and IL-4) upon activation of T cells in the presence (or absence) of target cells.

### Expression of surface activation markers

In another experiment, purified "2+1 IgG Crossfab (N-terminal)" (SEQ ID NOs 4, 5, 6, 7), targeting cynomolgus CD3 and human MCSP, was analyzed for its potential to up-regulate the surface activation marker CD25 on CD8⁺ T cells in the presence of tumor target cells. Briefly, human MCSP-expressing MV-3 tumor target cells were harvested with Cell Dissociation Buffer, washed and resuspendend in DMEM containing 2% FCS and 1% GlutaMax. 30 000 cells per well were plated in a round-bottom 96-well plate and the respective antibody dilution was added at the indicated concentrations (Figure 8). The bispecific construct and the different IgG controls were adjusted to the same molarity. Cynomolgus PBMC effector cells, isolated from blood of two healthy animals, were added to obtain a final E:T ratio of 3:1. After incubation for 43 h at 37°C, 5% CO₂, the cells were centrifuged at 350 x g for 5 min and washed twice with PBS, containing 0.1% BSA. Surface staining for CD8 (Miltenyi Biotech #130-080-601) and CD25 (BD #557138) was performed according to the supplier's suggestions. Cells were washed twice with 150 µl/well PBS containing 0.1% BSA and fixed for 15 min at 4°C, using 100 µl/well fixation buffer (BD #554655). After centrifugation, the samples were resuspended in 200 µl/well PBS with 0.1% BSA and analyzed using a FACS CantoII machine (Software FACS Diva).

As depicted in Figure 8, the bispecific construct induces concentration-dependent up-regulation of CD25 on CD8⁺ T cells only in the presence of target cells. The anti cyno CD3 IgG (clone FN-18) is also able to induce up-regulation of CD25 on CD8⁺ T cells, without being crosslinked to tumor target cells (see data obtained with cyno Nestor). There is no hyperactivation of cyno T cells with the maximal concentration of the bispecific construct (in the absence of target cells).

In another experiment, the CD3-MCSP "2+1 IgG Crossfab (N-terminal), linked light chain" (see SEQ ID NOs 1, 4, 5 and 85) was compared to the CD3-MCSP "2+1 IgG Crossfab (N-terminal)" (see SEQ ID NOs 1, 3, 4 and 5) for its potential to up-regulate the early activation marker CD69 or the late activation marker CD25 on CD8⁺ T cells in the presence of tumor target cells. Primary human PBMCs (isolated as described above) were incubated with the indicated concentrations of bispecific constructs for at least 22 h in the presence or absence of MCSP-positive Colo38 target cells. Briefly, 0.3 million primary human PBMCs were plated per well of a flat-bottom 96-well plate, containing the MCSP-positive target cells (or medium). The final effector to target cell (E:T) ratio was 10:1. The cells were incubated with the indicated concentration of the bispecific constructs and controls for the indicated incubation times at 37°C, 5% CO₂. The effector cells were stained for CD8, and CD69 or CD25 and analyzed by FACS CantoII.

Figure 19 shows the result of this experiment. There were no significant differences detected for CD69 (A) or CD25 up-regulation (B) between the two 2+1 IgG Crossfab molecules (with or without the linked light chain).

In yet another experiment, the CD3-MCSP "2+1 IgG Crossfab (N-terminal)" (see SEQ ID NOs 1, 3, 4, 5) and "1+1 IgG Crossfab (N-terminal)" (see SEQ ID NOs 1, 3, 4, 86) constructs were compared to a bispecific CD3-MCSP IgG-like construct having one antigen binding arm replaced by a Crossfab fragment ("1+1 CrossMab"; see SEQ ID NOs 4, 87, 88, 89) for their potential to up-regulate CD69 or CD25 on CD4⁺ or CD8⁺ T cells in the presence of tumor target cells. The assay was performed as described above, in the presence of absence of human MCSP expressing MV-3 tumor cells, with an incubation time of 24 h.

As shown in Figure 21, the "1+1 IgG Crossfab (N-terminal)" and "2+1 IgG Crossfab (N-terminal)" constructs induced more pronounced upregulation of activation markers than the "1+1 CrossMab" molecule.

### Example 4

### Re-directed T cell cytotoxicity mediated by cross-linked bispecific constructs targeting CD3 on T cells and MCSP on tumor cells (LDH release assay)

Bispecific constructs were analyzed for their potential to induce T cell-mediated apoptosis in tumor target cells upon crosslinkage of the construct via binding of the antigen binding moieties to their respective target antigens on cells.

In one experiment purified "2+1 IgG Crossfab (N-terminal)" construct (SEQ ID NOs 1, 3, 4, 5), targeting human CD3 and human MCSP, and the corresponding "(scFv)₂" molecule were compared. Briefly, huMCSP-expressing MDA-MB-435 human melanoma target cells were harvested with Cell Dissociation Buffer, washed and resuspendend in AIM-V medium (Invitrogen # 12055-091). 30 000 cells per well were plated in a round-bottom 96-well plate and the respective dilution of the construct was added at the indicated concentration. All constructs and controls were adjusted to the same molarity. Human pan T effector cells were added to obtain a final E:T ratio of 5:1. As a positive control for the activation of human pan T cells, 1 µg/ml PHA-M (Sigma #L8902) was used. For normalization, maximal lysis of the target cells (= 100%) was determined by incubation of the target cells with a final concentration of 1% Triton X-100. Minimal lysis (= 0%) refers to target cells co-incubated with effector cells, but without any construct or antibody. After an overnight incubation of 20 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 9, the "2+1 IgG Crossfab (N-terminal)" construct induces apoptosis in target cells comparable to the "(scFv)₂" molecule.

In a further experiment the purified "2+1 IgG Crossfab (N-terminal)" (SEQ ID NOs 1, 3, 4, 5), the "1+1 IgG Crossfab (N-terminal)" (SEQ ID NOs 1, 2, 3, 4) and the "(scFv)₂" molecule were analyzed for their potential to induce T cell-mediated apoptosis in tumor target cells upon crosslinkage of the construct via binding of both target antigens, CD3 and MCSP, on cells. huMCSP-expressing MDA-MB-435 human melanoma cells were used as target cells, the E:T ratio was 5:1, and the incubation time 20 h. The results are shown in Figure 10. The "2+1 IgG Crossfab (N-terminal)" construct induces apoptosis in target cells comparably to the "(scFv)₂" molecule. The comparison of the mono- and bivalent "IgG Crossfab (N-terminal)" formats shows that the bivalent one is more potent in this assay.

In yet another experiment, purified "2+1 IgG Crossfab (N-terminal)" (SEQ ID NOs 4, 5, 6, 7) targeting cynomolgus CD3 and human MCSP, and the corresponding "(scFv)₂" construct were compared, using MCSP-expressing human melanoma cell line (MV-3) as target cells. Briefly, MV-3 cells were harvested with Cell Dissociation Buffer, washed and resuspendend in DMEM containing 2% FCS and 1% GlutaMax. 30 000 cells per well were plated in a round-bottom 96-well plate and the respective dilution of construct or reference IgG was added at the concentrations indicated. The bispecific construct and the different IgG controls were adjusted to the same molarity. Cynomolgus PBMC effector cells, isolated from blood of healthy cynomolgus, were added to obtain a final E:T ratio of 10:1. After incubation for 26 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 11, the "2+1 IgG Crossfab (N-terminal)" construct is more potent in terms of EC50 than the "(scFv)₂" molecule.

In another set of experiments, the CD3-MCSP "2+1 IgG Crossfab (N-terminal), linked light chain" (see SEQ ID NOs 1, 4, 5 and 85) was compared to the CD3-MCSP "2+1 IgG Crossfab (N-terminal)" (see SEQ ID NOs 1, 3, 4 and 5). Briefly, target cells (human Colo-38, human MV-3 or WM266-4 melanoma cells) were harvested with Cell Dissociation Buffer on the day of the assay (or with trypsin one day before the assay was started), washed and resuspended in the appropriate cell culture medium (RPMI1640, including 2% FCS and 1% Glutamax). 20 000 - 30 000 cells per well were plated in a flat-bottom 96-well plate and the respective antibody dilution was added as indicated (triplicates). PBMCs as effector cells were added to obtain a final effector-to-target cell (E:T) ratio of 10:1. All constructs and controls were adjusted to the same molarity, incubation time was 22 h. Detection of LDH release and normalization was done as described above.

Figure 15 to 18 show the result of four assays performed with MV-3 melanoma cells (Figure 15), Colo-38 cells (Figure 16 and 17) or WM266-4 cells (Figure 18). As shown in Figure 15, the construct with the linked light chain was less potent compared to the one without the linked light chain in the assay with MV-3 cells as target cells. As shown in Figure 16 and 17, the construct with the linked light chain was more potent compared to the one without the linked light chain in the assays with high MCSP expressing Colo-38 cells as target cells. Finally, as shown in Figure 18, there was no significant difference between the two constructs when high MCSP-expressing WM266-4 cells were used as target cells.

In another experiment, two CEA-targeting "2+1 IgG Crossfab (N-terminal), inverted" constructs were compared, wherein in the Crossfab fragment either the V regions (VL/VH, see SEQ ID NOs 3, 8, 9, 10) or the C regions (CL/CH1, see SEQ ID NOs 9, 10, 87, 95) were exchanged. The assay was performed as described above, using human PBMCs as effector cells and human CEA-expressing target cells. Target cells (MKN-45 or LS-174T tumor cells) were harvested with trypsin-EDTA (LuBiosciences #25300-096), washed and resuspendend in RPMI1640 (Invitrogen #42404042), including 1% Glutamax (LuBiosciences #35050087) and 2% FCS. 30 000 cells per well were plated in a round-bottom 96-well plate and the bispecific constructs were added at the indicated concentrations. All constructs and controls were adjusted to the same molarity. Human PBMC effector cells were added to obtain a final E:T ratio of 10:1, incubation time was 28 h. EC50 values were calculated using the GraphPad Prism 5 software.

As shown in Figure 22, the construct with the CL/CH1 exchange shows slightly better activity on both target cell lines than the construct with the VL/VH exchange. Calculated EC50 values were 115 and 243 pM on MKN-45 cells, and 673 and 955 pM on LS-174T cells, for the CL/CH1-exchange construct and the VL/VH-exchange construct, respectively.

Similarly, two MCSP-targeting "2+1 IgG Crossfab (N-terminal)" constructs were compared, wherein in the Crossfab fragment either the V regions (VL/VH, see SEQ ID NOs 3, 91, 92, 93) or the C regions (CL/CH1, see SEQ ID NOs 87, 91, 93, 94) were exchanged. The assay was performed as described above, using human PBMCs as effector cells and human MCSP-expressing target cells. Target cells (WM266-4) were harvested with Cell Dissociation Buffer (LuBiosciences #13151014), washed and resuspendend in RPMI1640 (Invitrogen #42404042), including 1% Glutamax (LuBiosciences #35050087) and 2% FCS. 30 000 cells per well were plated in a round-bottom 96-well plate and the constructs were added at the indicated concentrations. All constructs and controls were adjusted to the same molarity. Human PBMC effector cells were added to obtain a final E:T ratio of 10:1, incubation time was 26 h. EC50 values were calculated using the GraphPad Prism 5 software.

As depicted in Figure 23, the two constructs show comparable activity, the construct with the CL/CH1 exchange having a slightly lower EC50 value (12.9 pM for the CL/CH1-exchange construct, compared to 16.8 pM for the VL/VH-exchange construct).

Figure 24 shows the result of a similar assay, performed with human MCSP-expressing MV-3 target cells. Again, both constructs show comparable activity, the construct with the CL/CH1 exchange having a slightly lower EC50 value (approximately 11.7 pM for the CL/CH1-exchange construct, compared to approximately 82.2 pM for the VL/VH-exchange construct). Exact EC50 values could not be calculated, since the killing curves did not reach a plateau at high concentrations of the compounds.

In a further experiment, the CD3-MCSP "2+1 IgG Crossfab (N-terminal)" (see SEQ ID NOs 1, 3, 4, 5) and "1+1 IgG Crossfab (N-terminal)" (see SEQ ID NOs 1, 3, 4, 86) constructs were compared to the CD3-MCSP "1+1 CrossMab" (see SEQ ID NOs 4, 87, 88, 89). The assay was performed as described above, using human PBMCs as effector cells and WM266-4 or MV-3 target cells (E:T ratio = 10:1) and an incubation time of 21 h.

As shown in Figure 25, the "2+1 IgG Crossfab (N-terminal)" construct is the most potent molecule in this assay, followed by the "1+1 IgG Crossfab (N-terminal)" and the "1+1 CrossMab". This ranking is even more pronounced with MV-3 cells, expressing medium levels of MCSP, compared to high MCSP expressing WM266-4 cells. The calculated EC50 values on MV-3 cells were 9.2, 40.9 and 88.4 pM, on WM266-4 cells 33.1, 28.4 and 53.9 pM, for the "2+1 IgG Crossfab (N-terminal)", the "1+1 IgG Crossfab (N-terminal)" and the "1+1 CrossMab", respectively.

### Example 5

### Binding of bispecific constructs to the respective target antigen on cells

Binding of the different bispecific constructs to CD3 on Jurkat (ATCC #TIB-152) cells, and the respective tumor antigen MCSP on WM266-4 cells or CEA on LS174-T cells, was determined by FACS. Briefly, cells were harvested, counted and checked for viability. 0.15 - 0.2 million cells per well were plated in a round-bottom 96-well plate and incubated with the indicated concentration of the bispecific constructs and controls for 30 min at 4°C. For a better comparison, the constructs were normalized to same molarity. Cells were washed with PBS containing 0.1% BSA once. After incubation with a FITC-or PE-conjugated secondary antibody for 30 min at 4°C, bound constructs were detected using a FACSCantoII (Software FACS Diva). A FITC- or PE-conjugated AffiniPure F(ab')2 Fragment goat anti-human IgG Fcγ Fragment Specific (Jackson Immuno Research Lab # 109-096-098 / working solution 1:20, or #109-116-170 / working solution 1:80, respectively) was used. Unless otherwise indicated, cells were fixed with 100 µl/well fixation buffer (BD #554655) for 15 min at 4°C in the dark, centrifuged for 6 min at 400 x g and kept in 200 µl/well PBS containing 0.1% BSA until analysis. EC50 values were calculated using the GraphPad Prism 5 software.

Figure 26 shows the binding of CD3/CEA "2+1 IgG Crossfab (N-terminal), inverted" bispecific constructs with either a VL/VH (see SEQ ID NOs 3, 8, 9, 10) or a CL/CH1 exchange (see SEQ ID NOs 9, 10, 87, 95) in the Crossfab fragment to human CD3, expressed by Jurkat cells, or to human CEA, expressed by LS-174T cells. As a control, the equivalent maximum concentration of the corresponding IgGs and the background staining due to the labeled 2ndary antibody (goat anti-human FITC-conjugated AffiniPure F(ab')2 Fragment, Fcγ Fragment-specific, Jackson Immuno Research Lab # 109-096-098) were assessed as well. Both constructs show good binding to human CEA, as well as to human CD3 on cells. The calculated EC50 values were 4.6 and 3.9 nM (CD3), and 9.3 and 6.7 nM (CEA) for the "2+1 IgG Crossfab (N-terminal), inverted (VL/VH)" and the "2+1 IgG Crossfab (N-terminal), inverted (CL/CH1)" constructs, respectively.

Figure 27 shows the binding of CD3/MCSP "2+1 IgG Crossfab (N-terminal)" (see SEQ ID NOs 1, 3, 4, 5) and "2+1 IgG Crossfab (N-terminal), inverted" (see SEQ ID NOs 4, 87, 89, 90) constructs to human CD3, expressed by Jurkat cells, or to human MCSP, expressed by WM266-4 cells. While binding of both construct to MCSP on cells was comparably good, the binding of the "inverted" construct to CD3 was reduced compared to the other construct. The calculated EC50 values were 6.1 and 1.66 nM (CD3), and 0.57 and 0.95 nM (MCSP) for the "2+1 IgG Crossfab (N-terminal), inverted" and the "2+1 IgG Crossfab (N-terminal)" constructs, respectively.

## Claims

1. A bispecific antigen binding molecule, comprising a first Fab fragment which specifically binds to a first antigen, a second Fab fragment which specifically binds to a second antigen, and an Fc domain composed of a first and a second subunit capable of stable association; wherein
a) the bispecific antigen binding molecule provides monovalent binding to the first and/or the second antigen,
b) (i) the first Fab fragment is fused at its C-terminus to the N-terminus of the second Fab fragment, which is in turn fused at its C-terminus to the N-terminus of the first Fc domain subunit, or (ii) the second Fab fragment is fused at its C-terminus to the N-terminus of the first Fab fragment, which is in turn fused at its C-terminus to the N-terminus of the first Fc domain subunit,
c) in the first and/or the second Fab fragment one of the following replacements is made: (i) the variable domains VL and VH are replaced by each other, or (ii) the constant domains CL and CH1 are replaced by each other,
provided that not the same replacement is made in the first and the second Fab fragment, and
d) the bispecific antigen binding molecule does not comprise a single-chain Fab fragment.

2. The bispecific antigen binding molecule of claim 1(b)(i), wherein the first Fab fragment is fused at the C-terminus of its heavy chain to the N-terminus of the heavy chain of the second Fab fragment, which is in turn fused at the C-terminus of its heavy chain to the N-terminus of the first Fc domain subunit.

3. The bispecific antigen binding molecule of claim 1(b)(ii), wherein the second Fab fragment is fused at the C-terminus of its heavy chain to the N-terminus of the heavy chain of the first Fab fragment, which is in turn fused at the C-terminus of its heavy chain to the N-terminus of the first Fc domain subunit.

4. The bispecific antigen binding molecule of any one of claims 1 to 3, wherein additionally the Fab light chain of the first Fab fragment and the Fab light chain of the second Fab fragment are fused to each other, optionally via a peptide linker.

5. The bispecific antigen binding molecule of any one of the preceding claims, wherein the replacement is made in the first Fab fragment.

6. The bispecific antigen binding molecule of any one of the preceding claims, consisting of the first Fab fragment, the second Fab fragment, the Fc domain, and optionally one or more peptide linkers.

7. The bispecific antigen binding molecule of any one of claims 1 to 5, comprising a third Fab fragment which specifically binds to the first or the second antigen.

8. The bispecific antigen binding molecule of claim 7, wherein
i) the third Fab fragment is fused to the second Fc domain subunit;
ii) the third Fab fragment is fused at its C-terminus to the N-terminus of the second Fc domain subunit; and/or
iii) the third Fab fragment is fused at the C-terminus of its heavy chain to the N-terminus of the second Fc domain subunit.

9. The bispecific antigen binding molecule of claim 7 or 8, wherein the third Fab fragment specifically binds to the second antigen.

10. The bispecific antigen binding molecule of any one of claims 7 to 9, wherein
i) the second Fab fragment, the third Fab fragment and the Fc domain are part of an immunoglobulin molecule;
ii) the second Fab fragment, the third Fab fragment and the Fc domain are part of an immunoglobulin molecule and the immunoglobulin molecule is an IgG class immunoglobulin molecule;
iii) the second Fab fragment, the third Fab fragment and the Fc domain are part of an immunoglobulin molecule and the immunoglobulin molecule is an IgG1 or IgG4 subclass immunoglobulin molecule; and/or
iv) the second Fab fragment, the third Fab fragment and the Fc domain are part of an immunoglobulin molecule and the immunoglobulin molecule is a human immunoglobulin molecule.

11. The bispecific antigen binding molecule of any one of claims 7 to 10, consisting of a first Fab fragment which specifically binds to the first antigen, an immunoglobulin molecule which specifically binds to the second antigen, and optionally one or more peptide linkers.

12. The bispecific antigen binding molecule of any one of the preceding claims, wherein
i) the same replacement is made in Fab fragments that specifically bind to the same antigen;
ii) the bispecific antigen binding molecule provides monovalent binding to the first antigen;
iii) a replacement is made only in the first Fab fragment;
iv) the Fc domain comprises a modification promoting the association of the first and second Fc domain subunit and wherein the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable;
v) the Fc domain is an IgG Fc domain;
vi) the Fc domain is an IgG1 or IgG4 Fc domain; and/or
vii) the Fc domain is human.

13. The bispecific antigen binding molecule of any one of the preceding claims, wherein (i) the Fc domain comprises one or more amino acid substitution that reduces the binding affinity of the Fc domain to an Fc receptor and/or reduces effector function, wherein said amino acid substitution is at a position selected from the group of E233, L234, L235, N297, P331 and P329 (EU numbering); or (ii) the Fc domain is engineered to comprise an increased proportion of non-fucosylated oligosaccharides, as compared to a non-engineered Fc domain.

14. An isolated polynucleotide encoding the bispecific antigen binding molecule of any one of claims 1 to 13.

15. A pharmaceutical composition comprising the bispecific antigen binding molecule of any one of claims 1 to 13 and a pharmaceutically acceptable carrier.

16. The bispecific antigen binding molecule of any one of claims 1 to 13 or the pharmaceutical composition of claim 15
i) for use as a medicament;
ii) for use in the treatment of a disease in an individual in need thereof; or
iii) for use in the treatment of a disease in an individual in need thereof, wherein the disease is cancer.

## Patentansprüche

1. Bispezifisches antigenbindendes Molekül, umfassend ein erstes Fab-Fragment, das spezifisch an ein erstes Antigen bindet, ein zweites Fab-Fragment, das spezifisch an ein zweites Antigen bindet, und eine Fc-Domäne, die aus einer ersten und einer zweiten Untereinheit besteht, die zu einer stabilen Assoziation in der Lage sind; wobei
a) das bispezifische antigenbindende Molekül eine monovalente Bindung an das erste und/oder das zweite Antigen bereitstellt,
b) (i) das erste Fab-Fragment an seinem C-Terminus an den N-Terminus des zweiten Fab-Fragments fusioniert ist, das wiederum an seinem C-Terminus an den N-Terminus der ersten Fc-Domänen-Untereinheit fusioniert ist, oder (ii) das zweite Fab-Fragment an seinem C-Terminus an den N-Terminus des ersten Fab-Fragments fusioniert ist, das wiederum an seinem C-Terminus an den N-Terminus der ersten Fc-Domänen-Untereinheit fusioniert ist,
c) in dem ersten und/oder dem zweiten Fab-Fragment eine der folgenden Ersetzungen vorgenommen ist: (i) die variablen Domänen VL und VH sind durch einander ersetzt, oder (ii) die konstanten Domänen CL und CH1 sind durch einander ersetzt, vorausgesetzt, dass in dem ersten und dem zweiten Fab-Fragment nicht die gleichen Ersetzungen vorgenommen sind, und
d) das bispezifische antigenbindende Molekül kein Einzelketten-Fab-Fragment umfasst.

2. Bispezifisches antigenbindendes Molekül nach Anspruch 1 (b) (i), wobei das erste Fab-Fragment an dem C-Terminus seiner Schwerkette an den N-Terminus der Schwerkette des zweiten Fab-Fragments fusioniert ist, das wiederum an dem C-Terminus seiner Schwerkette an den N-Terminus der ersten Fc-Domänen-Untereinheit fusioniert ist.

3. Bispezifisches antigenbindendes Molekül nach Anspruch 1 (b) (ii), wobei das zweite Fab-Fragment an dem C-Terminus seiner Schwerkette an den N-Terminus der Schwerkette des ersten Fab-Fragments fusioniert ist, das wiederum an dem C-Terminus seiner Schwerkette an den N-Terminus der ersten Fc-Domänen-Untereinheit fusioniert ist.

4. Bispezifisches antigenbindendes Molekül nach einem der Ansprüche 1 bis 3, wobei zusätzlich die Fab-Leichtkette des ersten Fab-Fragments und die Fab-Leichtkette des zweiten Fab-Fragments miteinander fusioniert sind, gegebenenfalls über einen Peptidlinker.

5. Bispezifisches antigenbindendes Molekül nach einem der vorhergehenden Ansprüche, wobei die Ersetzung in dem ersten Fab-Fragment vorgenommen ist.

6. Bispezifisches antigenbindendes Molekül nach einem der vorhergehenden Ansprüche, bestehend aus dem ersten Fab-Fragment, dem zweiten Fab-Fragment, der Fc-Domäne und gegebenenfalls einem oder mehreren Peptidlinkern.

7. Bispezifisches antigenbindendes Molekül nach einem der Ansprüche 1 bis 5, umfassend ein drittes Fab-Fragment, das spezifisch an das erste oder das zweite Antigen bindet.

8. Bispezifisches antigenbindendes Molekül nach Anspruch 7, wobei
i) das dritte Fab-Fragment an die zweite Fc-Domänen-Untereinheit fusioniert ist;
ii) das dritte Fab-Fragment an seinem C-Terminus an den N-Terminus der zweiten Fc-Domänen-Untereinheit fusioniert ist; und/oder
iii) das dritte Fab-Fragment an dem C-Terminus seiner Schwerkette an den N-Terminus der zweiten Fc-Domänen-Untereinheit fusioniert ist.

9. Bispezifisches antigenbindendes Molekül nach Anspruch 7 oder 8, wobei das dritte Fab-Fragment spezifisch an das zweite Antigen bindet.

10. Bispezifisches antigenbindendes Molekül nach einem der Ansprüche 7 bis 9, wobei
i) das zweite Fab-Fragment, das dritte Fab-Fragment und die Fc-Domäne Teil eines Immunglobulinmoleküls sind;
ii) das zweite Fab-Fragment, das dritte Fab-Fragment und die Fc-Domäne Teil eines Immunglobulinmoleküls sind und das Immunglobulinmolekül ein Immunglobulinmolekül der IgG-Klasse ist.
iii) das zweite Fab-Fragment, das dritte Fab-Fragment und die Fc-Domäne Teil eines Immunglobulinmoleküls sind und das Immunglobulinmolekül ein Immunglobulinmolekül der IgG1- oder IgG4-Unterklasse ist; und/oder
iv) das zweite Fab-Fragment, das dritte Fab-Fragment und die Fc-Domäne Teil eines Immunglobulinmoleküls sind und das Immunglobulinmolekül ein humanes Immunglobulinmolekül ist.

11. Bispezifisches antigenbindendes Molekül nach einem der Ansprüche 7 bis 10, bestehend aus einem ersten Fab-Fragment, das spezifisch an das erste Antigen bindet, einem Immunglobulinmolekül, das spezifisch an das zweite Antigen bindet, und gegebenenfalls einem oder mehreren Peptidlinkern.

12. Bispezifisches antigenbindendes Molekül nach einem der vorhergehenden Ansprüche, wobei
i) die gleiche Ersetzung in Fab-Fragmenten vorgenommen ist, die spezifisch an das gleiche Antigen binden;
ii) das bispezifische antigenbindende Molekül eine monovalente Bindung an das erste Antigen bereitstellt;
iii) eine Ersetzung nur in dem ersten Fab-Fragment vorgenommen ist;
iv) die Fc-Domäne eine Modifikation umfasst, die die Assoziation der ersten und zweiten Fc-Domänen-Untereinheit fördert, und wobei in der CH3-Domäne der ersten Untereinheit der Fc-Domäne ein Aminosäurerest durch einen Aminosäurerest ersetzt ist, der ein größeres Seitenkettenvolumen aufweist, wodurch ein Vorsprung innerhalb der CH3-Domäne der ersten Untereinheit erzeugt wird, der in einem Hohlraum innerhalb der CH3-Domäne der zweiten Untereinheit positionierbar ist, und in der CH3-Domäne der zweiten Untereinheit der Fc-Domäne ein Aminosäurerest durch einen Aminosäurerest ersetzt ist, der ein kleineres Seitenkettenvolumen aufweist, wodurch ein Hohlraum innerhalb der CH3-Domäne der zweiten Untereinheit erzeugt wird, innerhalb dessen der Vorsprung innerhalb der CH3-Domäne der ersten Untereinheit positionierbar ist;
v) die Fc-Domäne eine IgG-Fc-Domäne ist;
vi) die Fc-Domäne eine IgG1- oder IgG4-Fc-Domäne ist; und/oder
vii) die Fc-Domäne human ist.

13. Bispezifisches antigenbindendes Molekül nach einem der vorhergehenden Ansprüche, wobei (i) die Fc-Domäne eine oder mehrere Aminosäuresubstitutionen umfasst, die die Bindungsaffinität der Fc-Domäne an einen Fc-Rezeptor reduziert/en und/oder eine Effektorfunktion reduziert/en, wobei sich die Aminosäuresubstitution an einer Position befindet, die aus der Gruppe von E233, L234, L235, N297, P331 und P329 (EU-Nummerierung) ausgewählt ist; oder (ii) die Fc-Domäne verändert ist, um im Vergleich zu einer nicht veränderten Fc-Domäne einen erhöhten Anteil an nicht fucosylierten Oligosacchariden zu umfassen.

14. Isoliertes Polynukleotid, das das bispezifische antigenbindende Molekül nach einem der Ansprüche 1 bis 13 kodiert.

15. Pharmazeutische Zusammensetzung, umfassend das bispezifische antigenbindende Molekül nach einem der Ansprüche 1 bis 13 und einen pharmazeutisch annehmbaren Träger.

16. Bispezifisches antigenbindendes Molekül nach einem der Ansprüche 1 bis 13 oder die pharmazeutische Zusammensetzung nach Anspruch 15
i) zur Verwendung als ein Medikament;
ii) zur Verwendung bei der Behandlung einer Krankheit in einem Individuum, das diese benötigt; oder
iii) zur Verwendung bei der Behandlung einer Krankheit in einem Individuum, das diese benötigt, wobei die Krankheit Krebs ist.

## Revendications

1. Molécule bispécifique de liaison à un antigène, comprenant un premier fragment Fab qui se lie spécifiquement à un premier antigène, un deuxième fragment Fab qui se lie spécifiquement à un second antigène, et un domaine Fc se composant d'un premier et d'un second sous-motif susceptible d'établir une association stable ; dans laquelle
a) la molécule bispécifique de liaison à un antigène fournit une liaison monovalente au premier et/ou au second antigène,
b) (i) le premier fragment Fab est condensé au niveau de son extrémité C-terminale avec l'extrémité N-terminale du deuxième fragment Fab, qui est à son tour condensé au niveau de son extrémité C-terminale avec l'extrémité N-terminale du premier sous-motif du domaine Fc, ou (ii) le deuxième fragment Fab est condensé au niveau de son extrémité C-terminale avec l'extrémité N-terminale du premier fragment Fab, qui est à son tour condensé au niveau de son extrémité C-terminale avec l'extrémité N-terminale du premier sous-motif du domaine Fc,
c) dans le premier et/ou le deuxième fragment Fab, l'un des échanges suivants est mis en oeuvre : (i) les domaines variables VL et VH sont échangés, ou (ii) les domaines constants CL et CH1 sont échangés, à condition que le même échange ne soit pas mis en oeuvre dans le premier et dans le deuxième fragment Fab, et
d) la molécule bispécifique de liaison à un antigène ne comprend pas de fragment Fab monocaténaire.

2. Molécule bispécifique de liaison à un antigène selon la revendication 1(b)(i), dans laquelle le premier fragment Fab est condensé au niveau de l'extrémité C-terminale de sa chaîne lourde avec l'extrémité N-terminale de la chaîne lourde du deuxième fragment Fab, qui est à son tour condensé au niveau de l'extrémité C-terminale de sa chaîne lourde avec l'extrémité N-terminale du premier sous-motif du domaine Fc.

3. Molécule bispécifique de liaison à un antigène selon la revendication 1(b)(ii), dans laquelle le deuxième fragment Fab est condensé au niveau de l'extrémité C-terminale de sa chaîne lourde avec l'extrémité N-terminale de la chaîne lourde du premier fragment Fab, qui est à son tour condensé au niveau de l'extrémité C-terminale de sa chaîne lourde avec l'extrémité N-terminale du premier sous-motif du domaine Fc.

4. Molécule bispécifique de liaison à un antigène selon l'une quelconque des revendications 1 à 3, dans laquelle la chaîne légère Fab du premier fragment Fab et la chaîne légère Fab du deuxième fragment Fab sont en outre condensées l'une avec l'autre, éventuellement par l'intermédiaire d'un lieur peptidique.

5. Molécule bispécifique de liaison à un antigène selon l'une quelconque des revendications précédentes, dans laquelle l'échange est mis en oeuvre dans le premier fragment Fab.

6. Molécule bispécifique de liaison à un antigène selon l'une quelconque des revendications précédentes, constituée du premier fragment Fab, du deuxième fragment Fab, du domaine Fc, et éventuellement d'un ou de plusieurs lieurs peptidiques.

7. Molécule bispécifique de liaison à un antigène selon l'une quelconque des revendications 1 à 5, comprenant un troisième fragment Fab qui se lie spécifiquement au premier ou au second antigène.

8. Molécule bispécifique de liaison à un antigène selon la revendication 7, dans laquelle
i) le troisième fragment Fab est condensé avec le second sous-motif du domaine Fc ;
ii) le troisième fragment Fab est condensé au niveau de son extrémité C-terminale avec l'extrémité N-terminale du second sous-motif du domaine Fc ; et/ou
iii) le troisième fragment Fab est condensé au niveau de l'extrémité C-terminale de sa chaîne lourde avec l'extrémité N-terminale du second sous-motif du domaine Fc.

9. Molécule bispécifique de liaison à un antigène selon la revendication 7 ou 8, dans laquelle le troisième fragment Fab se lie spécifiquement au second antigène.

10. Molécule bispécifique de liaison à un antigène selon l'une quelconque des revendications 7 à 9, dans laquelle
i) le deuxième fragment Fab, le troisième fragment Fab et le domaine Fc font partie d'une molécule d'immunoglobuline ;
ii) le deuxième fragment Fab, le troisième fragment Fab et le domaine Fc font partie d'une molécule d'immunoglobuline et la molécule d'immunoglobuline est une molécule d'immunoglobuline de la classe des IgG ;
iii) le deuxième fragment Fab, le troisième fragment Fab et le domaine Fc font partie d'une molécule d'immunoglobuline et la molécule d'immunoglobuline est une molécule d'immunoglobuline de la sous-classe des IgG1 ou des IgG4 ; et/ou
iv) le deuxième fragment Fab, le troisième fragment Fab et le domaine Fc font partie d'une molécule d'immunoglobuline et la molécule d'immunoglobuline est une molécule d'immunoglobuline humaine.

11. Molécule bispécifique de liaison à un antigène selon l'une quelconque des revendications 7 à 10, constituée d'un premier fragment Fab qui se lie spécifiquement au premier antigène, d'une molécule d'immunoglobuline qui se lie spécifiquement au second antigène, et éventuellement d'un ou de plusieurs lieurs peptidiques.

12. Molécule bispécifique de liaison à un antigène selon l'une quelconque des revendications précédentes, dans laquelle
i) le même échange est mis en oeuvre dans les fragments Fab qui se lient spécifiquement au même antigène ;
ii) la molécule bispécifique de liaison à un antigène fournit une liaison monovalente au premier antigène ;
iii) un échange est mis en oeuvre uniquement dans le premier fragment Fab ;
iv) le domaine Fc comprend une modification favorisant l'association du premier et du second sous-motif du domaine Fc et, dans le domaine CH3 du premier sous-motif du domaine Fc, un résidu d'acide aminé est remplacé par un résidu d'acide aminé présentant un volume de chaîne latérale plus grand, générant ainsi une protubérance au sein du domaine CH3 du premier sous-motif qui peut être positionnée dans une cavité au sein du domaine CH3 du second sous-motif, et, dans le domaine CH3 du second sous-motif du domaine Fc, un résidu d'acide aminé est remplacé par un résidu d'acide aminé présentant un volume de chaîne latérale plus petit, générant ainsi une cavité au sein du domaine CH3 du second sous-motif à l'intérieur de laquelle peut être positionnée la protubérance au sein du domaine CH3 du premier sous-motif ;
v) le domaine Fc est un domaine Fc d'IgG ;
vi) le domaine Fc est un domaine Fc d'IgG1 ou d'IgG4 ; et/ou
vii) le domaine Fc est un domaine humain.

13. Molécule bispécifique de liaison à un antigène selon l'une quelconque des revendications précédentes, dans laquelle (i) le domaine Fc comprend une ou plusieurs substitutions d'acides aminés qui réduit l'affinité de liaison du domaine Fc à un récepteur Fc et/ou qui réduit une fonction effectrice, ladite substitution d'acides aminés étant réalisée au niveau d'une position choisie dans le groupe constitué par E233, L234, L235, N297, P331 et P329 (codage de l'UE) ; ou (ii) le domaine Fc est modifié de sorte à comprendre une proportion accrue d'oligosaccharides non fucosylés, par comparaison avec un domaine Fc non modifié.

14. Polynucléotide isolé codant pour la molécule bispécifique de liaison à un antigène selon l'une quelconque des revendications 1 à 13.

15. Composition pharmaceutique comprenant la molécule bispécifique de liaison à un antigène selon l'une quelconque de revendications 1 à 13 et un véhicule pharmaceutiquement acceptable.

16. Molécule bispécifique de liaison à un antigène selon l'une quelconque des revendications 1 à 13 ou composition pharmaceutique selon la revendication 15,
i) destinée à être utilisée comme médicament ;
ii) destinée à être utilisée dans le traitement d'une maladie chez un individu qui en a besoin ; ou
iii) destinée à être utilisée dans le traitement d'une maladie chez un individu qui en a besoin, ladite maladie étant un cancer.
